(19) Europäisches Patentamt
European Patent Office
Office européen des brevets

(11) Veröffentlichungsnummer: **0 186 043 B1**

# (12) EUROPÄISCHE PATENTSCHRIFT

(45) Veröffentlichungstag der Patentschrift: **19.06.91**

(51) Int. Cl.5: **C07D 493/22**, A61K 31/35, A01N 43/04, //(C07D493/22, 313:00,311:00,311:00,307:00)

(21) Anmeldenummer: **85115749.5**

(22) Anmeldetag: **11.12.85**

Die Akte enthält technische Angaben, die nach dem Eingang der Anmeldung eingereicht wurden und die nicht in dieser Patentschrift enthalten sind.

## (54) Milbemycinderivate mit pestiziden Eigenschaften.

(30) Priorität: **14.12.84 CH 5940/84**
**30.05.85 CH 2289/85**

(43) Veröffentlichungstag der Anmeldung:
**02.07.86 Patentblatt 86/27**

(45) Bekanntmachung des Hinweises auf die Patenterteilung:
**19.06.91 Patentblatt 91/25**

(84) Benannte Vertragsstaaten:
**AT BE CH DE FR IT LI LU NL SE**

(56) Entgegenhaltungen:
**EP-A- 1 688**
**EP-A- 1 689**
**EP-A- 2 615**
**EP-A- 7 812**
**EP-A- 8 184**
**EP-A- 40 913**
**EP-A- 46 045**
**EP-A- 58 518**
**EP-A- 65 403**
**EP-A- 73 660**
**EP-A- 74 758**
**EP-A- 89 202**
**EP-A- 102 721**
**EP-A- 110 667**
**EP-A- 115 930**
**FR-A- 2 387 231**
**US-A- 4 093 629**
**US-A- 4 423 209**

(73) Patentinhaber: **CIBA-GEIGY AG**
**Klybeckstrasse 141**
**CH-4002 Basel(CH)**

(72) Erfinder: **Frei, Bruno, Dr.**
**Bündtenstr. 16**
**CH-4410 Liestal(CH)**
Erfinder: **Mereyala, Hari Babu, Dr.**
**NCL-Colony, C-5, Pashan**
**Pune-411008(IN)**
Erfinder: **O'Sullivan, Anthony Cornelius, Dr.**
**Habsburgerstr. 38**
**CH-4055 Basel(CH)**
Erfinder: **Sato, Kazuo, Dr.**
**1-1105, 2-42-2, Tenno-cho, Hodogaya-ku**
**Yokohama-shi Kanagawa-Ken(JP)**
Erfinder: **Toshiaki, Yanai, Dr.**
**2292-72, Imajuku-cho, Asahi-ku**
**Yokohama-shi Kanagawa-Ken(JP)**

(74) Vertreter: **Zumstein, Fritz, Dr. et al**
**Bräuhausstrasse 4**
**W-8000 München 2(DE)**

**EP 0 186 043 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue 13ß-Milbemycin-Derivate der Formel I, ihre Herstellung und ihre Verwendung zur Bekämpfung von Schädlingen, ausgenommen die Anwendung am menschlichen oder tierischen Körper, und Schädlingsbekämpfungsmittel, die mindestens eine dieser Verbindungen als Wirkstoff enthalten. Die Wirkstoffe und Mittel eignen sich für die Verwendung bei der Bekämpfung von Tiere befallenden und pflanzenschädigenden Parasiten.

In der EP-A-1689 wird die Struktur von (als Ausgangsstoffe eiuzusetzenden) C-076-Verbindungen angegeben, ohne daß für sie jedoch eine konkrete, nacharbeitbare Herstellungsmethode beschrieben ist.

In der US-A-4093629 wird (als Zwischenprodukt) eine zu den anspruchsgemäßen Verbindungen strukturell ähnliche Verbindung beschrieben, jedoch ohne Offenbarung einer biologischen Aktivität.

Die neuen Verbindungen entsprechen der allgemeinen Formel I

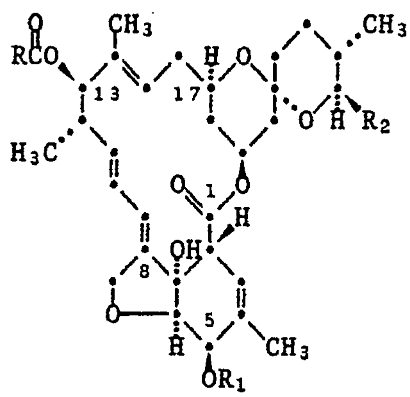

(I)

in welcher

$R_1$ Wasserstoff, die Silylgruppe -Si($R_6$)($R_7$)($R_8$), worin $R_6$, $R_7$ und $R_8$ unabhängig voneinander für $C_1$-$C_4$-Alkyl, Benzyl oder Phenyl stehen, oder die Acylgruppe $R_5$-C(O)-, worin $R_5$ für $C_1$-$C_{10}$-Alkyl, $C_1$-$C_{10}$-Haloalkyl oder einen unsubstituierten oder durch Substituenten der Gruppe bestehend aus Halogen, $C_1$-$C_3$-Alkyl, $C_1$-$C_3$-Haloalkyl, $C_1$-$C_3$-Alkoxy, $C_1$-$C_3$-Haloalkoxy, Nitro und Cyano substituierten Rest aus der Gruppe Phenyl und Benzyl steht,

$R_2$ Methyl, Ethyl, Isopropyl oder sek.Butyl und

R Wasserstoff, tert.Butyl, geradkettige oder verzweigte, durch 1 bis 7 Halogenatome, 1 bis 6 $C_1$-$C_6$-Alkoxygruppen oder eine unsubstituierte oder halogenierte, insbesondere durch 1 bis 3 Halogenatome substituierte Phenoxygruppe substituierte $C_1$-$C_{18}$-Alkylgruppen, mono- bis tetracyclische, aliphatische Gruppen mit 3 bis 10 Kohlenstoffatomen, welche in Verbindungen, in denen $R_1$ für Wasserstoff und $R_2$ für Aethyl oder Isopropyl steht, durch Substituenten der Gruppe bestehend aus $C_1$-$C_4$-Alkyl und halogeniertem $C_2$-$C_4$-Alkenyl ein- oder mehrfach substituiert sein können, durch 1 bis 7 Halogenatome, 1 bis 6 $C_1$-$C_6$-Alkoxygruppen oder durch $C_1$-$C_4$-Alkylgruppen substituierte Cycloalkylgruppen mit 3 bis 10 Kohlenstoffatomen, unsubstituierte oder durch 1 bis 7 Halogenatome oder 1 bis 6 $C_1$-$C_6$-Alkoxygruppen substituierte $C_2$-$C_6$-Alkenyl- oder Alkinylgruppen oder eine unsubstituierte oder durch 1 bis 3 Substituenten ausgewählt aus der Gruppe bestehend aus Halogenatomen, $C_1$-$C_6$-Alkyl, $C_1$-$C_6$-Alkoxy, $C_1$-$C_4$-Alkylthio oder Nitro substituierte Phenyl- oder Benzylgruppe bedeuten.

Unter den vorgenannten Bedeutungen sind als bevorzugt anzusehen: unsubstituiertes oder halogeniertes $C_1$-$C_8$-Alkyl, unsubstituiertes oder durch Methyl ein- oder mehrfach substituiertes $C_3$-$C_6$-Cycloalkyl, Adamantyl, unsubstituiertes oder halogeniertes $C_2$-$C_6$-Alkenyl und $C_2$-$C_6$-Alkinyl.

Unter dem Begriff Alkyl als Substituent oder Teil eines Substituenten sind je nach Zahl der angegebenen Kohlenstoffatome beispielsweise folgende Gruppen zu verstehen: Methyl- Ethyl, Propyl, Butyl, Pentyl, Hexyl, Heptyl, Octyl, Nonyl oder Decyl, sowie die Isomeren, wie beispielsweise Isopropyl, Isobutyl, tert.-Butyl oder Isopentyl. Haloalkyl steht für einen einfach bis perhalogenierten Alkylsubstituenten, wie beispielsweise $CHCl_2$, $CHF_2$, $CH_2Cl$, $CCl_3$, $CF_3$, $CH_2F$, $CH_2CH_2Cl$, $CHBr_2$. Unter Halogen soll hier und im folgenden Fluor, Chlor, Brom oder Jod, vorzugsweise Fluor, Chlor oder Brom, verstanden werden. Mono-bis tetracyclische, aliphatische Gruppen sind beispielsweise Cyclopropyl, Cyclobutyl, Cyclopentyl, Cyclohexyl, Decalin, Hydrindan, Bicycloheptan, Bicyclooctan, Norbornan, Bornan oder Adamantyl. Diese cycloaliphatischen Gruppen sind vorzugsweise unsubstituiert oder durch Methyl ein- oder mehrfach substituiert.Alkenyl steht für einen mindestens durch eine C=C-Doppelbindung charakterisierten aliphatischen, acyclischen Kohlenwasserstoffrest, wie beispielsweise für Vinyl, Propenyl-(1), Allyl, Butenyl-(1), Butenyl-(2) oder Butenyl-(3). Haloalkenyl bedeutet dementsprechend einen derartigen Alkenylrest, der ein- oder mehrfach halogeniert ist. Alkinyl steht für eine gerade oder verzweigte Kohlenstoffkette, die durch mindestens eine C≡C Dreifachbindung charakterisiert ist. Typische Vertreter sind beispielsweise Ethinyl, Propionyl-(1), Propargyl oder Butinyl-(1). Alkoxyalkyl steht für eine unverzweigte oder verzweigte, durch ein Sauerstoffatom unterbrochene Alkylgruppe, beispielsweise für $CH_2OCH_3$, $CH_2CH_2OCH_3$, $CH_2CH(CH_3)OCH_3$, $CH_2OC_2H_5$, $C(CH_3)_2OCH_3$, $CH_2OC_3H_{7-i}$ oder $CH_2CH_2CH_2OCH_3$.

Als substituiertes Phenyl kommen beispielsweise 2,4-Dichlorphenyl, 2,3,6-Trichlorphenyl, p-Bromphenyl, 2,4-Xylyl, 3-Nitrophenyl, 4-Chlor-2-methylphenyl, 4-Methyl-2-methoxyphenyl, 2,4,6-Trimethylphenyl oder p-Methylthiophenyl in Betracht.

Als Beispiele für R sind zu nennen Wasserstoff, tert.Butyl, Chlormethyl, Trifluormethyl, Trichlormethyl, Trichlorethyl, Trichlor-tert.Butyl, 1,2,2,2-Tetrachlorethyl, 1,3,3,3-Tetrachlorpropyl, 3-Chlorpropyl, Ethenyl, Propenyl, Propinyl, Methoxymethyl, Isopropoxymethyl, 1-Methyl-1-methoxyethyl, 2,2-Dimethylvinyl, 1,2,2-Trichlorvinyl, 1,3,3,3-Tetrachlorpropyl, 1,1-Dichlor-2,2,2-trifluorethyl, 1,3-Pentadienyl, Ethinyl, 1-Propinyl, 1-Butinyl, Cyclopropyl, 2,2-Dimethylcyclopropyl, 1-Methylcyclopropyl, 2,2-Dimethyl-3-(2,2-dichlorvinyl)-cyclopropyl, Cyclobutyl, Cyclopentyl, Cyclohexyl, Adamantyl, Phenyl, Benzyl, p-Tolyl, p-Chlorphenyl, 2,6-Dichlorphenyl oder 2,4-Dinitrophenyl oder 4-Fluorphenoxymethyl.

Verbindungen der Formel I, worin $R_1$ Wasserstoff bedeutet, sind bevorzugt. Acyl- und Silylgruppen als $R_1$ sind im allgemeinen als Schutzgruppen aufzufassen. Bevorzügte Acylgruppen sind diejenigen, in denen $R_5$ $C_1$-$C_6$-Alkyl, $C_1$-$C_6$-Haloalkyl oder unsubstituiertes oder durch Halogen, $C_1$-$C_3$-Alkyl, $CF_3$ oder Nitro substituiertes Phenyl bedeutet. Besonders bevorzugt ist die Acetylgruppe. Als Acylverbindung ist beispielsweise 5-O-Acetyl-13$\beta$-Formyloxy-milbemycin $A_4$ zu nennen. Geeignete Silylgruppen sind beispielsweise Trimethylsilyl, Diphenyl-tert.butylsilyl, bis(Isopropyl)methylsilyl oder Triphenylsilyl und vorzugsweise tert.Butyldimethylsilyl.

Verbindungen, worin $R_2$ sek.Butyl darstellt, sollen hier und im folgenden gleichfalls zu den Milbemycin-Derivaten gerechnet werden, obwohl sie nach der üblichen Systematik von Avermectin-Derivaten abgeleitet sind. Avermectin-Aglykone (mit einer OH-Gruppe in 13$\alpha$-Position) lassen sich jedoch gemäss US-PS 4,173,571 in Milbemycin-Homologe überführen.

In natürlich vorkommenden Milbemycinen ($R_1$ = H; $R_2$ = $CH_3$, $C_2H_5$ oder iso $C_3H_7$) ist die 13-Position anstelle der Estergruppe der erfindungsgemässen Verbindungen der Formel I ausschliesslich mit Wasserstoff besetzt, wie die nachstehende Formel zeigt:

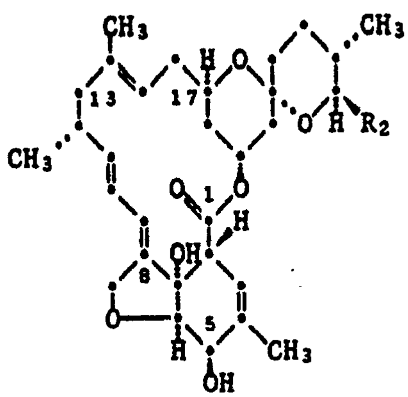

| | | |
|---|---|---|
| $R_2$ | = $CH_3$ | Milbemycin $A_3$ |
| $R_2$ | = $C_2H_5$ | Milbemycin $A_4$ |
| $R_2$ | = iso$C_3H_7$ | Milbemycin D |
| $R_2$ | = sec.$C_4H_9$ | 13-Deoxi-22,23-dihydro-C-076-Bla-aglycon. |

Bei Avermectinen dagegen steht in der 13-Position ein $\alpha$-L-Oleandrosyl-$\alpha$-L-oleandrose-Rest, der über Sauerstoff in $\alpha$-Konfiguration mit dem Makrolid-Molekül verknüpft ist. Avermectine unterscheiden sich strukturell ausserdem durch eine 23-OH-Gruppe oder $\Delta^{22,23}$-Doppelbindung und in der Regel durch einen Substituenten $R_2$ = sek.$C_4H_9$ von den Milbemycinen. Durch Hydrolyse des Zucker-Restes der Avermectine gelangt man leicht zu den entsprechenden Avermectin-Aglykonen, die eine in Nachbarstellung zu einer C=C-Doppelbindung befindliche 13$\alpha$-Hydroxy-Gruppe besitzen. Die Avermectin-Aglykone sind, wie vorstehend angegeben, in die Milbemycin-Homologen umwandelbar. Bei den Milbemycin-Derivaten der vorliegenden Anmeldung liegt die $\Delta^{22,23}$-Doppelbindung stets in hydrierter Form vor und der Substituent in 13-Position ist immer β-ständig.

Folgende Untergruppen von Verbindungen der Formel I sind auf Grund ihrer ausgeprägten parasitiziden und insektiziden Wirkung besonders bevorzugt:

Gruppe Ia: Verbindungen der Formel I, worin $R_1$ Wasserstoff darstellt, $R_2$ für Methyl, Ethyl, Isopropyl oder sek.Butyl steht und R folgende Bedeutungen hat:

- Wasserstoff, tert.Bütyl oder durch 1 bis 4 Halogenatome oder $C_1$-$C_4$-Alkoxy substituiertes $C_1$-$C_6$-Alkyl, $C_2$-$C_4$-Alkenyl, $C_2$-$C_4$-Alkinyl oder $C_3$-$C_6$-Cycloalkyl;
- unsubstituiertes oder ein bis dreifach durch Halogen, $C_1$-$C_4$-Alkyl, $C_1$-$C_4$-Alkoxy, $C_1$-$C_4$-Alkylthio oder Nitro substituiertes Phenyl;

Gruppe Ib: Verbindungen der Formel I, worin $R_1$ Wasserstoff darstellt, $R_2$ für Methyl, Ethyl, Isopropyl oder sek.Butyl steht und R folgende Bedeutungen hat:

- Wasserstoff, tert.Bütyl oder durch 1 bis 4 Chlor- oder Fluoratome oder Methoxy substituiertes $C_1$-$C_4$-Alkyl, $C_2$-$C_3$-Alkenyl, $C_2$-$C_3$-Alkinyl oder $C_3$-$C_6$-Cycloalkyl;
- unsubstituiertes oder durch Chlor, Fluor, $C_1$-$C_2$-Alkyl, $C_1$-$C_2$-Alkoxy, $C_1$-$C_2$-Alkylthio oder Nitro substituiertes Phenyl;

Gruppe Ic: Verbindungen der Formel I, worin $R_1$ Wasserstoff darstellt, $R_2$ für Methyl oder Ethyl steht und R folgende Bedeutungen hat:

- Wasserstoff, tert.Bütyl oder durch 1 bis 4 Chlor- oder Fluoratome oder Methoxy substituiertes $C_1$-$C_4$-Alkyl, $C_2$-$C_3$-Alkenyl, $C_2$-$C_3$-Alkinyl oder $C_3$-$C_6$-Cycloalkyl;
- unsubstituiertes oder durch Chlor, Fluor, $C_1$-$C_2$-Alkyl, $C_1$-$C_2$-Alkoxy, $C_1$-$C_2$-Alkylthio oder Nitro substituiertes Phenyl;

Gruppe Id: Verbindungen der Formel I, worin $R_1$ Wasserstoff darstellt, $R_2$ für Isopropyl oder sek.Butyl steht und R folgende Bedeutungen hat:

- Wasserstoff, tert.Bütyl oder durch 1 bis 4 Chlor- oder Fluoratome oder Methoxy substituiertes $C_1$-$C_4$-Alkyl, $C_2$-$C_3$-Alkenyl, $C_2$-$C_3$-Alkinyl oder $C_3$-$C_6$-Cycloalkyl;
- unsubstituiertes oder durch Chlor, Fluor, $C_1$-$C_2$-Alkyl, $C_1$-$C_2$-Alkoxy, $C_1$-$C_2$-Alkylthio oder Nitro substituiertes Phenyl;

Gruppe Ie: Verbindungen der Formel I, worin $R_1$ Wasserstoff darstellt, $R_2$ für Methyl, Ethyl, Isopropyl oder sek.Butyl steht und R folgende Bedeutungen hat:

- Wasserstoff, tert.Bütyl oder durch 1 bis 3 Chloratome oder Fluoratome oder Methoxy substituiertes $C_1$-$C_4$-Alkyl, $C_2$-$C_3$-Alkenyl, $C_2$-$C_3$-Alkinyl oder $C_3$-$C_6$-Cycloalkyl;
- unsubstituiertes oder durch Chlor, Fluor, $C_1$-$C_2$-Alkyl, $C_1$-$C_2$-Alkoxy, $C_1$-$C_2$-Alkylthio oder Nitro substituiertes Phenyl;

Gruppe If: Verbindungen der Formel I, worin $R_1$ Wasserstoff darstellt, $R_2$ für Methyl oder Ethyl steht und R folgende Bedeutungen hat:

- Wasserstoff, tert.Bütyl oder durch 1 bis 3 Chloratome oder Fluoratome oder Methoxy substituiertes $C_1$-$C_4$-Alkyl, $C_2$-$C_3$-Alkenyl, $C_2$-$C_3$-Alkinyl oder $C_3$-$C_6$-Cycloalkyl.

Gruppe Ig: Verbindungen der Formel I, worin $R_1$ Wasserstoff darstellt, $R_2$ für Isopropyl oder sek.Butyl steht und R folgende Bedeutungen hat:

- Wasserstoff, tert.Bütyl oder durch 1 bis 3 Chloratome oder Fluoratome oder Methoxy substituiertes $C_1$-$C_4$-Alkyl, $C_2$-$C_3$-Alkenyl, $C_2$-$C_3$-Alkinyl oder $C_3$-$C_6$-Cycloalkyl.

Gruppe Ih: Verbindungen der Formel I, worin $R_1$ Wasserstoff darstellt, $R_2$ für Ethyl oder Isopropyl steht und R folgende Bedeutungen hat: Wasserstoff, tert.Bütyl, $C_1$-$C_8$-Alkyl, welches durch $C_1$-$C_4$-Alkoxy oder ein- bis dreifach halogeniertes Phenoxy monosubstituiert oder durch 1 bis 5 Halogenatome substituiert ist, eine unsubstituierte oder durch Substituenten der Gruppe bestehend aus $C_1$-$C_4$-Alkyl und halogeniertem $C_2$-$C_4$-Alkenyl ein- oder mehrfach substituierte, mono- bis tetracyclische, aliphatische Gruppe mit insgesamt 3 bis 10 Kohlenstoffatomen im Ring oder Ringsystem, ein- bis dreifach halogeniertes $C_2$-$C_4$-Alkenyl, $C_3$-$C_4$-Alkinyl oder ein- bis dreifach durch Substituenten der Gruppe bestehend aus Halogen, $C_1$-$C_4$-Alkyl und Nitro substituiertes Phenyl.

Gruppe Ii: Verbindungen der Formel I, worin $R_1$ Wasserstoff darstellt, $R_2$ für Ethyl oder Isopropyl steht und R folgende Bedeutungen hat: Wasserstoff, tert.Bütyl, $C_1$-$C_8$-Alkyl, welches durch Substituenten der Gruppe bestehend aus Chlor und Fluor ein- bis dreifach substituiert ist, Fluorphenoxymethyl, unsubstituiertes oder durch eine Methylgruppe substituiertes $C_3$-$C_4$-Cycloalkyl, Adamantyl, Trichlorvinyl oder Monochlorphenyl.

Besonders bevorzugte 5-Hydroxy-Derivate der Formel I sind beispielsweise:

13β-Formyloxy-milbemycin D

13β-Pivaloyloxy-milbemycin D

13β-Formyloxy-milbemycin $A_3$

13β-Pivaloyloxy-milbemycin $A_3$
13β-Formyloxy-milbemycin $A_4$
13β-Pivaloyloxy-milbemycin $A_4$.
13β-(2'-Methoxy-2'-methylpropionyloxy)-milbemycin D
13β-(2'-Methoxy-2'-methylpropionyloxy)-milbemycin $A_4$
13β-Trichloracetoxy-milbemycin D
13β-(4'-Chlor-butanoyloxy)milbemycin D
13β-Trichloracryloyloxy-milbemycin D
13β-Cyclopropancarbonyloxy-milbemycin D
13β-Cyclobutancarbonyloxy-milbemycin D
13β-(3'-Chlor-2'2'-dimethyl-propionyloxy)-milbemycin $A_4$
13β-(3'-Chlor-2'2'-dimethyl-propionyloxy)-milbemycin D
13β-(1'-Methyl-cyclopropancarbonyloxy)-milbemycin $A_4$
13β-(1'-Methyl-cyclopropancarbonyloxy)-milbemycin D
13β-(1-Adamantancarbonyloxy)-milbemycin D
13β-(p-Fluorphenoxyacetoxy)-milbemycin $A_4$
13β-(2'-Chlor-2'-methyl-propionyloxy)-milbemycin $A_4$
13β-(2',2'-Dichlorpropionyloxy)-milbemycin $A_4$
13β-(p-Chlorbenzoyloxy)-milbemycin $A_4$
13β-(3',3',3'-Trifluorpropionyloxy)-milbemycin $A_4$
13β-Chloracetoxy-milbemycin $A_4$
13β-(2'-Chlor-3',3',3'-Trifluorpropionyloxy)-milbemycin D
13β-(3',3',3'-Trifluorpropionyloxy)-milbemycin D.

Bevorzugte an der 5-Hydroxygruppe mit einer Schutzgruppe versehene Verbindungen der Formel I sind z.B.:

5-O-tert.Butyldimethylsilyl-13β-Formyloxy-milbemycin D
5-O-tert.Butyldimethylsilyl-13β-Pivaloyloxy-milbemycin D
5-O-tert.Butyldimethylsilyl-13β-Formyloxy-milbemycin $A_3$
5-O-tert.Butyldimethylsilyl-13β-Pivaloyloxy-milbemycin $A_3$
5-O-tert.Butyldimethylsilyl-13β-Formyloxy-milbemycin $A_4$
5-O-tert.Butyldimethylsilyl-13β-Pivaloyloxy-milbemycin $A_4$
5-O-tert.Butyldimethylsilyl-13β-(2'-Methoxy-2'-methylpropionyloxy)-milbemycin D
5-O-tert.Butyldimethylsilyl-13β-(2'-Methoxy-2'-methylpropionyloxy)-milbemycin $A_4$
5-O-tert.Butyldimethylsilyl-13β-Trichloracetoxy-milbemycin D
5-O-tert.Butyldimethylsilyl-13β-(4'-Chlor-butanoyloxy)milbemycin D
5-O-tert.Butyldimethylsilyl-13β-Trichloracryloyloxy-milbemycin D
5-O-tert.Butyldimethylsilyl-13β-Cyclopropancarbonyloxy-milbemycin D
5-O-tert.Butyldimethylsilyl-13β-Cyclobutancarbonyloxy-milbemycin D
5-O-tert.Butyldimethylsilyl-13β-(3'Chlor-2'2'-dimethyl-propionyloxy)-milbemycin $A_4$
5-O-tert.Butyldimethylsilyl-13β-(3'-Chlor-2'2'-dimethyl-propionyloxy)-milbemycin D
5-O-tert.Butyldimethylsilyl-13β-(1'-Methyl-cyclopropancarbonyloxy)milbemycin $A_4$
5-O-tert.Butyldimethylsilyl-13β-(1'-Methyl-cyclopropancarbonyloxy)milbemycin D
5-O-tert.Butyldimethylsilyl-13β-(1-Adamantancarbonyloxy)-milbemycin D
5-O-tert.Butyldimethylsilyl-13β-(p-Fluorphenoxyacetoxy)-milbemycin $A_4$
5-O-tert.Butyldimethylsilyl-13β-(2'-Chlor-2'-methyl-propionyloxy)milbemycin $A_4$
5-O-tert.Butyldimethylsilyl-13β-(2',2'-Dichlorpropionyloxy)milbemycin $A_4$
5-O-tert.Butyldimethylsilyl-13β-(p-Chlorbenzoyloxy)-milbemycin $A_4$
5-O-tert.Butyldimethylsilyl-13β-(3',3',3'-Trifluorpropionyloxy)milbemycin $A_4$
5-O-tert.Butyldimethylsilyl-13β-Chloracetoxy-milbemycin $A_4$
5-O-tert.Butyldimethylsilyl-13β-(2'-Chlor-3',3',3'-Trifluorpropionyloxy)-milbemycin D
5-O-tert.Butyldimethylsilyl-13β-(3',3',3'-Trifluorpropionyloxy)milbemycin D.

Von besonderem Interesse sind Verbindungen der Formel I, in denen R für tert.Butyl steht und $R_1$ und $R_2$ die für Formel I angegebenen Bedeutungen haben, und von diesen insbesondere diejenigen Verbindungen, in denen $R_1$ Wasserstoff und $R_2$ Ethyl oder Isopropyl bedeutet.

Die vorliegende Erfindung betrifft auch Verfahren, die es erlauben, in der 13-Position von Milbemycin- oder 13-Deoxy-22,23-dihydro-Avermectinaglykon-Derivaten eine β-Acyloxy-Gruppe gezielt einzuführen und damit zu den hochwirksamen neuen Parasitiziden und Insektiziden der Formel I zu gelangen, die gleichzeitig auch für weitere Derivatisierungen verwendet werden können.

Zur Herstellung von Estern der Formel I, worin RCOO- in der $\beta$-Position steht, geht man von einer Verbindung der Formel II

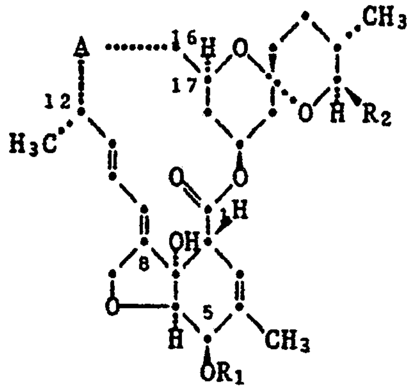

(II)

worin A für eine der Gruppen a oder b

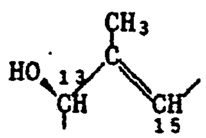

oder

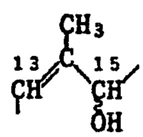

(a) [= 13β-Hydroxy-$\Delta^{14,15}$]

(b) [= $\Delta^{13,14}$-15-hydroxy]

steht, worin $R_1$ und $R_2$ die für Formel I angegebenen Bedeutungen haben, aus.
Eine Verbindung der Formel II, in welcher $R_1$ eine Schutzgruppe bedeutet und $R_2$ die unter Formel I angegebenen Bedeutungen besitzt, wird mit einem zur Einführung einer 13$\beta$-Estergruppe geeigneten Reagens behandelt. Danach kann die $R_1$-Schutzgruppe, sofern eine freie 5-Hydroxy-Verbindung erwünscht ist, hydrolytisch abgespalten werden.

Verbindungen der Formel II, worin A für die Gruppe a steht, werden hier und im folgenden mit IIa und die Verbindungen mit der Gruppe b mit IIb bezeichnet.

Zur Einführung der 13$\beta$-Estergruppe in Verbindungen der Formel II geeignete Reagenzien sind beispielsweise:

a) Säuren der Formel III

RCOOH (III)

b) Säureamide der Formel IV

$RCON(Alkyl)_2$ (IV)

worin die Alkylreste 1 bis 4 Kohlenstoffatome umfassen und vorzugsweise Methyl bedeuten,

c) Säurehalogenide der Formel V

RCOhal (V),

worin hal Halogen, bevorzugt Chlor oder Brom, bedeutet,

d) Säureanhydride der Formel VI

$(RCO)_2O$ (VI),

wobei sich die Säuren und Säureamide für alle Verbindungen der Formel II eignen, vorzugsweise aber für Verbindungen der Formel IIb verwendet werden, während Säurehalogenide und säureanhydride bei Verbindungen der Formel IIa zum Einsatz gelangen.

In den vorstehend angegebenen Formeln III-VI besitzt R die unter Formel I angegebenen Bedeutungen.

Die Reaktionen zur Herstellung von Verbindungen der Formel I werden vorzugsweise mit Verbindungen der Formeln IIa oder IIb durchgeführt, deren reaktive 5-Hydroxy-Gruppe geschützt ist.

Verbindungen der Formel I, worin $R_1$ eine Schutzgruppe darstellt, lassen sich durch einfache, beispielsweise hydrolytische Abspaltung der Schutzfunktion in die hochaktiven freien 5-Hydroxy-derivate ($R_1 = H$) überführen und haben somit Zwischenprodukte-Charakter. Im übrigen wird der biologische Wert dieser Verbindungen durch die Schutzgruppe nicht gemindert.

Das Verfahren wird im allgemeinen in einem reaktions-inerten Lösungsmittel oder in einem der beteiligten Reaktanden, sofern diese flüssig sind, durchgeführt. Geeignete Lösungsmittel sind beispielsweise: Ether und etherartige Verbindungen wie Dialkylether (Diethylether, Diisopropylether, tert.-Butylmethylether, Dimethoxyethan, Dioxan, Tetrahydrofuran oder Anisol); halogenierte Kohlenwasserstoffe wie Chlorbenzol, Methylenchlorid, Ethylenchlorid, Chloroform, Tetrachlorkohlenstoff oder Tetrachlorethylen; oder Sulfoxyde wie Dimethylsulfoxyd; ferner aromatische oder aliphatische Kohlenwasserstoffe, wie Benzol, Toluol, Xylole, Petrolether, Ligroin oder Cyclohexan. In manchen Fällen kann es von Vorteil sein, wenn die Reaktionen unter Schutzgasatmosphäre (beispielsweise Argon, Helium oder Stickstoff) und/oder in absoluten Lösungsmitteln durchgeführt werden. Gewünschtenfalls können die Endprodukte auf übliche Art und Weise gereinigt werden, beispielsweise durch Waschen, Digerieren, Extraktion, Umkristallisation oder Chromatographie.

Die Umsetzung von Verbindungen der Formel IIa oder IIb mit Säuren oder Säuresmiden der Formeln III bzw. IV erfolgt in Gegenwart von Orthoestern sowie in Gegenwart katalytischer Mengen einer weiteren Säure. Als dafür geeignete Säuren können Protonensäuren oder Lewis-Säuren eingesetzt werden. Beispiele derartiger Säuren sind anorganische Säuren: Halogenwasserstoffsäure wie Chlorwasserstoffsäure, Bromwasserstoffsäure oder Jodwasserstoffsäure, Perchlorsäure sowie Schwefelsäure und organische Säuren wie Essigsäure, Trifluoressigsäure, Trichloressigsäure, Propionsäure, Oxalsäure, Ameisensäure, Benzolsulfonsäure, p-Toluolsulfonsäure oder Methansulfonsäure sowie Lewis-Säuren, wie $BF_3$, $AlCl_3$ oder $ZnCl_2$. Besonders bevorzugt sind p-Toluolsulfonsäure (auch als TsOH bezeichnet) und Schwefelsäure.

Die bei dieser Reaktion benötigten Orthoester haben die Formel VII

$R_3C(OR_4)_3$ (VII),

worin $R_3$ Wasserstoff oder $C_1$-$C_4$-Alkyl, bevorzugt Methyl, und $R_4$ $C_1$-$C_4$-Alkyl, bevorzugt Methyl oder Ethyl bedeuten.

Bei Verwendung von Säuren oder Säureamiden der Formeln III bzw. IV zur Herstellung von Verbindungen der Formel I liegen die Reaktionstemperaturen im allgemeinen im Bereich von 0° bis 150° C, bevorzugt von 20° bis 130° C.

Die Reaktion von Verbindungen der Formel IIa mit Säurehalogeniden oder Säureanhydriden der Formeln V bzw. VI wird normalerweise in den obengenannten reaktionsinerten Lösungsmitteln im allgemeinen bei Temperaturen von -20° bis 100° C, vorzugsweise von 0° bis 70° C, durchgeführt. Zum Abfangen der dabei als Nebenprodukt gebildeten Säuren findet die Reaktion zweckmässigerweise in Gegenwart eines Neutralisationsmittels statt.

Als solche kommen organische Basen in Betracht, beispielsweise tertiäre Amine wie Trialkylamine (Trimethylamin, Triethylamin, Diisopropylmethylamin oder Tripropylamin), Pyridin und Pyridinbasen (4-Dimethylaminopyridin oder 4-Pyrrolidylaminopyridin), bevorzugt ist Pyridin. Das Neutralisationsmittel wird üblicherweise in mindestens äquimolarer Menge in bezug auf die Ausgangsstoffe eingesetzt.

Bei der Reaktion von Verbindungen der Formel IIb mit Säuren oder Säureamiden der Formeln III bzw. IV in Gegenwart von Orthoestern der Formel VII sowie einer katalytisch wirksamen Säure können neben den Verbindungen der Formel I als Nebenprodukte auch Verbindungen der Formel VIII

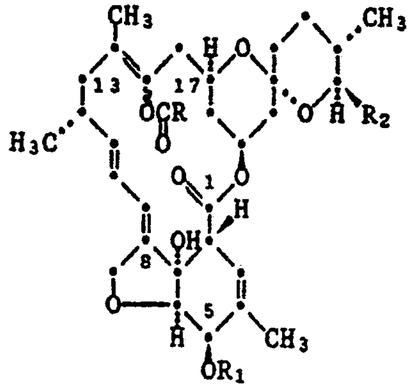

(VIII)

worin $R_1$, $R_2$ und R die unter Formel I angegebenen Bedeutungen besitzen, gebildet werden.

Die erhaltenen Reaktionsprodukte lassen sich durch übliche Trennungsmethoden, wie beispielsweise durch fraktionierte Kristallisation oder durch Chromatographie, voneinander trennen. Unter Chromatographie werden Säulen-, Dickschicht- oder Dünnschicht-Chromatographie sowie bevorzugt Hochdruck-Flüssigkeits-Chromatographie an mineralischen Trägermaterialien wie Silicagel oder an organischen Austauscherharzen verstanden.

In einer anderen Verfahrensvariante setzt man Säuren der Formel III als solche ein und führt die Reaktion in Gegenwart wasserabspaltender Reagenzien durch. Beispielsweise arbeitet man in Anwesenheit von Dicyclohexylcarbodiimid und Pyridin oder in Gegenwart von Azodicarbonsäuredialkylester und Triphenylphosphin.

Die zur Gewinnung der erfindungsgemässen Verbindungen der Formel I mittels der hierin beschriebenen Verfahren benötigten Ausgangsverbindungen der Formel IIa werden erhalten durch Reaktion von Verbindungen der Formel IIb mit Chromat-, Halochromat- oder Dichromat-Ionen, insbesondere mit Pyridiniumdichromat [ $= (Pyr)_2^{+} Cr_2O_7$ ] oder mit Pyridiniumchlorochromat [ $= (Pyr)^{+} ClCrO_3$ ].

Es werden inerte wasserfreie, vorzugsweise polare Lösungsmittel, z.B. Dimethylformamid (=DMF) verwendet. Die Reaktion wird bei Temperaturen von -10° C bis +60° C, bevorzugt +10° C bis +40° C, durchgeführt.

Die Verbindungen der Formel IIb [= $\Delta^{13,14}$-15-Hydroxy] lassen sich aus 14,15-Epoxy-Milbemycinen der Formel IX

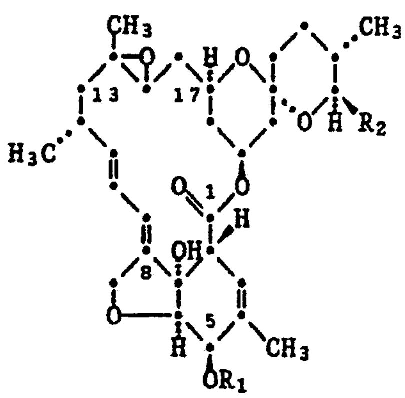

(IX)

worin $R_1$ und $R_2$ die für die Formel I genannten Bedeutungen haben, mit Hilfe des Komplex-Reagenzes $[HN_3]_m/[Al(Ethyl)_3]_n$, worin m und n unabhängig voneinander die Zahl 1 oder 2 oder einen Zahlenwert zwischen 1 und 2 darstellen, in inerten trockenen Lösungsmitteln im Temperaturbereich von 30° bis +10° C, vorzugsweise 20° bis -5° C, herstellen.

Als inerte Lösungsmittel kommen dafür vorzugsweise aliphatische und aromatische Kohlenwasserstoffe wie Benzol, Toluol, Xylol, Petrolether; Ether wie Diethylether, tert.Butylmethylether, Tetrahydrofuran, Dioxan oder Anisol in Frage.

Die Reaktion wird vorteilhaft unter Schutzgas, wie Stickstoff oder Argon, durchgeführt.

Stickstoffwasserstoffsäure $HN_3$ lässt sich in statu nascendi in den $[HN_3]_m/[Al(Et)_3]_n$-Komplex überführen,

indem man im vorgesehenen trockenen Lösungsmittel oder Lösungsmittel-Gemisch Na-Azid suspendiert und daraus mit einer stärkeren Säure, beispielsweise $H_2SO_4$ (bevorzugt Oleum, um absolut trockene Reaktionsbedingungen zu gewährleisten), $HN_3$ in der Lösung in Freiheit setzt. $Al(Et)_3$ sollte in der Lösung bereits vorliegen oder kurz danach zugegeben werden. Die zur Reaktion vorgesehene Epoxy-Verbindung kann gleichfalls bereits vorliegen oder zu einem geeigneten Zeitpunkt zur Lösung dosiert werden.

Die für die Verbindungen IIb verwendeten Ausgangsverbindungen der Formel IX lassen sich leicht herstellen durch Epoxydierung der aus der US-PS 3,950,360 bekanntgewordenen und ursprünglich als "Antibiotika B-41-A", später als "Milbemycin-A"-Verbindungen und der aus der US-PS 4,346,171 bekanntgewordenen und als "B-41-D" oder "Milbemycin-D" bezeichneten Verbindungen sowie der aus der US-PS 4,173,571 und aus Tetrahedron Letters, Vol. 24, No. 48, pp. 5333-5336 (1983) bekanntgewordenen 13-Deoxy-22,23-dihydro-Avermectine ($R_2$ = sec.Butyl) der Formel

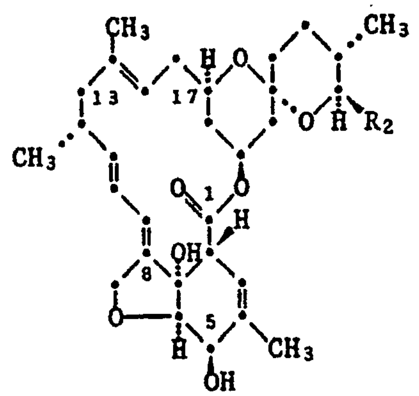

| | |
|---|---|
| $R_2 = CH_3$ | Milbemycin $A_3$ |
| $R_2 = C_2H_5$ | Milbemycin $A_4$ |
| $R_2 = isoC_3H_7$ | Milbemycin D |
| $R_2 = sec.C_4H_9$ | 13-Deoxy-22,23-dihydro-C-076-Bla-aglycon. |

Die Epoxidierung wird in einer Lösungsmittelphase im Temperaturbereich von -10° bis +20° C, vorzugsweise -5° bis +5° C, durchgeführt.

Die Epoxidierung wird mit Persäuren wie beispielsweise Peressigsäure, Trifluorperessigsäure, Perbenzoesäure oder Chlorperbenzoesäure durchgeführt.

Die 13β-Hydroxy-$\Delta^{14,15}$-Verbindungen der Formel IIa lassen sich aus Verbindungen der Formel IIb, worin $R_1$ für eine Schutzgruppe steht, durch Reaktion mit Pyridiniumdichromat [= $(Pyr)_2^+Cr_2O_7$] herstellen. Man arbeitet hierbei in Dimethylformamid und bei Temperaturen zwischen ca. -10° und +60° C. Die $R_1$-Schutzgruppe wird, sofern gewünscht, anschliessend hydrolytisch abgespalten.

Durch Acylierung oder Silylierung der 5-OH-Gruppe werden alle jene Derivate der Formeln I, IIa, IIb und IX hergestellt, bei denen $R_1$ eine andere Bedeutung als Wasserstoff ($R_1$ = OH-Schutzgruppe) hat. Die Einführung der Acylgruppe erfolgt üblicherweise mit den entsprechenden Acylhalogeniden oder Acylanhydriden und wird vorzugsweise zur Einführung der eingangs definierten $R_5C(O)$- Gruppe benutzt. Zur Silylierung verwendet man zweckmässigerweise ein Silan der Formel $Y\text{-}Si(R_6)(R_7)(R_8)$, worin $R_6$, $R_7$ und $R_8$ einen der eingangs genannten Reste darstellen, wobei der Begriff Acylhalogenid für Acylchlorid oder Acylbromid steht und wobei Y eine Silylabgangsgruppe bedeutet. Zu den Silylabgangsgruppen Y zählen beispielsweise Bromid, Chlorid, Cyanid, Azid, Acetamid, Trifluoracetat oder Trifluormethansulfonat.

5-O-Acylierungen und 5-O-Silylierungen werden in wasserfreiem Milieu, vorzugsweise in inerten Lösungsmitteln und besonders bevorzugt in aprotischen Lösungsmitteln durchgeführt. Die Reaktion läuft vorteilhaft im Temperaturbereich von 0° bis +80° C, bevorzugt bei +10° bis +40° C, ab. Vorzugsweise wird eine organische Base zugegeben. Es kommen als solche beispielsweise tertiäre Amine wie Triethylamin, Triethylendiamin, Triazol und bevorzugt Pyridin, Imidazol oder 1,8-Diazabicyclo[5.4.0]-undec-7-en (DBU) in Frage.

Die Entfernung dieser Silyl- und Acylreste $R_1$ in der 5-Position geschieht durch selektive milde Hydrolyse (→ $R_1$ = H) beispielsweise mit Arylsulfonsäure in alkoholischer Lösung oder nach einer anderen dem Fachmann geläufigen Methode.

Das beschriebene Verfahren zur Herstellung der Verbindungen der Formel I ist in allen Teilschritten ein Bestandteil vorliegender Erfindung.

Die Verbindungen der Formel I eignen sich ausgezeichnet zur Bekämpfung von Schädlingen, welche

Tiere und Pflanzen befallen,darunter insbesondere von tierparasitären Ekto-parasiten. Zu letzteren zählen unter der Ordnung Acarina insbesondere Schädlinge der Familien Ixodidae, Dermanyssidae, Sarcoptidae, Psoroptidae; die Ordnungen Mallophaga; Siphonaptera, Anoplura (z.B. Familie der Haemotopinidae); unter der Ordnung Diptera insbesondere Schädlinge der Familien Muscidae, Calliphoridae, Oestridae, Tabanidae, Hippoboscidae, Gastrophilidae.

Die Verbindungen I sind auch einsetzbar gegen Hygiene-Schädlinge, insbesondere der Ordnungen Diptera mit den Familien Sarcophagidae, Anophilidae, Culicidae; der Ordnung Orthoptera, der Ordnung Dictyoptera (z.B. Familie Blattidae) und der Ordnung Hymenoptera (z.B. Familie Formicidae).

Die Verbindungen I besitzen auch nachhaltige Wirksamkeit bei pflanzenparasitären Milben und Insekten. Bei Spinnmilben der Ordnung Acarina sind sie wirksam gegen Eier, Nymphen und Adulte von Tetranychidae (Tetranychus spp. und Panonychus spp.).

Hohe Aktivität besitzen sie bei den saugenden Insekten der Ordnung Homoptera, insbesondere gegen Schädlinge der Familien Aphididae, Delphacidae, Cicadellidae, Psyllidae, Coccidae, Diaspididae und Eriophydidae (z.B. die Rostmilbe auf Citrusfrüchten): der Ordnungen Hemiptera; Heteroptera und Thysanoptera; sowie bei den pflanzenfressenden Insekten der Ordnungen Lepidoptera; Coleoptera; Diptera und Orthoptera.

Sie sind ebenfalls als Bodeninsektizid gegen Schädlinge im Erdboden geeignet.

Die Verbindungen der Formel I sind daher gegen alle Entwicklungsstadien saugender und fressender Insekten an Kulturen wie Getreide, Baumwolle, Reis, Mais, Soja, Kartoffeln, Gemüse, Früchten, Tabak, Hopfen, Citrus, Avocados und anderen wirksam.

Die Verbindungen der Formel I sind auch wirksam gegen Pflanzen-Nematoden der Arten Meloidogyne, Heterodera, Pratylenchus, Ditylenchus, Radopholus, Rhizoglyphus und andere.

Ferner sind die Verbindungen gegen Helminthen wirksam, unter denen die endoparasitären Nematoden die Ursache schwerer Erkrankungen an Säugetieren und Geflügel sein können, z.B. an Schafen, Schweinen, Ziegen, Rindern, Pferden, Eseln, Hunden, Katzen, Meerschweinchen, Ziervögeln. Typische Nematoden dieser Indikation sind: Haemonchus, Trichostrongylus, Ostertagia, Nematodirus, Cooperia, Ascaris, Bunostomum, Oesophagostomum, Chabertia, Trichuris, Strongylus, Trichonema, Dictyocaulus, Capillaria, Heterakis, Toxocara, Ascaridia, Oxyuris, Ancylostoma, Uncinaria, Toxascaris und Parascaris. Der besondere Vorteil der Verbindungen der Formel I ist ihre Wirksamkeit gegen solche Parasiten, die gegen Wirkstoffe auf Benzimidazol-Basis resistent sind.

Gewisse Spezies der Arten Nematodirus, Cooperia und Oesophagostomum greifen den Intestinaltrakt des Wirtstiers an, während andere der Arten Haemonchus und Ostertagia im Magen und solche der Art Dictyocaulus im Lungengewebe parasitieren. Parasiten der Familien Filariidae und Setariidae finden sich im internen Zellgewebe und den Organen, z.B. dem Herzen, den Blutgefässen, den Lymphgefässen und dem subcutanen Gewebe. Hier ist vor allem der Herzwurm des Hundes, Dirofilaria immitis, zu nennen. Die Verbindungen der Formel I sind gegen diese Parasiten hoch wirksam.

Weiter sind die Verbindungen der Formel I zur Bekämpfung von humanpathogenen Parasiten geeignet, unter denen als typische, im Verdauungstrakt vorkommende Vertreter solche der Arten Ancylostoma, Necator, Ascaris, Strongyloides, Trichinella, Capillaria, Trichuris und Enterobius zu nennen sind. Wirksam sind die Verbindungen vorliegender Erfindung auch gegen Parasiten der Arten Wuchereria, Brugia, Onchocerca und Loa aus der Familie der Filariidae, die im Blut, im Gewebe und verschiedenen Organen vorkommen, ferner gegen Dracunculus und Parasiten der Arten Strongyloides und Trichinella, die speziell den Gastro-Intestinalkanal infizieren.

Die Verbindungen der Formel I werden in unveränderter Form oder vorzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Verbindungen der Formel I werden bei Warmblütern in Aufwandmengen von 0,01 bis 10 mg/kg Körpergewicht dosiert. Ueber geschlossenen Kultur-Anbauflächen werden sie in Mengen von 10 g bis 1000 g pro Hektar angewendet. Sie kommen ferner in Pferchen, Gehegen, Stallungen oder sonstigen Räumen zur Anwendung.

Die Formulierungen, d.h. die den Wirkstoff der Formel I enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen

$C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder -ethylether, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z.B. für Stäubemittel und diapergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüber hinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes nichtionogene, kation-und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäure-methyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettaulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxyd-Addukten. Die sulfonierten Benzimidazolderivate enthalten vorzugsweise 2-Sulfonsäuregruppen und einen Fettsäurerest mit 8 bis 22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxyd-Adduktes oder Phospholipide in Frage.

Die in der Formulierungstechnik gebräuchlichen Tenside sind u.a. in folgender Publikation beschrieben:
"Mc Cutcheon's Detergents and Emulsifiers Annual"
MC Publishing Corp., Ridgewood, New Jersey, 1982.

Die pestiziden Zubereitungen enthalten in der Regel 0,01 bis 95 %, insbesondere 0,1 bis 80 %, Wirkstoff der Formel I, 5 bis 99,99 % eines festen oder flüssigen Zusatzstoffes und 0 bis 25 %, insbesondere 0,1 bis 25 %, eines Tensides.

Während als Handelsware eher konzentrierte Mittel bevorzugt werden, verwendet der Endverbraucher in der Regel verdünnte Mittel mit 1-10'000 ppm Wirkstoffgehalt.

Ein weiterer Gegenstand vorliegender Erfindung betrifft daher Schädlingsbekämpfungsmittel, die neben üblichen Trägerstoffen und/oder Verteilungsmitteln als Wirkstoff mindestens eine Verbindung der Formel I enthalten.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

## Herstellungsbeispiele

### 1. Herstellung von Ausgangs- und Zwischenprodukten

**Beispiel 1.1: Herstellung von 14,15-Epoxy-Milbemycin D (Formel IX)**

Zu einer Lösung von 550 mg Milbemycin D in 5 ml Dichlormethan wird unter Eiskühlung eine Lösung von 170 mg Chlorperbenzoesäure in 5 ml Dichlormethan gegeben. Nach 1-stündigem Rühren bei 0° bis +5°C werden nochmals 170 mg des Oxidationsmittels hinzugefügt und weitere 30 min gerührt. Nach beendeter Reaktion wird die Lösung in eine eisgekühlte Lösung von Natriumsulfit gegossen und mit

Essigsäure-ethylester extrahiert. Die vereinigten Extrakte werden einmal mit Wasser gewaschen, getrocknet und im Vakuum eingedampft. Das Rohprodukt wird durch Chromatographierung über eine Silicagel-Säule (Elutionsmittel n-Hexan/Essigsäureethylester 20:15) gereinigt. Es werden 450 mg 14,15-Epoxy-Milbemycin D als amorphe, weisse Substanz erhalten.

**Beispiel 1.2: Herstellung von 15-Hydroxy-$\Delta^{13,14}$-Milbemycin D (Formel IIb)**

Zu einer Lösung von 2,1 ml (1,75 g, 15,3 mmol) Triethylaluminium in 8,5 ml abs. Diethylether werden bei -20° C 9,5 ml (0,41 g, 9,53 mmol) einer 6,96 %igen Lösung von $HN_3$ in Diethylether gegeben. Diese Mischung wird dann bei -10° C unter stark exothermer Reaktion zu 1,8 g (3,15 mmol) 14,15-Epoxy-Milbemycin D (in Substanz) gegeben. Nach 1 Stunde bei Raumtemperatur werden 4 ml absoluter Ether zugegeben und das gallertartige Reaktionsgemisch wird kräftig gerührt. Nach 4 Stunden wird wie im Beispiel 1.1 aufgearbeitet. Chromatographische Reinigung an 70 g Kieselgel ($CH_2Cl_2$/Aceton 10:1) ergibt 200 mg (10 %) 14-Azido-15-hydroxy-Milbemycin D und 820 mg (45 %) 15-Hydroxy-$\Delta^{13,14}$-Milbemycin D, Smp.: 151-153° C (aus Methanol).

**Beispiel 1.3: Herstellung von 5-O-t-Butyldimethylsilyl-14,15-epoxy-Milbemycin D (Formel IX)**

Eine Lösung von 2,21 g (3,86 mmol) 14,15-Epoxy-Milbemycin D, 757 mg (5,02 mmol) t-Butyl-dimethylchlorsilan und 342 mg (5,02 mmol) Imidazol in 4 ml Dimethylformamid wird 90 min bei Raumtemperatur gerührt. Anschliessend werden 80 ml Diethylether zugegeben, und das Gemisch wird über 20 g Kieselgel filtriert und eingeengt. Es werden 2,65 g (100 %) 5-O-t-Butyldimethylsilyl-14,15-epoxy-Milbemycin D erhalten.

$^1$H-NMR (300 MHz. Lösungsmittel $CDCl_3$. Messwerte $\delta$ bezogen auf $Si(CH_3)_4$ (= Tetramethylsilan = TMS)-).

0,12 ppm (s) $(CH_3)_2Si$-O-;
0,92 ppm (s) $(t.C_4H_9)Si$-O-;
1,23 ppm (breites s) $C_{14}CH_3$, d.h. Signal der $CH_3$-Gruppe in 14-Position);
2,56 ppm (d; J = 9 Hz) ($C_{15}H$, d.h. Signal des Protons in 15-Position).

Aehnlich lässt sich durch Reaktion mit Trimethylsilyl-trifluor-methansulfonat das entsprechende 5-O-Trimethylsilyl-14,15-epoxy-Milbemycin D herstellen, Smp. 92-97° C.

**Beispiel 1.4: Herstellung von 5-O-t-Butyldimethylsilyl-15-hydroxy-$\Delta^{13,14}$-Milbemycin D (Formel IIb)**

Eine Lösung des $HN_3/Et_3Al$-Komplex-Reagenz (hergestellt aus einer Lösung von 4,97 ml Triethylaluminium in 7 ml abs. Tetrahydrofuran (THF) und 9,15 ml einer 2,39 M Lösung von $HN_3$(21,9 mmol) in abs. Diethylether) wird unter Argon zu einer Lösung von 5,0 g (7,29 mmol) 5-O-t-Butyldimethylsilyl-14,15-epoxy-Milbemycin D in ca. 20 ml abs. Tetrahydrofuran (THF) gegeben, und das Gemisch wird 15 Stunden unter Rückfluss erhitzt. Anschliessend werden bei Raumtemperatur 250 ml Ether, 2 ml Methanol und schliesslich ein Gemisch von 10 g $Na_2SO_4 \cdot 10\ H_2O$ und 10 g Celite zugegeben. Das Gemisch wird filtriert und eingeengt. Die chromatographische Reinigung des Rohproduktes an 160 g Kieselgel (0 bis 30 % Essigsäureethylester in Hexan) ergibt 2,37 g (47 %) 5-O-t-Butyldimethylsilyl-15-hydroxy-$\Delta^{13,14}$-Milbemycin D.

$^1$H-NMR (300 MHz, $CDCl_3$):
1,59 ppm(d; J = 1 Hz), ($C_{14}CH_3$);
4,06 ppm (dd; $J_1$ = 11 Hz; $J_2$ = 4 Hz) ($C_{15}H$);
5,15 ppm (d; J = 8 Hz) ($C_{13}H$):

Daneben werden 109 mg (2 %) 13$\beta$-Azido-5-O-t-butyldimethylsilyl-Milbemycin D gewonnen.

**Beispiel 1.5: Herstellung von 14,15-Epoxy-Milbemycin $A_4$ ($R_2 = C_2H_5$) [Formel IX]**

Zu einer Lösung von 5,7 g (10,5 mmol) Milbemycin $A_4$ in 140 ml Dichlormethan und 120 ml 0,5 M $NaHCO_3$-Lösung wird bei Raumtemperatur eine Lösung von 2,43 g (14,08 mmol) m-Chlorobenzopersäure in 70 ml Dichlormethan tropfenweise zugegeben. Das Gemisch wird 1 Stunde bei Raumtemperatur kräftig gerührt und dann mit 300 ml Dichlormethan verdünnt. Die organische Phase wird mit wässriger $NaHCO_3$-Lösung gewaschen, mit $Na_2SO_4$ getrocknet und eingeengt. Es werden 5,7 g Epoxyd als Rohprodukt erhalten.

**Beispiel 1.6: Herstellung von 5-O-t-Butyldimethylsilyl-14,15-epoxy-Milbemycin $A_4$ (Formel IX)**

5,7 g 14,15-Epoxy-Milbemycin $A_4$ werden in 10 ml trockenem Dimethylformamid (DMF) gelöst. Bei Raumtemperatur werden 0,63 g (9,16 mmol) Imidazol und 1,4 g (9,34 mmol) t-Butyldimethylchlorsilan zugegeben. Das Gemisch wird 1 Stunde bei Raumtemperatur gerührt und an 150 g Kieselgel chromatographiert (Hexan/Ether 4:1), wobei 2,84 g (40 % d.Th. Ausbeute, bezogen auf Milbemycin $A_4$) des silylierten Epoxi-Derivats erhalten werden.

**Beispiel 1.7: Herstellung von 5-O-t-Butyldimethylsilyl-15-hydroxy-$\Delta^{13,14}$-Milbemycin $A_4$ (Formel IIb)**

Das Komplex-Reagenz $HN_3/Al(Ethyl)_3$ wird wie folgt hergestellt: 2,8 ml (12,2 mmol) $Al(C_2H_5)_3$ in 4 ml abs.Tetrahydrofuran (THF) werden unter Argon bei ca. -20°C langsam mit 5,28 ml (20,4 mmol) einer 10 %igen Lösung von $HN_3$ in abs. Diethylether versetzt. Zu dieser Lösung wird unter Argon eine Lösung von 2,84 g (4,25 mmol) 5-O-t-Butyldimethylsilyl-14,15-epoxy-milbemycin $A_4$ (Bsp. 1.6) in ca. 12 ml abs. Tetrahydrofuran gegeben, und das so erhaltene Gemisch wird 4 Stunden unter Rückfluss erhitzt. Bei Raumtemperatur werden 500 ml Diethylether, 10 g $Na_2SO_4 \cdot 10H_2O$ und 10 g Celite zugegeben, und das Gemisch wird filtriert und eingeengt. Die Chromatographie des Rohproduktes an 100 g Kieselgel (Hexan/Diethylether 7:2) ergibt 1,72 g (60 % d.Th.) der Titel-Verbindung.
$^1H$-NMR (300 MHz, $CDCl_3$):
1,59 ppm (br. s) ($C_{14}CH_3$);
4,05 ppm (br. s) ($C_{15}H$);
5,15 ppm (d; J = 6 Hz) ($C_{13}H$).
Daneben werden 0,1 g 13$\beta$-Azido-5-O-t-butyldimethylsilyl-Milbemycin $A_4$ erhalten.

**Beispiel 1.8: Herstellung von 15-Hydroxy-$\Delta^{13,14}$-Milbemycin $A_4$ (Formel IIb)**

Die Hydrolyse von 5-O-t-Butyldimethylsilyl-15-hydroxy-$\Delta^{13,14}$-Milbemycin $A_4$ (Beispiel 1.7) mit einer 1 %igen Lösung von p-Toluolsulfonsäure in Methanol und die Aufarbeitung in Diethylether mit 5%iger wässriger Na-Hydrogencarbonat-Lösung ergibt die Titel-Verbindung.

**Beispiel 1.9: Herstellung von 14,15-Epoxy-Milbemycin $A_3$ ($R_2 = CH_3$) (Formel IX)**

Nach der Vorschrift des Beispiels 1.1 werden aus 220 mg Milbemycin $A_3$ in 5 ml Dichlormethan und 320 mg Benzoepersäure in 5 ml Dichlormethan bei -2° bis +5°C während 1,5 Stunden und Reinigung über eine Silicagel-Säule 190 mg 14,15-Epoxy-Milbemycin $A_3$ gewonnen.

**Beispiel 1.10: Herstellung von 5-O-tert.Butyldimethylsilyl-14,15-epoxy-Milbemycin $A_3$ (Formel IX)**

Nach der Vorschrift des Beispiels 1.3 werden aus 190 mg 14,15-Epoxi-Milbemycin $A_3$ und 120 mg tert.-Butyldimethylchlorsilan in Gegenwart von Imidazol 217 mg der Titel-Verbindung erhalten.

**Beispiel 1.11: Herstellung von 5-O-tert.-Butyldimethylsilyl-15-hydroxy-$\Delta^{13,14}$-Milbemycin $A_3$ (Formel IIb)**

Analog zur Epoxyd-Spaltung des Beispiels 1.2 werden aus 210 mg 5-O-tert.-Butyldimethylsilyl-14,15-epoxy-Milbemycin $A_3$ in absolutem Diethylether mit Hilfe des Komplex-Reagenz $HN_3/Et_3Al$ unter Argon nach anschliessender Reinigung 203 mg der Titelverbindung erhalten.
$^1H$-NMR (300 HMz, $CDCl_3$):
1,58 ppm (br. s) ($C_{14}CH_3$);
4,05 ppm (br. s) ($C_{15}H$);
5,15 ppm (d; J=6 Hz) ($C_{13}H$).

**Beispiel 1.12: Herstellung von 15-Hydroxy-$\Delta^{13,14}$-Milbemycin $A_3$ (Formel IIb)**

Analog zu Beispiel 1.4 wird das Reagens $HN_3/Al(C_2H_5)_3$ frisch hergestellt und bei -10°C zu einer Lösung von 830 mg (3,05 mmol) 14,15-Epoxy-Milbemycin $A_3$ in 7 ml trockenem Diethylether getropft. Nach der Aufarbeitung werden 385 mg 15-Hydroxy-$\Delta^{13,14}$-Milbemycin $A_3$ und 92 mg 14-Azido-15-hydroxy-Milbemycin $A_3$ erhalten.

**Beispiel 1.13: Herstellung von 13-Deoxy-14,15-epoxy-22,23-dihydroavermectin-Bla-aglykon ($R_2$ =**

**sec.$C_4H_9$) (Formel IX)**

Analog zu Beispiel 1.1 erhält man aus 520 mg 13-Deoxi-22,-23-dihydroavermectin-Bla-aglykon [US-PS 4,173,571 und Tetrahedron Letters, Vol. 24, No. 48, pp. 5333-5336 (1983)] und 210 mg m-Chlorbenzoepersäure in 20 ml Dichlormethan 510 mg der Titelverbindung.

**Beispiel 1.14: Herstellung von 5-O-t.Butyldimethylsilyl-13-deoxi-14,15-epoxy-22,23-dihydro-avermectin-Bla-aglykon (Formel IX)**

Analog zu Beispiel 1.3 erhält man aus 220 mg 13-Deoxy-14,15-epoxy-22,23-dihydro-avermectin-Bla-aglykon (Beispiel 1.13) und 55 mg tert.Butyldimethylchlorsilan in Gegenwart von 25 mg Imidazol in 5 ml trockenem DMF 108 mg der Titelverbindung.

**Beispiel 1.15: Herstellung von 13-Deoxy-15-hydroxy-$\Delta^{13,14}$-22,23-dihydro-avermectin-Bla-aglykon (Formel IIb)**

Analog zu Beispiel 1.4 erhält man aus 220 mg 13-Deoxy-14,15-epoxi-22,23-dihydro-avermectin-Bla-aglykon (Beispiel 1.13) mit dem Komplex-Reagenz, bestehend aus 320 mg $Al(C_2H_5)_3$ und 110 mg einer 6,96 %igen Lösung von $HN_3$ in total 16 ml trockenem Diethylether 112 mg der Titelverbindung. Daneben werden 61 mg 13-Deoxi-14-azido-15-hydroxy-22,23-dihydro-avermectin-Bla-aglykon erhalten.

**Beispiel 1.16: Herstellung von 5-O-tert.Butyldimethylsilyl-13$\beta$-hydroxy-milbemycin D und von 13$\beta$-Hydroxy-milbemycin D (Formel IIa)**

Eine Lösung bestehend aus 286 mg (0,41 mmol) 5-O-tert.Butyldimethylsilyl-15-hydroxy-$\Delta^{13,14}$-milbemycin D und 209 mg (0,56 mmol) Pyridiniumdichromat (PDC) in 3 ml Dimethylformamid (DMF) wird 30 min bei Raumtemperatur gerührt. Anschliessend wird 1 ml Isopropanol zugegeben, 5 min weitergerührt und dann mit 50 ml Ether verdünnt. Nach weiteren 10 min wird das Gemisch durch Kieselgel filtriert und eingeengt. Nach chromatographischer Reinigung des Rohproduktes an 20 g Kieselgel (Ether/Hexan 1:2) werden 165 mg (57 %) 5-O-t-Butyl-dimethylsilyl-13$\beta$-hydroxy-Milbemycin D erhalten.
$^1$H-NMR (300 MHz; $CDCl_3$; TMS):
1,59 ppm (br. s)($C_{14}CH_3$)
3,70 ppm (d; J = 10 Hz)($C_{13}H$).

105 mg (0,153 mmol) der so gewonnenen Verbindung werden mit 1 ml einer 1 %igen Lösung von p-Toluolsulfonsäure in Methanol 1 Stunde bei Raumtemperatur gerührt. Das Gemisch wird mit 20 ml Ether verdünnt, durch Kieselgel filtriert, eingeengt. Der Rückstand wird an ca. 10 g Kieselgel chromatographiert (Aceton/Dichlormethan 1:4), wobei 73 mg (83 %) 13$\beta$-Hydroxy-Milbemycin D erhalten werden.
$^1$H-NMR (300 MHz; $CDCl_3$; TMS):
1,58 ppm (br.s)($C_{14}CH_3$)
3,71 ppm (d; J = 10 Hz)($C_{13}H$).

**Beispiel 1.17 Herstellung von 5-O-tert.Butyldimethylsilyl-13$\beta$-hydroxy-milbemycin $A_4$**

Eine Lösung bestehend aus 1,06 g (1,59 mmol) 5-O-tert.Butyldimethylsilyl-15-hydroxy-$\Delta^{13,14}$-milbemycin $A_4$ und 383 mg (1,02 mmol) Pyridiniumdichromat (PDC) in 5 ml Dimethylformamid (DMF) wird 30 min. bei Raumtemperatur gerührt. Anschliessend wird 1 ml Isopropanol zugegeben, 5 min. weitergerührt und dann mit 50 ml Ether verdünnt. Nach weiteren 10 min. wird das Gemisch durch Kieselgel filtriert und eingeengt. Nach chromatographischer Reinigung des Rohproduktes an 20 g Kieselgel (Ether/Hexan 1:2) werden 625 mg (59 %) 5-O-t-Butyldimethylsilyl-13$\beta$-hydroxy-Milbemycin $A_4$ erhalten. $^1$H-NMR (300 MHz; $CDCl_3$; TMS):
0,98 ppm (t; J = 7 Hz) ($CH_3CH_2$)
1,95 ppm (br. s) ($C_{14}CH_3$)
3,69 ppm (d; J = 9 Hz) ($C_{13}H$)

2. Herstellung der Endprodukte

**Beispiel 2.1: Herstellung von 13$\beta$-Pivaloyloxy-milbemycin D und 15-Pivaloyloxy-$\Delta^{13,14}$-milbemycin D.**

Eine Lösung von 278 mg (0,40 mmol) 5-O-tert.-Butyldimethylsilyl-15-hydroxy-$\Delta^{13,14}$-milbemycin D, 208 mg (2,04 mmol) Pivalinsäure und 129 mg (0,80 mmol) Essigsäure-triethylorthoester in 2 ml abs. Toluol wird in einem Kolben mit Claisen-Aufsatz während 5 min. auf 130° C erhitzt. Die Aufarbeitung und chromatographische Reinigung an 100 g Kieselgel (Laufmittel Hexan/Diethylether 5:1) ergibt 200 mg (64 %) 5-O-tert.-Butyldimethylsilyl-13β-pivaloyloxy-milbemycin D und 100 mg (32 %) 5-O-tert.-Butyldimethylsilyl-15-pival oyloxy-$\Delta^{13,14}$-milbemycin D. Diese beiden Silylether werden während 2 Std. bei Raumtemperatur mit einer Lösung von 3 ml 1 % p-Toluolsulfonsäure (TsOH) in Methanol (MeOH) behandelt, wobei 165 mg (96 %) 13β-Pivaloyloxy-milbemycin D

$^1$H-NMR (300 MHz; $CDCl_3$; TMS):
1,19 ppm (s) ($(CH_3)_3C$)
1,56 ppm (br. s) ($C_{14}CH_3$)
4,90 ppm (d; J = 10,6 Hz ($C_{13}H$)

und 82 mg (95 %) 15-Pivaloyloxy-$\Delta^{13,14}$-milbemycin D erhalten werden. $^1$H-NMR (300 MHz; $CDCl_3$; TMS):
1,18 ppm (s) ($(CH_3)_3C$)
1,53 ppm (br. s) ($C_{14}CH_3$)
5,16 ppm (dd; J = 9 und 5 Hz) ($C_{15}H$)

Analog zu Beispiel 2.1 werden ausgehend von 5-O-tert.Butyldimethylsilyl-15-hydroxy-milbemycin $A_4$ die folgenden Verbindungen hergestellt:

2.1.1 **13β-(3'-Chlor-2',2'-dimethyl-propionyloxy)-milbemycin $A_4$**
$^1$H-NMR (300 MHz, $CDCl_3$, TMS):
1.27, 1.28 ppm (2s) ($ClCH_2C(\underline{CH_3})_2CO$)
1.55 ppm (s) ($C_{14}CH_3$)
3.60 ppm (br.s) ($Cl\underline{CH_2}C(CH_3)_2CO$)
4.94 ppm (d, J = 10.5 Hz) ($C_{13}H$)

2.1.2 **13β-(2'-Chloro-2'methyl-propionyloxy)-milbemycin $A_4$**
$^1$H-NMR (300 MHz, $CDCl_3$, TMS):
1.51 ppm (s) ($2CH_3$-CCl-CO)
4.92 ppm (d, J = 10 Hz) ($C_{13}H$)

2.1.3 **13β-(2,2'-Dichlorpropionyloxy)-milbemycin $A_4$**
$^1$H-NMR (300 MHz, $CDCl_3$, TMS):
1.53 ppm (s) ($CH_3$-$CCl_2$-CO)
4.94 ppm (d, J = 10 Hz) ($C_{13}H$)

**Beispiel 2.2: Herstellung von 13β-Pivaloyloxy-milbemycin D**

Zu einer Lösung von 200 mg (0,29 mmol) 5-O-tert.-Butyldimethylsilyl-13β-hydroxymilbemycin D in 5 ml trockenem Chloroform werden 0,4 ml (2,9 mmol) Triethylamin und 0,18 ml (1,45 mmol) Pivaloylchlorid zugegeben und das Gemisch wird unter Stickstoff 2 Std. unter Rückfluss erhitzt. Die Behandlung des Rohprodukts (200 mg) mit 3 ml einer 1 % Lösung von p-TsOH in MeOH während 2 Std. bei Raumtemperatur ergibt 166 mg (86 %) 13β-Pivaloyloxy-milbemycin D.

Analog zu Beispiel 2.2 werden ausgehend von 5-O-tert.Butyl-dimethylsilyl-13β-hydroxy-milbemycin D oder A4 die folgenden Verbindungen hergestellt:

2.2.1 **13β-(2'-Methoxy-2'-methylpropionyloxy)-milbemycin D**
$^1$H-NMR (300 MHz, $CDCl_3$, TMS):
1.41 ppm (s) ($CH_3OC(CH_3)_2CO$)
3.25 ppm (s) ($CH_3OC(\overline{CH_3})_2CO$)
4.97 ppm (d, J = 10.5 Hz) ($C_{13}H$)

2.2.2 **13β-(2'-Methoxy-2'-methyl-propionyloxy)-milbemycin $A_4$**
$^1$H-NMR (300 MHz, $CDCl_3$, TMS):
1.41 ppm (s) ($CH_3OC(\underline{CH_3})_2CO$)
3.26 ppm (s) ($\underline{CH_3}\ OC(\overline{CH_3})_2CO$)
497 ppm (d, J = 10.5 Hz) ($C_{13}H$)

2.2.3 **13β-Trichloracetoxy-milbemycin D**
$^1$H-NMR (250 MHz, $CDCl_3$ TMS):
1.62 ppm (s) ($C_{14}CH_3$)
4.99 ppm (d, J = 10 Hz) ($C_{13}H$).

2.2.4 **13β-(cis/trans-2',2'-Dimethyl-3'-[2",2"-dichlorvinyl]-cyclopropancarbonyloxy)-milbemycin D**

$^1$H-NMR (300 MHz; $CDCl_3$; TMS):
1,55 ppm (br. s) ($C_{14}CH_3$)
1,87 ppm (br. s) ($C_4CH_3$)
4,91 ppm (d, J = 10,2 Hz) ($C_{13}H$)
4,94 ppm (d, J = 10,7 Hz) ($C_{13}H$)
6,20 ppm (d, J = 8,8 Hz) ($Cl_2C=CH$)
6,24 ppm (d, J = 8,8 Hz) ($Cl_2C=\underline{CH}$)

**Beispiel 2.3: Herstellung von 13β-Pivaloyloxy-milbemycin $A_4$**

Zu einer Lösung von 100 mg (0,15 mmol) 5-O-tert.-Butyldimethylsilyl-13β-hydroxymilbemycin $A_4$ in 2,5 ml trockenem Chloroform werden 0,2 ml (1,5 mmol) Triethylamin und 0,09 ml (0,07 mmol) Pivaloylchlorid zugegeben. Das Gemisch wird dann unter Stickstoff 2 Std. unter Rückfluss erhitzt. Die Behandlung des Rohprodukts (100 mg) mit 3 ml einer 1 % Lösung von p-TsOH in MeOH während 2 Std. bei Raumtemperatur ergibt 82 mg (85 %) 13β-pivaloyloxy-milbemycin $A_4$.
$^1$H-NMR (300 MHz; $CDCl_3$; TMS):
1,20 ppm (s) ($(CH_3)_3C$)
0,98 ppm (t, J = 7 Hz), ($\underline{CH_3}CH_2$)
1,53 ppm (br. s) ($C_{14}CH_3$)
4,90 ppm (D; J = 10,5 Hz) ($C_{13}H$)

**Beispiel 2.4: Herstellung von 13β-Formyloxy-milbemycin D**

Eine Lösung von 136 mg (0,198 mmol) 5-O-tert.-Butyldimethylsilyl-15-hydroxy-$\Delta^{13,14}$-milbemycin D und 0,1 ml Essigsäuretriethylorthoester in 3 ml $H_2SO_4$/Diisopropylether/Dimethylformamid (1:10:90) wird 10 Min. bei Raumtemperatur gerührt. Die Aufarbeitung in Hexan mit 5 % wässriger $NaHCO_3$-Lösung und Wasser und die chromatographische Reinigung des Rohprodukts an 20 g Kieselgel (Laufmittel Ethylacetat:Hexan 1:4) lieferte 80 mg (57 %) 5-O-tert.Butyldimethylsilyl-13β-formyloxy-milbemycin D.

Die Behandlung von 155 mg (0,217 mmol) 5.O-tert.Butyldimethylsilyl-13β-formyloxy-milbemycin D mit 2 ml einer 40 % wässrigen Lösung von HF und Actonitril (1:99) während 2 Std. bei Raumtemperatur ergibt nach der Aufarbeitung in Diethylether mit 5 % wässriger $NaHCO_3$-Lösung und der chromatographischen Reinigung an 20 g Kieselgel (Laufmittel Ethylacetat/Hexan 2:3) 105 mg (81 %) 13β-Formyloxy-milbemycin D.
$^1$H-NMR (300 MHz; $CDCl_3$; TMS):
1,56 ppm (br. s) ($C_{14}CH_3$)
1,87 ppm (br. s) ($C_4CH_3$)
5,05 ppm (d, J = 10.5 Hz) ($C_{13}H$)
8,08 ppm (s) (OCHO)
Massenspektrum m/e:
600 ($M^+$, $C_{34}H_{48}O_9$), 472, 426, 293, 209, 181, 151.

**Beispiel 2.5: Herstellung von 13β-Pivaloyloxy-milbemycin $A_4$**

Eine Lösung von 100 mg (0,149 mmol) 5-O-tert-Butyldimethylsilyl-15-hydroxy-$\Delta^{13,14}$-milbemycin $A_4$, 100 mg (1,02 mmol) Pivalinsäure und 65 mg (0,40 mmol) Essigsäure-triethylorthoester in 2 ml abs. Toluol wird in einem Kolben mit Claisen-Aufsatz während 5 min. auf 130 °C erhitzt. Die Aufarbeitung und chromatographische Reinigung an 100 g Kieselgel (Laufmittel Hexan/Diethylether 5:1) ergibt 73 mg (65 %) 5-O-tert-Butyldimethylsilyl-13β-pivaloyloxy-milbemycin $A_4$.

Der Silylether wird während 2 Std. bei Raumtemperatur mit einer Lösung von 3 ml 1%iger Toluolsulfonsäure in Methanol behandelt, wobei 60 mg (96 %) 13β-Pivaloyloxy-milbemycin $A_4$ erhalten werden.

**Beispiel 2.6: Herstellung von 13β-(4'-Chlor-butanoyloxy)-milbemycin D**

Zu einer Lösung von 100 mg (0,145 mmol) 5-O-tert-Butyldimethylsilyl-13β-hydroxy-milbemycin D in 1 ml Pyridin und 2 ml Dichlormethan werden bei 0 °C vorsichtig 24,5 mg (0,174 mmol) 4-Chlorbuttersäurechlorid in 0,5 ml Dichlormethan zugegeben. Das Gemisch wird dann aufgearbeitet, und die Behandlung des Rohproduktes mit 3 ml einer Lösung von 1 % p-TsOH in MeOH während 2 Std. bei Raumtemperatur ergibt

92,6 mg (94 %) 13β-(4'-Chlor-butanoyloxy)-milbemycin D.
$^1$H-NMR (300 MHz; $CDCl_3$; TMS):
1,54 ppm (br. s) ($C_{14}CH_3$)
1,82 ppm (br. s) ($C_4CH_3$)
3,58 ppm (d, J = 6,3 Hz) (2H-$C_4$')
4,96 ppm (d, J = 10,5 Hz) ($C_{13}H$)

**Beispiel 2.7: Herstellung von 13β-(3'-Chlor-2',2'-dimethyl-propionyloxy)-milbemycin D**

Eine Lösung von 100 mg (0,145 mmol) 5-O-tert.Butyldimethylsilyl-15-hydroxy-$\Delta^{13,14}$-milbemycin D, 78 mg (0,57 mmol) 3-Chlor-2,2-dimethyl-propionsäure und 47 mg (0,29 mmol) Essigsäure-triethylorthoester in 3 ml abs. Toluol wird in einem Kolben mit Claisen-Aufsatz 5 min. auf 100 °C erhitzt. Die Aufarbeitung und chromatographische Reinigung an 60 g Kieselgel (Laufmittel Hexan/Diethylether 5:1) ergibt 50 mg 5-O-tert.Butyldimethylsilyl-13β-(3'Chlor-2',2'-dimethyl-propionyloxy)-milbemycin D. Der Silylether wird während 2 Std. bei Raumtemperatur mit einer Lösung von 3 ml 1%iger TsOH in MeOH behandelt, wobei 41 mg (41 %) 13β-(3'-Chlor-2',2'-dimethyl-propionyloxy)-milbemycin D erhalten werden.
$^1$H-NMR (300 MHz; $CDCl_3$; TMS):
1,27 ppm (s, $2CH_3$-$C_2$')
1,54 ppm (br. s) ($C_{14}CH_3$)
1,87 ppm (br. s) ($C_4CH_3$)
4,96 ppm (d, J = 10,5 Hz) ($C_{13}H$)

**Beispiel 2.8: Herstellung von 13β-Cyclopropancarbonyloxy-milbemycin D**

Eine Lösung von 150 mg (0,215 mmol) 5-O-tert.Butyldimethylsilyl-15-hydroxy-$\Delta^{13,14}$-milbemycin D, 94 mg (1,075 mmol) Cyclopropancarbonsäure und 71 mg (0,47 mmol) Essigsäure-Triethylorthoester in 2 ml abs. Toluol wird in einem Kolben mit Claisen-Aufsatz während 5 min. auf 100 °C erhitzt. Die Aufarbeitung und chromatographische Reinigung an 80 g Kieselgel (Laufmittel Hexan/Diethylether 5:1) ergibt 100 mg 5-O-tert.Butyldimethylsilyl-13β-Cyclopropancarbonyloxy-milbemycin D. Der Silylether wird während 2 Std. bei Raumtemperatur mit einer Lösung von 3 ml 1%iger TsOH in MeOH behandelt, wobei 75 mg (53 %) 13β-Cyclopropancarbonyloxy-milbemycin D erhalten werden.
$^1$H-NMR (300 MHz; $CDCl_3$; TMS):
1,55 ppm (br. s) ($c_{14}CH_3$)
1,87 ppm (br. s) ($c_4CH_3$)
4,95 ppm (d, J = 10,5 Hz) ($C_{13}H$)

**Beispiel 2.9: Herstellung von 13β-(1'-Methyl-cyclopropancarbonyloxy)-milbemycin D**

Eine Lösung von 278 mg (0,40 mmol) 5-O-tert.Butyldimethylsilyl-15-hydroxy-$\Delta^{13,14}$-milbemycin D, 204 mg (2,04 mmol) 1-Methyl-cyclopropancarbonsäure und 132 mg (0,82 mmol) Essigsäure-triethylorthoester in 5 ml abs.Toluol wird in einem Kolben mit Claisen-Aufsatz während 5 min. auf 100 °C erhitzt. Die Aufarbeitung und chromatographische Reinigung an 100 g Kieselgel (Laufmittel Hexan/ Diethylether 5:1) ergibt 150 mg 5-O-tert.Butyldimethylsilyl-13β-(1'-Methyl-cyclopropancarbonyloxy)-milbemycin D. Der Silylether wird während 2 Std. bei Raumtemperatur mit einer Lösung von 3 ml 1%iger 7sOH in MeOH behandelt, wobei 127 mg (48 %) 13β-(1'-Methyl-cyclopropancarbonyloxy)-milbemycin D erhalten werden.
$^1$H-NMR (300 MHz; $CDCl_3$; TMS):
1,28 ppm (s) ($C_1$'$CH_3$)
1,52 ppm (br.s) ($C_{14}CH_3$)
1,87 ppm (br.s) ($C_4CH_3$)
4,91 ppm (d, J = 10,5 Hz) ($C_{13}H$)

**Beispiel 2.10: Herstellung von 13β-(1'-Methyl-cyclopropancarbonyloxy)-milbemycin A4**

Eine Lösung von 140 mg (0,206 mmol) 5-O-tert.Butyldimethylsilyl-15-hydroxy-$\Delta^{13,14}$-milbemycin $A_4$, 102 mg (1,01 mmol) 1-Methylcyclopropancarbonsäure und 66 mg (0,41 mmol) Essigsäure-triethylorthoester in 5 ml abs. Toluol wird in einem Kolben mit Claisen-Aufsatz während 5 min. auf 100 °C erhitzt. Die Aufarbeitung und chromatographische Reinigung an 40 g Kieselgel (Laufmittel Hexan/ Diethylether 5:1) ergibt 58 mg 5-O-tert.Butyldimethylsilyl-13β-(1'-Methyl-cyclopropancarbonyloxy)-milbemycin $A_4$. Der Silyle-

ther wird während 2 Std. bei Raumtemperatur mit einer Lösung von 3 ml 1%iger TsOH in MeOH behandelt, wobei 47 mg (35 %) 13β-(1'-Methylcyclopropancarbonyloxy)-milbemycin $A_4$ erhalten werden.
$^1$H-NMR (300 MHz; $CDCl_3$; TMS):
1,33 ppm (s) ($C_1$'$CH_3$)
1,52 ppm (br.s) ($C_{14}CH_3$)
1,87 ppm (br. s) ($C_4CH_3$)
4,90 ppm (d, J = 10,5 Hz) ($C_{13}H$)

**Beispiel 2.11: Herstellung von 13β-Cyclobutancarbonyloxy-milbemycin D**

Zu einer Lösung von 100 mg (0,145 mmol) 5-O-tert.Butyldimethylsilyl-13β-hydroxy-milbemycin D in 2 ml Pyridin werden bei 0 °C vorsichtig 20,6 mg (0,174 mmol) Cyclobutancarbonsäurechlorid in 0,5 ml Dichlormethan zugegeben. Das Gemisch wird dann aufgearbeitet, und die Behandlung des Rohproduktes mit 3 ml einer Lösung von 1 % p-TsOH in MeOH während 2 Std. bei Raumtemperatur ergibt 45 mg (89 %) 13β-Cyclobutancarbonyloxy-milbemycin D.
$^1$H-NMR (300 MHz; $CDCl_3$; TMS):
1,53 ppm (br.s) ($C_{14}CH_3$)
1,87 ppm (br. s) ($C_4CH_3$)
4,94 ppm (d, J = 10,6 Hz) ($C_{13}H$)

**Beispiel 2.12: Herstellung von 13β-(1-Adamantancarbonyloxy)-milbemycin D**

Eine Lösung von 278 mg (0,40 mmol) 5-O-tert.Butyldimethylsilyl-15-hydroxy-$\Delta^{13,14}$-milbemycin D, 367 mg (2,03 mmol) 1-Adamantancarbonsäure und 133 mg (0,82 mmol) Essigsäure-triethylorthoester in 5 ml abs. Toluol wird in einem Kolben mit Claisen-Aufsatz während 5 min. auf 100 °C erhitzt. Die Aufarbeitung und chromatographische Reinigung an 120 g Kieselgel (Laufmittel Hexan/Diethylether 5:1) ergibt 140 mg 5-O-tert.Butyldimethylsilyl-13β-1-Adamantancarbonyloxy-milbemycin D. Der Silylether wird während 2 Std. bei Raumtemperatur mit einer Lösung von 3 ml 1%iger TsOH in MeOH behandelt, wobei 113 mg (38 %) 13β-(1-Adamantancarbonyloxy)-milbemycin D erhalten werden.
$^1$H-NMR (300 MHz; $CDCl_3$; TMS):
1,53 ppm (br.s) ($c_{14}CH_3$)
1,88 ppm (br. s) ($c_4CH_3$)
4,90 ppm (d, J = 10,5 Hz) ($C_{13}H$)

**Beispiel 2.13: Herstellung von 13β-Trichloracryloyloxy-milbemycin D**

Zu einer Lösung von 120 mg (0,175 mmol) 5-O-tert.Butyldimethylsilyl-13β-hydroxy-milbemycin D in 2 ml Dichlormethan werden 34 mg (0,337 mmol) Triethylamin und bei 0 °C vorsichtig 44,0 mg (0,225 mmol) Trichloracryloylchlorid in 0,5 ml Dichlormethan zugegeben. Das Gemisch wird dann aufgearbeitet, und die Behandlung des Rohproduktes mit 3 ml einer Lösung von 1 % p-TsOH in MeOH während 2 Std. bei Raumtemperatur ergibt 60 mg (47 %) 13β-Trichloracryloyloxy-milbemycin D.
Smp. 160-163 °C
$^1$H-NMR (300 MHz; $CDCl_3$; TMS):
1,54 ppm (br.s) ($C_{14}CH_3$)
1,87 ppm (br. s) ($C_4CH_3$)
5,04 ppm (d, J = 10.6 Hz) ($C_{13}H$)

Nach den vorstehend beschriebenen Verfahren lassen sich beispielsweise auch folgende Verbindungen der Formel I herstellen:

13β-(p-Chlorbenzoyloxy)-milbemycin $A_4$:

$$IR\ \nu_{max}^{KBr}\ cm^{-1}$$

: 3470, 1720, 1595
Massenspectrum (m/e) : 696 ($M^+$), 568, 522
$^1$H-NMR (270 MHz, $CDCl_3$, TMS) $\delta_{ppm}$ : 3.97 (d, 1H, $C_6H$, J=6.2 Hz), 5.19 (d, 1H, $C_{13}H$, J=10.3 Hz), 7.42

(d, 2H, aromatisch, J = 8.8 Hz), 7.97 (d, 2H, aromatisch, J = 8.8 Hz)

13β-(p-Fluorphenoxyacetoxy)-milbemycin $A_4$:

$$IR\ \nu_{max}^{KBr}\ cm^{-1}$$

: 3460, 1760, 1735, 1720
Massenspectrum (m/e) : 540 ($M^+$-170), 522, 504
$^1$H-NMR (270 MHz, $CDCl_3$, TMS) $\delta_{ppm}$: 3.96 (d, 1H, $C_6H$, J = 6.2 Hz), 4.59

(br. s, 2H, $OC\underline{H_2}COO$),

5.05 (d, 1H, $C_{13}H$, J = 10.6 Hz), 6.75-6.85 (m, 2H, aromatisch), 6.9-7.05 (m, 2H, aromatisch)

5-O-Acetyl-13β-Formyloxy-milbemycin $A_4$:

$$IR\ \nu_{max}^{KBr}\ cm^{-1}$$

: 3450, 1730
Massenspectrum (m/e) : 628 ($M^+$), 570, 522, 458
$^1$H-NMR (270 MHz, $CDCl_3$, TMS) $\delta_{ppm}$: 2.16

(s, 3H, $OCOC\underline{H_3}$),

4.07 (d, 1H, $C_6H$, J = 6.2 Hz), 5.05 (d, 1H, $c_{13}H$, J = 10.3 Hz), 8.09 (s, 1H, OC$\underline{H}$O)

13β-(p-[tert.Butyl]benzoyloxy)-milbemycin $A_4$
Massenspektrum (m/e): 718 ($M^+$), 700, 590, 540, 522.
$^1$H-NMR (270 MHz, $CDCl_3$, TMS):
1,33 ppm (s) ($(CH_3)_3)CC_6H_4$)
5,19 ppm (d, J = 10 Hz) ($C_{13}H$)
7,35 ppm (d, J = 9 Hz) (2 H aromat.)
7,96 ppm (d, J = 9 Hz) (2 H aromat.)

13β-(3'-Chlorpropionyloxy-milbemycin $A_4$
Massenspektrum (m/e): 648 ($M^+$), 612, 540, 522.
$^1$H-NMR (270 MHz, $CDCl_3$, TMS):
2,80 ppm (t, J = 7 Hz) ($ClCH_2CH_2$
3,77 ppm (t, J = 7 Hz) ($ClCH_2\overline{CH_2}$)
5,00 ppm (d, J = 10 Hz) ($\overline{C_{13}}H$)

13β-(3',3',3'-Trifluorpropionyloxy)-milbemycin D:
$^1$H-NMR (250 MHz, $CDCl_3$, TMS):
3,17 ppm (q) ($CF_3CH_2$)
5,10 ppm (d, J = 10 Hz) ($c_{13}H$)

13β-(3',3',3'-Trifluorpropionyloxy)-milbemycin $A_4$:
$^1$H-NMR (300 MHz, $CDCl_3$, TMS):
3,15 ppm (q) ($CF_3CH_2$
5,01 ppm (d, J = 10 Hz) ($C_{13}H$)

13β-(2'-Chlor-3',3',3'-trifluorpropionyloxy)-milbemycin D:
$^1$H-NMR (250 MHz, $CDCl_3$, TMS):
4,60 ppm (q) ($CF_3CHCl$)
5,02 ppm (d, J = 10 Hz) ($C_{13}H$)

13β-Chloracetyl-milbemycin $A_4$:
$^1$H-NMR (300 MHz, $CDCl_3$, TMS):
4,06 ppm (s) ($ClCH_2$)
5,02 ppm (d, J = 10 Hz) ($C_{13}H$)

Analog zu den beschriebenen Arbeitsweisen werden auch die nachfolgend zusammen mit Verbindungen der vorgängigen Beispiele genannten Verbindungen der Formel I hergestellt:

Tabelle 1

Typische Vertreter von Verbindungen der Formel I, worin $R_1$ Wasserstoff ist.

| Verb. Nr. | $R_2$ | R |
|---|---|---|
| 1.01 | $CH_3$ | H |
| 1.02 | $C_2H_5$ | H |
| 1.03 | $C_3H_7$-iso | H |
| 1.04 | $C_4H_9$-sek | H |
| 1.05 | $CH_3$ | $C(CH_3)_3$ |
| 1.06 | $C_2H_5$ | $C(CH_3)_3$ |
| 1.07 | $C_3H_7$-iso | $C(CH_3)_3$ |
| 1.08 | $C_4H_9$-sek | $C(CH_3)_3$ |
| 1.09 | $CH_3$ | $CH_3OCH_2$ |
| 1.10 | $C_2H_5$ | $CH_3OCH_2$ |
| 1.11 | $C_3H_7$-iso | $CH_3OCH_2$ |
| 1.12 | $C_4H_9$-sek | $CH_3OCH_2$ |
| 1.13 | $CH_3$ | $CH_3OC(CH_3)_2$ |
| 1.14 | $C_2H_5$ | $CH_3OC(CH_3)_2$ |
| 1.15 | $C_3H_7$-iso | $CH_3OC(CH_3)_2$ |
| 1.16 | $C_4H_9$-sek | $CH_3OC(CH_3)_2$ |
| 1.17 | $CH_3$ | $CCl_3$ |
| 1.18 | $C_2H_5$ | $CCl_3$ |
| 1.19 | $C_3H_7$-iso | $CCl_3$ |
| 1.20 | $C_4H_9$-sek | $CCl_3$ |
| 1.21 | $CH_3$ | $CF_3$ |
| 1.22 | $C_2H_5$ | $CF_3$ |
| 1.23 | $C_3H_7$-iso | $CF_3CHCl$ |
| 1.24 | $C_4H_9$-sek | $CF_3$ |
| 1.25 | $CH_3$ | $C(Cl_3)CHCl$ |
| 1.26 | $C_2H_5$ | $C(Cl_3)CHCl$ |
| 1.27 | $C_3H_7$-iso | $CF_3CH_2$ |
| 1.28 | $C_4H_9$-sek | $C(Cl_3)CHCl$ |
| 1.29 | $CH_3$ | $ClCH_2CH_2CH_2$ |
| 1.30 | $C_2H_5$ | $ClCH_2CH_2CH_2$ |

Tabelle (Fortsetzung)

| Verb. Nr. | $R_2$ | R |
|---|---|---|
| 1.31 | $C_3H_7$-iso | $ClCH_2CH_2CH_2$ |
| 1.32 | $C_4H_9$-sek | $ClCH_2CH_2CH_2$ |
| 1.33 | $CH_3$ | $CH_2=CH$ |
| 1.34 | $C_2H_5$ | $CH_2=CH$ |
| 1.35 | $C_3H_7$-iso | $CH_2=CH$ |
| 1.36 | $C_4H_9$-sek | $CH_2=CH$ |
| 1.37 | $CH_3$ | $CH_2=CH-CH_2$ |
| 1.38 | $C_2H_5$ | $CH_2=CH-CH_2$ |
| 1.39 | $C_3H_7$-iso | $CH_2=CH-CH_2$ |
| 1.40 | $C_4H_9$-sek | $CH_2=CH-CH_2$ |
| 1.41 | $CH_3$ | $CH\equiv C-CH_2$ |
| 1.42 | $C_2H_5$ | $CH\equiv C-CH_2$ |
| 1.43 | $C_3H_7$-iso | $CH\equiv C-CH_2$ |
| 1.44 | $C_4H_9$-sek | $CH\equiv C-CH_2$ |
| 1.45 | $CH_3$ | $(CH_3)_2C=CH$ |
| 1.46 | $C_2H_5$ | $(CH_3)_2C=CH$ |
| 1.47 | $C_3H_7$-iso | $(CH_3)_2C=CH$ |
| 1.48 | $C_4H_9$-sek | $(CH_3)_2C=CH$ |
| 1.49 | $CH_3$ | $(Cl)_2C=C(Cl)$ |
| 1.50 | $C_2H_5$ | $(Cl)_2C=C(Cl)$ |
| 1.51 | $C_3H_7$-iso | $(Cl)_2C=C(Cl)$ |
| 1.52 | $C_4H_9$-sek | $(Cl)_2C=C(Cl)$ |
| 1.53 | $CH_3$ | $CF_3CCl_2$ |
| 1.54 | $C_2H_5$ | $CF_3CCl_2$ |
| 1.55 | $C_3H_7$-iso | $CF_3CCl_2$ |
| 1.65 | $C_4H_9$-sek | $CF_3CCl_2$ |
| 1.57 | $CH_3$ | Cyclopropyl |
| 1.58 | $C_2H_5$ | Cyclopropyl |
| 1.59 | $C_3H_7$-iso | Cyclopropyl |
| 1.60 | $C_4H_9$-sek | Cyclopropyl |
| 1.61 | $CH_3$ | 2,2-Dimethyl-cyclopropyl |
| 1.62 | $C_2H_5$ | 2,2-Dimethyl-cyclopropyl |
| 1.63 | $C_3H_7$-iso | 2,2-Dimethyl-cyclopropyl |
| 1.64 | $C_4H_9$-sek | 2,2-Dimethyl-cyclopropyl |

Tabelle (Fortsetzung)

| Verb. Nr. | $R_2$ | R |
|---|---|---|
| 1.65 | $CH_3$ | 2,2-Dimethyl-3-(2,2-dichlorvinyl)-cyclopropyl |
| 1.66 | $C_2H_5$ | 2,2-Dimethyl-3-(2,2-dichlorvinyl)-cyclopropyl |
| 1.67 | $C_3H_7$-iso | 2,2-Dimethyl-3-(2,2-dichlorvinyl)-cyclopropyl |
| 1.68 | $C_4H_9$-sek | 2,2-Dimethyl-3-(2,2-dichlorvinyl)-cyclopropyl |
| 1.69 | $CH_3$ | Cyclobutyl |
| 1.70 | $C_2H_5$ | Cyclobutyl |
| 1.71 | $C_3H_7$-iso | Cyclobutyl |
| 1.72 | $C_4H_9$-sek | Cyclobutyl |
| 1.73 | $CH_3$ | Cyclohexyl |
| 1.74 | $C_2H_5$ | Cyclohexyl |
| 1.75 | $C_3H_7$-iso | Cyclohexyl |
| 1.76 | $C_4H_9$-sek | Cyclohexyl |
| 1.77 | $CH_3$ | Phenyl |
| 1.78 | $C_2H_5$ | Phenyl |
| 1.79 | $C_3H_7$-iso | Phenyl |
| 1.80 | $C_4H_9$-sek | Phenyl |
| 1.81 | $CH_3$ | p-Chlorphenyl |
| 1.82 | $C_2H_5$ | p-Chlorphenyl |
| 1.83 | $C_3H_7$-iso | p-Chlorphenyl |
| 1.84 | $C_4H_9$-sek | p-Chlorphenyl |
| 1.85 | $CH_3$ | p-Tolyl |
| 1.86 | $C_2H_5$ | p-Tolyl |
| 1.87 | $C_3H_7$-iso | p-Tolyl |
| 1.88 | $C_4H_9$-sek | p-Tolyl |
| 1.89 | $CH_3$ | p-Nitrophenyl |
| 1.90 | $C_2H_5$ | p-Nitrophenyl |
| 1.91 | $C_3H_7$-iso | p-Nitrophenyl |
| 1.92 | $C_4H_9$-sek | p-Nitrophenyl |
| 1.93 | $CH_3$ | $ClCH_2C(CH_3)_2$ |
| 1.94 | $C_2H_5$ | $ClCH_2C(CH_3)_2$ |
| 1.95 | $C_3H_7$-iso | $ClCH_2C(CH_3)_2$ |
| 1.96 | $C_4H_9$-sek | $ClCH_2C(CH_3)_2$ |
| 1.97 | $CH_3$ | 1-Methylcyclopropyl |
| 1.98 | $C_2H_5$ | 1-Methylcyclopropyl |

Tabelle (Fortsetzung)

| Verb. Nr. | $R_2$ | R |
|---|---|---|
| 1.99 | $C_3H_7$-iso | 1-Methylcyclopropyl |
| 2.00 | $C_4H_9$-sek | 1-Methylcyclopropyl |
| 2.01 | $CH_3$ | Adamantyl |
| 2.02 | $C_2H_5$ | Adamantyl |
| 2.03 | $C_3H_7$-iso | Adamantyl |
| 2.04 | $C_4H_9$-sek | Adamantyl |
| 2.05 | $C_2H_5$ | p-Fluorphenoxymethyl |
| 2.06 | $C_2H_5$ | $ClC(CH_3)_2$ |
| 2.07 | $C_2H_5$ | $CH_3CCl_2$ |
| 2.08 | $C_2H_5$ | $ClCH_2$ |
| 2.09 | $C_2H_5$ | $CF_3CH_2$ |
| 2.10 | $C_2H_5$ | 1-Methylcyclobutyl |
| 2.11 | $C_2H_5$ | 1-Methylcyclopentyl |
| 2.12 | $C_2H_5$ | $FCH_2C(CH_3)_2$ |
| 2.13 | $C_2H_5$ | $CH_2=C(CH_3)$ |
| 2.14 | $C_2H_5$ | $ClCH_2CH_2$ |
| 2.15 | $C_2H_5$ | p-(tert.$C_4H_9$)phenyl |

Formulierungsbeispiele für den Wirkstoff der Formel I

(% = Gewichtsprozent)

| Spritzpulver | a) | b) | c) |
|---|---|---|---|
| Wirkstoff aus Tabelle 1 | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyethylenglykol-ether (7-8 Mol AeO) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| Emulsions-Konzentrat | |
|---|---|
| Wirkstoff aus Tabelle 1 | 10 % |
| Octylphenolpolyethylenglykolether (4-5 Mol AeO) | 3 % |
| Ca-Dodecylbenzolsulfonat | 3 % |
| Ricinusölpolyglykolether (36 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| Stäubemittel | a) | b) |
|---|---|---|
| Wirkstoff aus Tabelle 1 | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

| Extruder Granulat | |
|---|---|
| Wirkstoff aus Tabelle 1 | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

| Tabletten, Pellets | | |
|---|---|---|
| I | Wirkstoff aus Tabelle 1 | 33,0 % |
| | Methylcellulose | 0,80 % |
| | Kieselsäure hochdispers | 0,80 % |
| | Maisstärke | 8,40 % |

Methylcellulose in Wasser einrühren und quellen lassen; Kieselsäure in die Quellung einrühren und homogen suspendieren. Wirkstoff und Maisstärke mischen. In diese Mischung die wässrige Suspension einarbeiten und zu einem Teig kneten. Diese Masse durch ein Sieb (Maschenweite 12 M) granulieren und dann trocknen.

| | | |
|---|---|---|
| II | Milchzucker krist. | 22,50 % |
| | Maisstärke | 17,00 % |
| | mikrokrist. Cellulose | 16,50 % |
| | Magnesiumstearat | 1,00 % |

Alle 4 Hilfsstoffe gut mischen
Phasen I und II mischen und zu Tabletten oder Pellets verpressen.

Bei einer Verwendung von Verbindungen der Formel I oder entsprechender Mittel zur Bekämpfung von endoparasitären Nematoden, Cestoden und Trematoden bei Haus- und Nutztieren, wie Rindern, Schafen, Ziegen, Katzen und Hunden, können die Verbindungen oder Mittel den Tieren sowohl als Einzeldosis wie auch wiederholt verabreicht werden, wobei die einzelnen Gaben je nach Tierart vorzugsweise zwischen 0,1 und 10 mg pro kg Körpergewicht betragen. Durch eine protrahierte Verabreichung erzielt man in manchen Fällen eine bessere Wirkung oder man kann mit geringeren Gesamtdosen auskommen. Der Wirkstoff bzw. die ihn enthaltenden Mittel können auch dem Futter oder den Tränken zugesetzt werden. Das Fertigfutter enthält die Wirkstoffkombinationen vorzugsweise in einer Konzentration von 0,005 bis 0,1 Gew. %. Die Mittel können in Form von Lösungen, Emulsionen, Suspensionen, Pulver, Tabletten, Pellets, Bolussen oder Kapseln peroral den Tieren verabreicht werden. Soweit die physikalischen und toxikologischen Eigenschaften von Lösungen oder Emulsionen dies zulassen, können die Verbindungen der Formel I bzw. die sie enthaltenden Mittel den Tieren auch beispielsweise subcutan injiziert, intraruminal verabreicht oder mittels der Pour-on-Methode auf den Körper der Tiere appliziert werden. Ferner ist eine Verabreichung des Wirkstoffs an die Tiere auch durch Lecksteine (Salz) oder Molasse-Blöcke möglich.

Biologische Beispiele

1. Insektizide Frassgift-Wirkung bei Spodoptera littoralis

Eingetopfte Baumwollpflanzen im 5-Blatt-Stadium werden mit einer acetonisch/wässrigen Versuchslösung besprüht, die 1, 3, 12,5 oder 50 ppm der zu prüfenden Verbindung enthält.

Nach dem Antrocknen des Belags werden die Pflanzen mit ca. 30 Larven ($L_1$-Stadium) von Spodoptera littoralis besetzt. Pro Versuchsverbindung und pro Test-Spezies verwendet man zwei Pflanzen. Der Versuch wird bei ca. 24° C und 60 % relativer Luftfeuchtigkeit durchgeführt. Auswertungen und Zwischenauswertungen auf moribunde Tiere, Larven und Frassschäden erfolgen nach 24 Stunden, 48 Stunden und 72 Stunden.

Die Verbindungen der Formeln I erzielen bereits bei einer Wirkstoffkonzentration von 3 ppm eine vollständige Abtötung nach 24 Stunden. Besonders bemerkenswert sind hierbei die Verbindungen Nr. 1.06, 1.23, 1.27, 1.71, 1.94, 1.95 und 1.99.

2. Wirkung gegen pflanzenschädigende Akariden OP-sensible Tetranychus urticae

Die Primärblätter von Bohnenpflanzen (Phaseolus vulgaris) werden 16 Stunden vor dem Versuch mit einem durch T. urticae infestierten, aus einer Massenzucht stammenden Blattstück belegt. Die so mit allen Milben-Stadien infestierten Pflanzen werden nach Entfernung des Blattstücks mit einer Versuchslösung bis zur Tropfnässe besprüht, die wahlweise 0,2 ppm, 0,4 ppm oder 1,6 ppm der zu prüfenden Verbindung enthält. Die Temperatur in der Gewächshauskabine beträgt ca. 25° C.

Nach sieben Tagen wird unter dem Binokular auf den Prozentsatz mobiler Stadien (Adulte und Nymphen) und auf vorhandene Eier ausgewertet.

Die Verbindungen der Formel I erzielen bei einer Wirkstoffkonzentration von 0,4 ppm vollständige Abtötung. Besonders bemerkenswert sind hierbei die Verbindungen Nr. 1.07 und 1.99.

3. Wirkung gegen $L_1$- Larven von Lucilia sericata

1 ml einer wässrigen Suspension der zu prüfenden Aktivsubstanz werden so mit 3 ml eines speziellen Larvenzuchtmediums bei ca. 50° C vermischt, dass ein Homogenisat von wahlweise 250 ppm oder 100 ppm Wirkstoffgehalt entsteht. In jede Reagenzglas-Probe werden ca. 30 Lucilia-Larven ($L_1$) eingesetzt. Nach 4 Tagen wird die Mortalitätsrate bestimmt. Die Verbindungen der Formel I erzielen mit 250 ppm, die nachfolgend genannten Verbindungen Nr. 1.06, 1.07, 1.23, 1.31, 1.51, 1.59, 1.67, 1.71, 1.82, 1.95, 1.98, 1.99

und 2.03 bereits mit 100 ppm, eine Wirkung von 100 %.

4. Akarizide Wirkung gegen Boophilus microplus (Biarra-Stamm)

Auf einer PVC-Platte wird waagrecht ein Klebstreifen so befestigt, das darauf 10 mit Blut vollgesogene Zecken-Weibchen von Boophilus microplus (Biarra-Stamm) nebeneinander in einer Reihe mit dem Rücken aufgeklebt werden können. Jeder Zecke wird mit einer Injektionsnadel 1 µl einer Flüssigkeit injiziert, die eine 1:1-Mischung von Polyethylenglykol und Aceton darstellt und in der eine bestimmte Wirkstoffmenge von wahlweise 1, 0,5, 0,1 oder 0,01 µg pro Zecke gelöst ist. Kontrolltiere erhalten eine wirkstofffreie Injektion. Nach der Behandlung werden die Tiere unter Normalbedingungen in einem Insektarium bei ca. 28 °C und 80 % relativer Luftfeuchtigkeit gehalten, bis die Eiablage erfolgt und die Larven aus den Eiern der Kontrolltiere geschlüpft sind.

Die Aktivität einer geprüften Substanz wird mit der $IR_{90}$ bestimmt, d.h. es wird jene Wirkstoffdosis ermittelt, bei der noch nach 30 Tagen 9 von 10 Zeckenweibchen (= 90 %) Eier ablegen. die nicht schlupffähig sind.

Die Verbindungen der Formeln I erzielen eine $IR_{90}$ von 0,5 µg. Besonders erwähnenswert sind hier die Verbindungen Nr. 1.03, 1.06, 1.07, 1.51, 1.67 und 1.95.

5. Versuch an mit Nematoden (Haemonchus concortus und Trichostrongylus colubriformis) infizierten Schafen

Der Wirkstoff wird als Suspension formuliert mit einer Magensonde oder durch Injektion in den Pansen eines Schafes gegeben, das mit Haemonchus concortus und Trichostrongylus colubriformis künstlich infiziert worden ist. Pro Dosis werden 1 bis 3 Tiere verwendet. Jedes Schaf wird nur einmal mit einer einzigen Dosis behandelt, und zwar mit 0,5 mg/kg Körpergewicht. Die Evaluierung erfolgt durch Vergleich der Anzahl der vor und nach Behandlung im Kot der Schafe ausgeschiedenen Wurmeier.

Gleichzeitig und gleichartig infizierte aber unbehandelte Schafe dienen als Kontrolle. Schafe, die mit einer der Verbindungen der Formel I bei 0,5 mg/kg behandelt werden, zeigen im Vergleich zu unbehandelten, aber infizierten Vergleichsgruppen keinen Nematodenbefall (= komplette Reduktion der Wurmeier im Kot). Besonders bemerkenswert sind hierbei die Verbindungen Nr. 1.06, 1.07, 1.23, 1.95 und 1.99.

6. Kontaktwirkung auf Aphis craccivora

Erbsenkeimlinge, die mit sämtlichen Entwicklungsstadien der Laus infiziert sind, werden mit einer aus einem Emulsionskonzentrat hergestellten Wirkstofflösung besprüht, die wahlweise 50 ppm, 25 ppm oder 12,5 ppm Wirkstoff enthält. Nach 3 Tagen wird auf mehr als 80 % tote bzw. abgefallene Blattläuse ausgewertet. Nur bei dieser Wirkungshöhe wird ein Präparat als wirksam eingestuft.

Die Verbindungen der Formel I erzielen bei einer Konzentration von 12,5 ppm eine vollständige Abtötung (= 100 %). Besonders bemerkenswert sind hierbei die Verbindungen Nr. 1.06, 1.07, 1.31, 1.67, 1.71, 1.94, 1.95, 1.99 und 2.03.

7. Larvizidwirkung gegen Aedes aegypti

Auf die Oberfläche von 150 ml Wasser, das sich in einem Behälter befindet, wird soviel einer 0,1 %igen acetonischen Lösung des Wirkstoffes pipettiert, dass Konzentrationen von wahlweise 10 ppm, 3,3 ppm und 1,6 ppm erhalten werden. Nach Verdunsten des Acetons wird der Behälter mit ca. 30-40 3 Tage alten Aedes-Larven beschickt. Nach 1, 2 und 5 Tagen wird die Mortalität geprüft.

Die Verbindungen der Formeln I bewirken in diesem Test bei einer Konzentration von 1,6 ppm bereits nach einem Tag eine vollständige Abtötung sämtlicher Larven. Besonders hervorzuheben sind hierbei die Verbindungen Nr. 1.07, 1.23, 1.27, 1.31, 1.51, 1.59, 1.67, 1.71, 1.94, 1.95, 1.98, 1.99 und 2.03.

**Ansprüche**

**1.** Verbindungen der Formel I

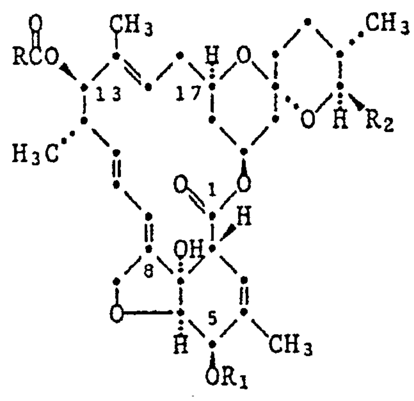

(I)

in welcher

$R_1$ Wasserstoff, die Silylgruppe $-Si(R_6)(R_7)(R_8)$, worin $R_6$, $R_7$ und $R_8$ unabhängig voneinander für $C_1$-$C_4$-Alkyl, Benzyl oder Phenyl stehen, oder die Acylgruppe $R_5$-C(O)-, worin $R_5$ für $C_1$-$C_{10}$-Alkyl, $c_1$-$C_{10}$-Haloalkyl oder einen unsubstituierten oder durch Substituenten der Gruppe bestehend aus Halogen, $C_1$-$C_3$-Alkyl, $C_1$-$C_3$-Haloalkyl, $C_1$-$C_3$-Alkoxy, $C_1$-$C_3$-Haloalkoxy, Nitro und Cyano substituierten Rest aus der Gruppe Phenyl und Benzyl steht,

$R_2$ Methyl, Ethyl, Isopropyl oder sek.Butyl und

R Wasserstoff, tert.Butyl, geradkettige oder verzweigte, durch 1 bis 7 Halogenatome, 1 bis 6 $C_1$-$C_6$-Alkoxygruppen oder eine unsubstituierte oder halogenierte Phenoxygruppe substituierte $C_1$-$C_{18}$-Alkylgruppen, mono- bis tetracyclische, aliphatische Gruppen mit 3 bis 10 Kohlenstoffatomen, welche in Verbindungen, in denen $R_1$ für Wasserstoff und $R_2$ für Aethyl oder Isopropyl steht, durch Substituenten der Gruppe bestehend aus $C_1$-$C_4$-Alkyl und halogeniertem $C_2$-$C_4$-Alkenyl ein- oder mehrfach substituiert sein können, durch 1 bis 7 Halogenatome, 1 bis 6 $C_1$-$C_6$-Alkoxygruppen oder durch $C_1$-$C_4$-Alkylgruppen substituierte Cycloalkylgruppen mit 3 bis 10 Kohlenstoffatomen, unsubstituierte oder durch 1 bis 7 Halogenatome oder 1 bis 6 $C_1$-$C_6$-Alkoxygruppen substituierte $C_2$-$C_6$-Alkenyl-oder Alkinylgruppen oder eine unsubstituierte oder durch 1 bis 3 Substituenten ausgewählt aus der Gruppe bestehend aus Halogenatomen, $C_1$-$C_6$-Alkyl, $C_1$-$C_6$-Alkoxy, $C_1$-$C_4$-Alkylthio oder Nitro substituierte Phenyl- oder Benzylgruppe bedeuten.

**2.** Verbindungen der Formel I nach Anspruch 1, worin $R_1$ Wasserstoff darstellt, $R_2$ für Methyl, Ethyl, Isopropyl oder sek.Butyl steht und R Wasserstoff, tert.Bütyl, durch 1 bis 4 Halogenatome oder $C_1$-$C_4$-Alkoxy substituiertes $C_1$-$C_6$-Alkyl, $C_2$-$C_4$-Alkenyl, $C_2$-$C_4$-Alkinyl oder $C_3$-$C_6$-Cycloalkyl; oder unsubstituiertes oder ein- bis dreifach durch Halogen, $C_1$-$C_4$-Alkyl, $C_1$-$C_4$-Alkoxy, $C_1$-$C_4$-Alkylthio oder Nitro substituiertes Phenyl bedeutet.

**3.** Verbindungen der Formel I nach Anspruch 1, worin $R_1$ Wasserstoff darstellt, $R_2$ für Methyl, Ethyl, Isopropyl oder sek.Butyl steht und R Wasserstoff, tert.Bütyl. durch 1 bis 4 Chlor- oder Fluoratome oder Methoxy substituiertes $C_1$-$C_4$-Alkyl, $C_2$-$C_3$-Alkenyl, $C_2C_3$-Alkinyl oder $C_3$-$C_6$-Cycloalkyl; oder unsubstituiertes oder durch Chlor, Fluor, $C_1$-$C_2$-Alkyl, $C_1$-$C_2$-Alkoxy, $C_1$-$C_2$-Alkylthio oder Nitro substituiertes Phenyl bedeutet.

**4.** Verbindungen der Formel I nach Anspruch 1, worin $R_1$ Wasserstoff darstellt, $R_2$ für Ethyl oder Isopropyl steht und R Wasserstoff, tert.Bütyl, $C_1$-$C_8$-Alkyl, welches durch $C_1$-$C_4$-Alkoxy oder ein- bis dreifach halogeniertes Phenoxy monosubstituiert oder durch 1 bis 5 Halogenatome substituiert ist, eine unsubstituierte oder durch Substituenten der Gruppe bestehend aus $C_1$-$C_4$-Alkyl und halogeniertem $C_2$-$C_4$-Alkenyl ein- oder mehrfach substituierte, mono-bis tetracyclische, aliphatische Gruppe mit insgesamt 3 bis 10 Kohlenstoffatomen im Ring oder Ringsystem, ein- bis dreifach halogeniertes $C_2$-$C_4$-Alkenyl, $C_3$-$C_4$-Alkinyl oder ein- bis dreifach durch Substituenten der Gruppe bestehend aus Halogen, $C_1$-$C_4$-Alkyl und Nitro substituiertes Phenyl bedeutet.

**5.** Verbindungen der Formel I nach Anspruch 1, worin $R_1$ Wasserstoff darstellt, $R_2$ für Ethyl oder Isopropyl steht und R Wasserstoff, tert.Bütyl, $C_1$-$C_8$-Alkyl, welches durch Substituenten der Gruppe bestehend aus Chlor und Fluor ein- bis dreifach substituiert ist, Fluorphenoxymethyl, unsubstituiertes oder durch eine Methylgruppe substituiertes $C_3$-$C_4$-Cycloalkyl, Adamantyl, Trichlorvinyl oder Monochlorphenyl

bedeutet.

6. Verbindungen der Formel I nach Anspruch 1, worin $R_1$ den Rest -Si($R_6$)($R_7$)($R_8$) darstellt, wobei $R_6$, $R_7$ und $R_8$ unabhängig voneinander $C_1$-$C_4$-Alkyl, Benzyl oder Phenyl bedeuten, und R und $R_2$ die in Anspruch 1 angegebenen Bedeutungen besitzen.

7. Verbindungen der Formel I nach Anspruch 1, worin $R_1$ einen Rest aus der Gruppe Trimethylsilyl, Diphenyl-tert.butylsilyl, bis(Isopropyl)-methylsilyl, Triphenylsilyl und tert.Butyl-dimethylsilyl bedeutet.

8. Verbindungen der Formel I nach Anspruch 1, worin $R_1$ den Rest $R_5$-C(O)- darstellt, wobei $R_5$ $C_1$-$C_{10}$-Alkyl, $C_1$-$C_{10}$-Haloalkyl oder einen unsubstituierten oder durch Substituenten der Gruppe bestehend aus Halogen, $C_1$-$C_3$-Alkyl, $C_1$-$C_3$-Haloalkyl, $C_1$-$C_3$-Alkoxy, $C_1$-$C_3$-Haloalkoxy, Cyano und Nitro substituierten Rest aus der Gruppe Phenyl und Benzyl bedeutet, und R und $R_2$ die in Anspruch 1 angegebenen Bedeutungen hat.

9. Verbindung nach Anspruch 1, ausgewählt aus der Gruppe:
13β-Formyloxy-milbemycin D
13β-Pivaloyloxy-milbemycin D
13β-Formyloxy-milbemycin $A_3$
13β-Pivaloyloxy-milbemycin $A_3$
13β-Formyloxy-milbemycin $A_4$
13β-Pivaloyloxy-milbemycin $A_4$.
13β-(2'-Methoxy-2'-methylpropionyloxy)-milbemycin D
13β-(2'-Methoxy-2'-methylpropionyloxy)-milbemycin $A_4$
13β-Trichloracetoxy-milbemycin D
13β-(4'-Chlor-butanoyloxy)milbemycin D
13β-Trichloracryloyloxy-milbemycin D
13β-Cyclopropancarbonyloxy-milbemycin D
13β-Cyclobutancarbonyloxy-milbemycin D
13β-(3'-Chlor-2'2'-dimethyl-propionyloxy)-milbemycin $A_4$
13β-(3'Chlor-2'2'-dimethyl-propionyloxy)-milbemycin D
13β-(1'Methyl-cyclopropancarbonyloxy)-milbemycin $A_4$
13β-(1'-Methyl-cyclopropancarbonyloxy)-milbemycin D
13β-(1-Adamantancarbonyloxy)-milbemycin D
13β-(p-Fluorphenoxyacetoxy)-milbemycin $A_4$
13β-(2'Chlor-2'-methyl-propionyloxy)-milbemycin $A_4$
13β-(2'2'-Dichlorpropionyloxy)-milbemycin $A_4$
13β-(p-Chlorbenzoyloxy)-milbemycin $A_4$
13β-(3',3',3'-Trifluorpropionyloxy)-milbemycin $A_4$
13β-Chloracetoxy-milbemycin $A_4$
13β-(2'-Chlor-3',3',3'-Trifluorpropionyloxy-milbemycin D
13β-(3',3',3'-Trifluorpropionyloxy)-milbemycin D.

10. Verbindung nach Anspruch 1, ausgewählt aus der Gruppe:
5-O-tert.Butyldimethylsilyl-13β-Formyloxy-milbemycin D
5-O-tert-Butyldimethylsilyl-13β-Pivaloyloxy-milbemycin D
5-O-tert.Butyldimethylsilyl-13β-Formyloxy-milbemycin $A_3$
5-O-tert.Butyldimethylsilyl-13β-Pivaloyloxy-milbemycin $A_3$
5-O-tert.Butyldimethylsilyl-13β-Formyloxy-milbemycin $A_4$
5-O-tert.Butyldimethylsilyl-13β-Pivaloyloxy-milbemycin $A_4$.
5-O-tert.Butyldimethylsilyl-13β-(2'-Methoxy-2'-methylpropionyloxy)-milbemycin D
5-O-tert.Butyldimethylsilyl-13β-(2'-Methoxy-2'-methylpropionyloxy)-milbemycin $A_4$
5-O-tert.Butyldimethylsilyl-13β-Trichloracetoxy-milbemycin D
5-O-tert.Butyldimethylsilyl-13β-(4'-Chlor-butanoyloxy)milbemycin D
5-O-tert.Butyldimethylsilyl-13β-Trichloracryloyloxy-milbemycin D
5-O-tert.Butyldimethylsilyl-13β-Cyclopropancarbonyloxy-milbemycin D
5-O-tert.Butyldimethylsilyl-13β-Cyclobutancarbonyloxy-milbemycin D

5-O-tert.Butyldimethylsilyl-13β-(3'-Chlor-2'2'-dimethyl-propionyloxy)-milbemycin $A_4$
5-O-tert.Butyldimethylsilyl-13β-(3'-Chlor-2'2-dimethyl-propionyloxy)-milbemycin D
5-O-tert.Butyldimethylsilyl-13β-(1'-Methyl-cyclopropancarbonyloxy)-milbemycin $A_4$
5-O-tert.Butyldimethylsilyl-13β-(1'-Methyl-cyclopropancarbonyloxy)-milbemycin D
5-O-tert.Butyldimethylsilyl-13β-(1-Adamantancarbonyloxy)-milbemycin D
5-O-tert.Butyldimethylsilyl-13β-(p-Fluorphenoxyacetoxy)-milbemycin $A_4$
5-O-tert.Butyldimethylsilyl-13β-(2'-Chlor-2'-methyl-propionyloxy)-milbemycin $A_4$
5-O-tert.Butyldimethylsilyl-13β-(2',2'-Dichlorpropionyloxy)-milbemycin $A_4$
5-O-tert.Butyldimethylsilyl-13β-(p-Chlorbenzoyloxy)-milbemycin $A_4$
5-O-tert.Butyldimethylsilyl-13β-(3',3',3'-Trifluorpropionyloxy)-milbemycin $A_4$
5-O-tert.Butyldimethylsilyl-13β-Chloracetoxy-milbemycin $A_4$
5-O-tert.Butyldimethylsilyl-13β-(2'-Chlor-3',3',3'-Trifluorpropionyloxy)-milbemycin D
5-O-tert.Butyldimethylsilyl-13β-(3',3',3'-Trifluorpropionyloxy)-milbemycin D.

**11.** Verfahren zur Herstellung von Verbindungen der Formel I

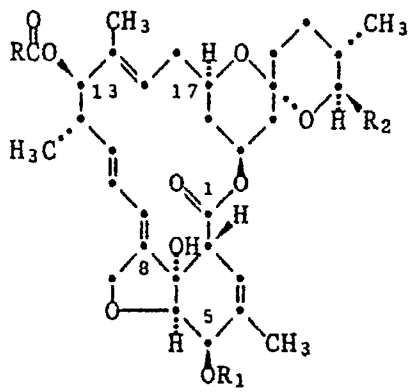

(I)

in welcher
$R_1$ Wasserstoff, die Silylgruppe $-Si(R_6)(R_7)(R_8)$, worin $R_6$, $R_7$ und $R_8$ unabhängig voneinander für $C_1$-$C_4$-Alkyl, Benzyl oder Phenyl stehen, oder die Acylgruppe $R_5$-C(O)-, worin $R_5$ für $C_1$-$C_{10}$-Alkyl, $c_1$-$C_{10}$-Haloalkyl oder einen unsubstituierten oder durch Substituenten der Gruppe bestehend aus Halogen, $C_1$-$C_3$-Alkyl, $C_1$-$C_3$-Haloalkyl, $C_1$-$C_3$-Alkoxy, $C_1$-$C_3$-Haloalkoxy, Nitro und Cyano substituierten Rest aus der Gruppe Phenyl und Benzyl steht,
$R_2$ Methyl, Ethyl, Isopropyl oder sek.Butyl und
R Wasserstoff, tert.Butyl, geradkettige oder verzweigte, durch 1 bis 7 Halogenatome, 1 bis 6 $C_1$-$C_6$-Alkoxygruppen oder eine unsubstituierte oder halogenierte Phenoxygruppe substituierte $C_1$-$C_{18}$-Alkylgruppen, mono- bis tetracyclische, aliphatische Gruppen mit 3 bis 10 Kohlenstoffatomen, welche in Verbindungen, in denen $R_1$ für Wasserstoff und $R_2$ für Aethyl oder Isopropyl steht, durch Substituenten der Gruppe bestehend aus $C_1$-$C_4$-Alkyl und halogeniertem $C_2$-$C_4$-Alkenyl ein- oder mehrfach substituiert sein können, durch 1 bis 7 Halogenatome, 1 bis 6 $C_1$-$C_6$-Alkoxygruppen oder durch $C_1$-$C_4$-Alkylgruppen substituierte Cycloalkylgruppen mit 3 bis 10 Kohlenstoffatomen, unsubstituierte oder durch 1 bis 7 Halogenatome oder 1 bis 6 $C_1$-$C_6$-Alkoxygruppen substituierte $C_2$-$C_6$-Alkenyl-oder Alkinylgruppen oder eine unsubstituierte oder durch 1 bis 3 Substituenten ausgewählt aus der Gruppe bestehend aus Halogenatomen, $C_1$-$C_6$-Alkyl, $C_1$-$C_6$-Alkoxy, $C_1$-$C_4$-Alkylthio oder Nitro substituierte Phenyl- oder Benzylgruppe bedeuten, dadurch gekennzeichnet, dass man Verbindungen der Formel II

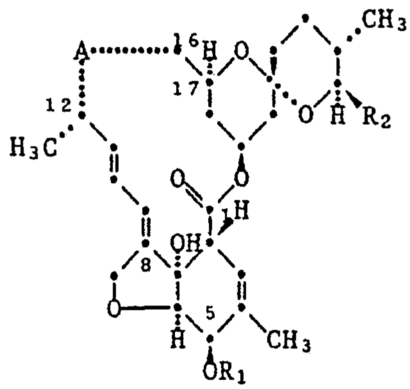

(II)

worin A für eine Gruppe a oder b

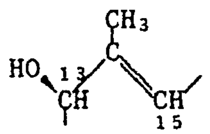

(a)

oder

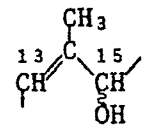

(b)

[= 13β-Hydroxy-$\Delta^{14,15}$] [= $\Delta^{13,14}$-15-hydroxy]

steht, $R_1$ eine Schutzgruppe bedeutet und $R_2$ die unter Formel I angegebenen Bedeutungen besitzt, mit einem zur Einführung einer 13β-Estergruppe geeigneten Reagenz behandelt, woraufhin die $R_1$-Schutzgruppe, sofern freie 5-Hydroxy-Verbindungen erwünscht sind, abgespalten wird.

**12.** Verfahren nach Anspruch 11, dadurch gekennzeichnet, dass zur Einführung einer 13β-Estergruppe in Verbindungen der Formel II als Reagenz eingesetzt wird

i) bei Verbindungen der Formeln IIa oder IIb:

a) eine Säure der Formel III

RCOOH (III)

oder

b) ein Säureamid der Formel IV

$RCON(Alkyl)_2$ (IV),

worin die Alkylreste 1 bis 4 Kohlenstoffatome umfassen und vorzugsweise Methyl darstellen, oder

ii) bei Verbindungen der Formel IIa:

c) ein Säurehalogenid der Formel V

RCOhal (V)

worin hal Halogen, bevorzugt Chlor oder Brom bedeutet, oder

d) ein Säureanhydrid der Formel VI

$(RCO)_2O$ (VI)

in welchen R die unter Formel I angegebenen Bedeutungen besitzt.

**13.** Verfahren nach Anspruch 12, dadurch gekennzeichnet, dass die Reaktion von Verbindungen der Formel IIa oder IIb mit einer Säure der Formel III oder einem Säureamid der Formel IV in Gegenwart eines Orthoesters der Formel VII

$R_3C(OR_4)_3$ (VII)

worin $R_3$ Wasserstoff oder $C_1$-$C_4$-Alkyl und $R_4$ $C_1$-$C_4$-Alkyl bedeuten und zusätzlich in Gegenwart einer katalytisch wirkenden Säure bei Temperaturen im Bereich von 0° bis 150° C durchgeführt wird.

**14.** Verfahren nach Anspruch 12, dadurch gekennzeichnet, dass die Reaktion von Verbindungen der Formel IIa mit einem Säurechlorid oder Säurebromid der Formel V oder einem Säureanhydrid der Formel VI in einem reaktionsinerten Lösungsmittel bei Temperaturen von -20° bis 100° C durchgeführt wird.

**15.** Verfahren nach Anspruch 14, dadurch gekennzeichnet, dass als Neutralisationsmittel eine Base eingesetzt wird.

**16.** Mittel zur Bekämpfung von Schädlingen, dadurch gekennzeichnet, dass es neben Trägermitteln, Verteilungsmitteln oder Träger- und Verteilungsmitteln als Wirkstoff mindestens eine Verbindung der Formel I gemäss Anspruch 1 enthält.

**17.** Verfahren zur Bekämpfung von Schädlingen, dadurch gekennzeichnet, dass man eine pestizid-wirksame Menge mindestens einer Verbindung der Formel I gemäss Anspruch 1 auf oder in die Wirtspflanzen oder sonstigen Lebensräume der Schädlinge mit Ausnahme menschlicher oder tierischer Körper appliziert.

**18.** Verfahren nach Anspruch 17, dadurch gekennzeichnet, dass die zu bekämpfenden Schädlinge Tiere befallende Endo- oder Ektoparasiten sind.

**19.** Verfahren nach Anspruch 17, dadurch gekennzeichnet, dass die zu bekämpfenden Schädlinge pflanzenschädigende Parasiten sind.

**20.** Verbindungen der Formel I gemäss Anspruch 1 zur Verwendung als antiparasitäres Mittel gegen Tiere befallende Endo- und Ektoparasiten.

**21.** Verwendung einer Verbindung der Formel I gemäß Auspruch 1 zur Herstellung eines Arzneimittels zur Bekämpfung von Tiere befallenden Endo- und Ektoparasiten.

Patentansprüche für folgenden Vertragsstaat: AT

**1.** Mittel zur Bekämpfung von Schädlingen, dadurch gekennzeichnet, dass es neben Trägermitteln, Verteilungsmitteln oder Träger- und Verteilungsmitteln als Wirkstoff mindestens eine Verbindung der Formel I

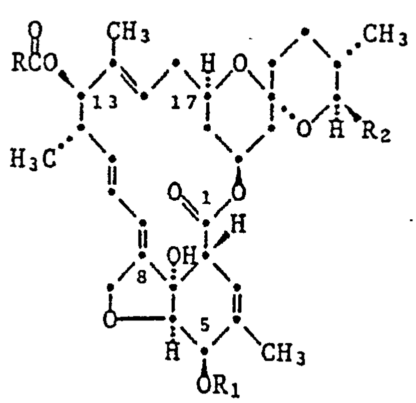

(I)

in welcher

$R_1$ Wasserstoff, die Silylgruppe -Si($R_6$)($R_7$)($R_8$), worin $R_6$, $R_7$ und $R_8$ unabhängig voneinander für $C_1$-$C_4$-Alkyl, Benzyl oder Phenyl stehen, oder die Acylgruppe $R_5$-C(O)-, worin $R_5$ für $C_1$-$C_{10}$-Alkyl, $C_1$-$C_{10}$-Haloalkyl oder einen unsubstituierten oder durch Substituenten der Gruppe bestehend aus Halogen, $C_1$-$C_3$-Alkyl, $C_1$-$C_3$-Haloalkyl, $c_1$-$C_3$-Alkoxy, $C_1$-$C_3$-Haloalkoxy, Nitro und Cyano substituierten Rest aus der Gruppe Phenyl und Benzyl steht,
$R_2$ Methyl, Ethyl, Isopropyl oder sek.Butyl und
R Wasserstoff, tert.Butyl, geradkettige oder verzweigte, durch 1 bis 7 Halogenatome, 1 bis 6-$C_1$-$C_6$-Alkoxygruppen oder eine unsubstituierte oder halogenierte Phenoxygruppe substituierte $C_1$-$C_{18}$-Alkylgruppen, mono- bis tetracyclische, aliphatische Gruppen mit 3 bis 10 Kohlenstoffatomen, welche in Verbindungen, in denen $R_1$ für Wasserstoff und $R_2$ für Aethyl oder Isopropyl steht, durch Substituenten der Gruppe bestehend aus $C_1$-$C_4$-Alkyl und halogeniertem $C_2$-$C_4$-Alkenyl ein- oder mehrfach substituiert sein können, durch 1 bis 7 Halogenatome, 1 bis 6 $C_1$-$C_6$-Alkoxygruppen oder durch $C_1$-$C_4$-Alkylgruppen substituierte Cycloalkylgruppen mit 3 bis 10 Kohlenstoffatomen, unsubstituierte oder durch 1 bis 7 Halogenatome oder 1 bis 6 $C_1$-$C_6$-Alkoxygruppen substituierte $C_2$-$C_6$-Alkenyl-oder Alkinylgruppen oder eine unsubstituierte oder durch 1 bis 3 substituenten ausgewählt aus der Gruppe bestehend aus Halogenatomen, $C_1$-$C_6$-Alkyl, $C_1$-$C_6$-Alkoxy, $C_1$-$C_4$-Alkylthio oder Nitro substituierte Phenyl- oder Benzylgruppe bedeuten.

**2.** Mittel nach Anspruch 1, welches eine Verbindung der Formel I enthält, worin $R_1$ Wasserstoff darstellt, $R_2$ für Methyl, Ethyl, Isopropyl oder sek.Butyl steht und R Wasserstoff, tert.Bütyl, durch 1 bis 4 Halogenatome oder $C_1$-$C_4$-Alkoxy substituiertes $C_1$-$C_6$-Alkyl, $C_2$-$C_4$-Alkenyl, $C_2$-$C_4$-Alkinyl oder $C_3$-$C_6$-Cycloalkyl; oder unsubstituiertes oder ein- bis dreifach durch Halogen, $C_1$-$C_4$-Alkyl, $C_1$-$C_4$-Alkoxy, $C_1$-$C_4$-Alkylthio oder Nitro substituiertes Phenyl bedeutet.

**3.** Mittel nach Anspruch 1, welches eine Verbindung der Formel I enthält, worin $R_1$ Wasserstoff darstellt, $R_2$ für Methyl, Ethyl, Isopropyl oder sek.Butyl steht und R Wasserstoff, tert.Bütyl, durch 1 bis 4 Chlor- oder Fluoratome oder Methoxy substituiertes $C_1$-$C_4$-Alkyl, $C_2$-$C_3$-Alkenyl, $C_2$-$C_3$-Alkinyl oder $C_3$-$C_6$-Cycloalkyl; oder unsubstituiertes oder durch Chlor, Fluor, $C_1$-$C_2$-Alkyl, $C_1$-$C_2$-Alkoxy, $C_1$-$C_2$-Alkylthio oder Nitro substituiertes Phenyl bedeutet.

**4.** Mittel nach Anspruch 1, welches eine Verbindung der Formel I enthält, worin $R_1$ Wasserstoff darstellt, $R_2$ für Ethyl oder Isopropyl steht und R Wasserstoff, tert.Bütyl, $C_1$-$C_8$-Alkyl, welches durch $C_1$-$C_4$-Alkoxy oder ein- bis dreifach halogeniertes Phenoxy monosubstituiert oder durch 1 bis 5 Halogenatome substituiert ist, eine unsubstituierte oder durch Substituenten der Gruppe bestehend aus $C_1$-$C_4$-Alkyl und halogeniertem $C_2$-$C_4$-Alkenyl ein- oder mehrfach substituierte, mono-bis tetracyclische, aliphatische Gruppe mit insgesamt 3 bis 10 Kohlenstoffatomen im Ring oder Ringsystem, ein- bis dreifach halogeniertes $C_2$-$C_4$-Alkenyl, $C_3$-C-Alkinyl oder ein- bis dreifach durch Substituenten der Gruppe bestehend aus Halogen, $C_1$-$C_4$-Alkyl und Nitro substituiertes Phenyl bedeutet.

**5.** Mittel nach Anspruch 1, welches eine Verbindung der Formel I enthält, worin $R_1$ Wasserstoff darstellt, $R_2$ für Ethyl oder Isopropyl steht und R Wasserstoff, tert.Bütyl, $C_1$-$C_8$-Alkyl, welches durch Substituenten der Gruppe bestehend aus Chlor und Fluor ein- bis dreifach substituiert ist, Fluorphenoxymethyl, unsubstituiertes oder durch eine Methylgruppe substituiertes $C_3$-$C_4$-Cycloalkyl, Adamantyl, Trichlorvinyl oder Monochlorphenyl bedeutet.

**6.** Mittel nach Anspruch 1, welches eine Verbindung der Formel I enthält, worin $R_1$ den Rest -Si($R_6$)($R_7$)-($R_8$) darstellt, wobei $R_6$, $R_7$ und $R_8$ unabhängig voneinander $C_1$-$C_4$-Alkyl, Benzyl oder Phenyl bedeuten, und R und $R_2$ die in Anspruch 1 angegebenen Bedeutungen besitzen.

**7.** Mittel nach Anspruch 1, welches eine Verbindung der Formel I enthält, worin $R_1$ einen Rest aus der Gruppe Trimethylsilyl, Diphenyl-tert.butylsilyl, bis(Isopropyl)-methylsilyl, Triphenylsilyl und tert.Butyldimethylsilyl bedeutet.

**8.** Mittel nach Anspruch 1, welches eine Verbindung der Formel I enthält, worin $R_1$ den Rest $R_5$-C(O)-darstellt, wobei $R_5$ $C_1$-$c_{10}$-Alkyl, $C_1$-$C_{10}$-Haloalkyl oder einen unsubstituierten oder durch Substituenten der Gruppe bestehend aus Halogen, $C_1$-$C_3$-Alkyl, $C_1$-$C_3$-Haloalkyl, $C_1$-$C_3$-Alkoxy, $C_1$-$C_3$-Haloalkoxy, Cyano und Nitro substituierten Rest aus der Gruppe Phenyl und Benzyl bedeutet, und R und $R_2$ die in Anspruch 1 angegebenen Bedeutungen haben.

9. Mittel nach Anspruch 1, welches eine Verbindung der Formel I, ausgewählt aus der Gruppe:
13β-Formyloxy-milbemycin D
13β-Pivaloyloxy-milbemycin D
13β-Formyloxy-milbemycin $A_3$
13β-Pivaloyloxy-milbemycin $A_3$
13β-Formyloxy-milbemycin $A_4$
13β-Pivaloyloxy-milbemycin $A_4$.
13β-(2'-Methoxy-2'-methylpropionyloxy)-milbemycin D
13β-(2'-Methoxy-2'-methylpropionyloxy)-milbemycin $A_4$
13β-Trichloracetoxy-milbemycin D
13β-(4'-Chlor-butanoyloxy)milbemycin D
13β-Trichloracryloyloxy-milbemycin D
13β-Cyclopropancarbonyloxy-milbemycin D
13β-Cyclobutancarbonyloxy-milbemycin D
13β-(3'-Chlor-2'2'-dimethyl-propionyloxy)-milbemycin $A_4$
13β-(3'-Chlor-2'2'-dimethyl-propionyloxy)-milbemycin D
13β-(1'-Methyl-cyclopropancarbonyloxy)-milbemycin $A_4$
13β-(1'-Methyl-cyclopropancarbonyloxy)-milbemycin D
13β-(1-Adamantancarbonyloxy)-milbemycin D
13β-(p-Fluorphenoxyacetoxy)-milbemycin $A_4$
13β-(2'-Chlor-2'-methyl-propionyloxy)-milbemycin $A_4$
13β-(2',2'-Dichlorpropionyloxy)-milbemycin $A_4$
13β-(p-Chlorbenzoyloxy)-milbemycin $A_4$
13β-(3'.3',3'-Trifluorpropionyloxy)-milbemycin $A_4$
13β-Chloracetoxy-milbemycin $A_4$
13β-(2'-Chlor-3',3',3'-Trifluorpropionyloxy)-milbemycin D
13β-(3',3',3'-Trifluorpropionyloxy)-milbemycin D
enthält.

10. Mittel nach Anspruch 1, welches eine Verbindung der Formel I, ausgewählt aus der Gruppe:
5-O-tert.Butyldimethylsilyl-13β-Formyloxy-milbemycin D
5-O-tert.Butyldimethylsilyl-13β-Pivaloyloxy-milbemycin D
5-O-tert.Butyldimethylsilyl-13β-Formyloxy-milbemycin $A_3$
5-O-tert.Butyldimethylsilyl-13β-Pivaloyloxy-milbemycin $A_3$
5-O-tert.Butyldimethylsilyl-13β-Formyloxy-milbemycin $A_4$
5-O-tert.Butyldimethylsilyl-13β-Pivaloyloxy-milbemycin $A_4$.
5-O-tert.Butyldimethylsilyl-13β-(2'-Methoxy-2'-methylpropionyloxy)-milbemycin D
5-O-tert.Butyldimethylsilyl-13β-(2'-Methoxy-2'-methylpropionyloxy)-milbemycin $A_4$
5-O-tert.Butyldimethylsilyl-13β-Trichloracetoxy-milbemycin D
5-O-tert.Butyldimethylsilyl-13β-(4'-Chlor-butanoyloxy)milbemycin D
5-O-tert.Butyldimethylsilyl-13β-Trichloracryloyloxy-milbemycin D
5-O-tert.Butyldimethylsilyl-13β-Cyclopropancarbonyloxy-milbemycin D
5-O-tert.Butyldimethylsilyl-13β-Cyclobutancarbonyloxy-milbemycin D
5-O-tert.Butyldimethylsilyl-13β-(3'-Chlor-2'2'-dimethyl-propionyloxy)-milbemycin $A_4$
5-O-tert.Butyldimethylsilyl-13β-(3'-Chlor-2'2-dimethyl-propionyloxy)-milbemycin D
5-O-tert.Butyldimethylsilyl-13β-(1'-Methyl-cyclopropancarbonyloxy)-milbemycin $A_4$
5-O-tert.Butyldimethylsilyl-13β-(1'-Methyl-cyclopropancarbonyloxy)-milbemycin D
5-O-tert.Butyldimethylsilyl-13β-(1-Adamantancarbonyloxy)-milbemycin D
5-O-tert.Butyldimethylsilyl-13β-(p-Fluorphenoxyacetoxy)-milbemycin $A_4$
5-O-tert.Butyldimethylsilyl-13β-(2'-Chlor-2'-methyl-propionyloxy)-milbemycin $A_4$
5-O-tert.Butyldimethylsilyl-13β-(2',2'-Dichlorpropionyloxy)-milbemycin $A_4$
5-O-tert.Butyldimethylsilyl-13β-(p-Chlorbenzoyloxy)-milbemycin $A_4$
5-O-tert.Butyldimethylsilyl-13β-(3',3',3'-Trifluorpropionyloxy)-milbemycin $A_4$
5-O-tert.Butyldimethylsilyl-13β-Chloracetoxy-milbemycin $A_4$
5-O-tert.Butyldimethylsilyl-13β-(2'-Chlor-3',3',3'-Trifluorpropionyloxy)-milbemycin D
5-O-tert.Butyldimethylsilyl-13β-(3',3',3'-Trifluorpropionyloxy)-milbemycin D
enthält.

**11.** Verfahren zur Herstellung von Verbindungen der Formel I

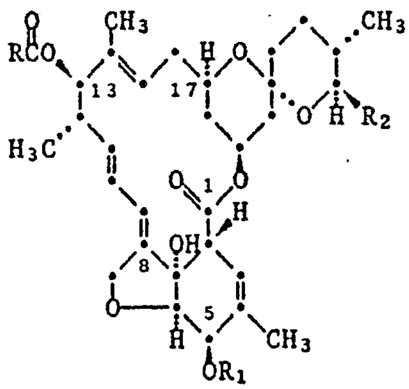

(I)

in welcher

$R_1$ Wasserstoff, die Silylgruppe -Si($R_6$)($R_7$)($R_8$), worin $R_6$, $R_7$ und $R_8$ unabhängig voneinander für $C_1$-$C_4$-Alkyl, Benzyl oder Phenyl stehen, oder die Acylgruppe $R_5$-C(O)-, worin $R_5$ für $C_1$-$C_{10}$-Alkyl, $C_1$-$C_{10}$-Haloalkyl oder einen unsubstituierten oder durch Substituenten der Gruppe bestehend aus Halogen, $C_1$-$C_3$-Alkyl, $C_1$-$C_3$-Haloalkyl, $C_1$-$C_3$-Alkoxy, $C_1$-$C_3$-Haloalkoxy, Nitro und Cyano substituierten Rest aus der Gruppe Phenyl und Benzyl steht,

$R_2$ Methyl, Ethyl, Isopropyl oder sek.Butyl und

R Wasserstoff, tert.Butyl, geradkettige oder verzweigte, durch 1 bis 7 Halogenatome, 1 bis 6 $C_1$-$C_6$-Alkoxygruppen oder eine unsubstituierte oder halogenierte Phenoxygruppe substituierte $C_1$-$C_{18}$-Alkylgruppen, mono- bis tetracyclische, aliphatische Gruppen mit 3 bis 10 Kohlenstoffatomen, welche in Verbindungen, in denen $R_1$ für Wasserstoff und $R_2$ für Aethyl oder Isopropyl steht, durch Substituenten der Gruppe bestehend aus $C_1$-$C_4$-Alkyl und halogeniertem $C_2$-$C_4$-Alkenyl ein- oder mehrfach substituiert sein können, durch 1 bis 7 Halogenatome, 1 bis 6 $C_1$-$C_6$-Alkoxygruppen oder durch $C_1$-$C_4$-Alkylgruppen substituierte Cycloalkylgruppen mit 3 bis 10 Kohlenstoffatomen, unsubstituierte oder durch 1 bis 7 Halogenatome oder 1 bis 6 $C_1$-$C_6$-Alkoxygruppen substituierte $C_2$-$C_6$-Alkenyl- oder Alkinylgruppen oder eine unsubstituierte oder durch 1 bis 3 Substituenten ausgewählt aus der Gruppe bestehend aus Halogenatomen, $C_1$-$C_6$-Alkyl, $c_1$-$C_6$-Alkoxy, $C_1$-$C_4$-Alkylthio oder Nitro substituierte Phenyl- oder Benzylgruppe bedeuten, dadurch gekennzeichnet, dass man Verbindungen der Formel II

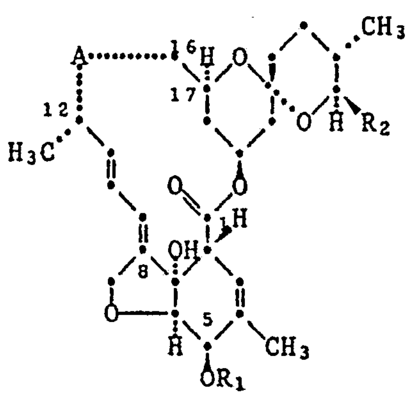

(II)

worin A für eine Gruppe a oder b

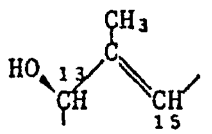 oder 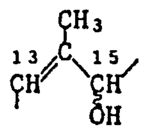

(a) (b)

[= 13β-Hydroxy-$\Delta^{14,15}$] [= $\Delta^{13,14}$-15-hydroxy]

steht, $R_1$ eine Schutzgruppe bedeutet und $R_2$ die unter Formel I angegebenen Bedeutungen besitzt, mit einem zur Einführung einer 13β-Estergruppe geeigneten Reagenz behandelt, woraufhin die $R_1$-Schutzgruppe, sofern freie 5-Hydroxy-Verbindungen erwünscht sind, abgespalten wird.

**12.** Verfahren nach Anspruch 11, dadurch gekennzeichnet, dass zur Einführung einer 13β-Estergruppe in Verbindungen der Formel II als Reagenz eingesetzt wird

i) bei Verbindungen der Formeln IIa oder IIb:

a) eine Säure der Formel III

RCOOH (III)

oder

b) ein Säureamid der Formel IV

$RCON(Alkyl)_2$ (IV),

worin die Alkylreste 1 bis 4 Kohlenstoffatome umfassen und vorzugsweise Methyl darstellen, oder

ii) bei Verbindungen der Formel IIa:

c) ein Säurehalogenid der Formel V

RCOhal (V)

worin hal Halogen, bevorzugt Chlor oder Brom bedeutet, oder

d) ein Säureanhydrid der Formel VI

$(RCO)_2O$ (VI)

in welchen R die unter Formel I angegebenen Bedeutungen besitzt.

**13.** Verfahren nach Anspruch 12, dadurch gekennzeichnet, dass die Reaktion von Verbindungen der Formel IIa oder IIb mit einer Säure der Formel III oder einem Säureamid der Formel IV in Gegenwart eines Orthoesters der Formel VII

$R_3C(OR_4)_3$ (VII)

worin $R_3$ Wasserstoff oder $C_1$-$C_4$-Alkyl und $R_4$ $C_1$-$C_4$-Alkyl bedeuten und zusätzlich in Gegenwart einer katalytisch wirkenden Säure bei Temperaturen im Bereich von 0° bis 150° C durchgeführt wird.

**14.** Verfahren nach Anspruch 12, dadurch gekennzeichnet, dass die Reaktion von Verbindungen der Formel IIa mit einem Säurechlorid oder Säurebromid der Formel V oder einem Säureanhydrid der Formel VI in einem reaktionsinerten Lösungsmittel bei Temperaturen von -20° bis 100° C durchgeführt wird.

**15.** Verfahren nach Anspruch 14, dadurch gekennzeichnet, dass als Neutralisationsmittel eine Base eingesetzt wird.

**16.** Verfahren zur Bekämpfung von Schädlingen, dadurch gekennzeichnet, dass man eine pestizid-wirksame Menge mindestens einer Verbindung der Formel I gemäss Anspruch 1 auf oder in die Wirtspflanzen oder sonstigen Lebensräume der Schädlinge mit Ausnahme menschlicher oder tierischer Körper appliziert.

**17.** Verfahren nach Anspruch 16, dadurch gekennzeichnet, dass die zu bekämpfenden Schädlinge Tiere befallende Endo- oder Ektoparasiten sind.

**18.** Verfahren nach Anspruch 16, dadurch gekennzeichnet, dass die zu bekämpfenden Schädlinge pflanzenschädigende Parasiten sind.

**19.** Verbindungen der Formel I gemäss Anspruch 1 zur Verwendung als antiparasitäres Mittel gegen Tiere befallende Endo- und Ektoparasiten.

**20.** Verwendung einer Verbindung der Formel I gemäß Ausspruch 1 zur Herstellung eines Arzneimittels zur Bekämpfung von Tiere befallenden Endo- und Ektoparasiten.

**Claims**

**1.** A compound of the formula I

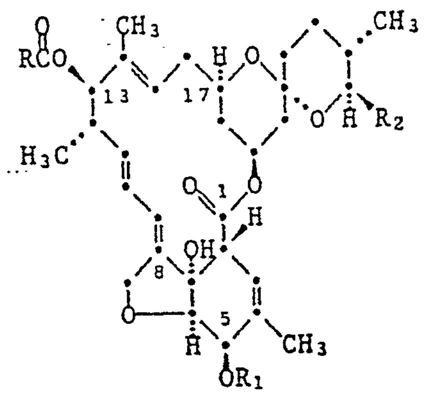

(I)

in which

$R_1$ is hydrogen, the silyl group, $-Si(R_6)(R_7)(R_8)$ in which $R_6$, $R_7$ and $R_8$ are independently of one another $C_1$-$C_4$alkyl, benzyl or phenyl, or the acyl group $R_5$-C(O)- in which $R_5$ is $C_1$-$C_{10}$ alkyl, $C_1$-$C_{10}$haloalkyl or a radical from the group consisting of phenyl and benzyl which is unsubstituted or substituted by substituents from the group consisting of halogen, $C_1$-$C_3$alkyl, $C_1$-$C_3$haloalkyl, $C_1$-$C_3$alkoxy, $C_1$-$C_3$haloalkoxy, nitro and cyano, $R_2$ is methyl, ethyl, isopropyl or sec-butyl, and R is hydrogen, tert-butyl, a straight-chain or branched $C_1$- $C_{18}$alkyl group which is substituted by 1 to 7 halogen atoms, 1 to 6 $C_1$-$C_6$alkoxy groups or an unsubstituted or halogenated phenoxy group, or is a mono- to tetracyclic aliphatic group having 3 to 10 carbon atoms, which in compounds where $R_1$ is hydrogen and $R_2$ is ethyl or isopropyl can be substituted by one or more substituents from the group consisting of $C_1$-$C_4$alkyl and halogenated $C_2$-$C_4$alkenyl, or is a cycloalkyl group having 3 to 10 carbon atoms which is substituted by 1 to 7 halogen atoms, 1 to 6 $C_1$-$C_6$alkoxy groups or by $C_1$-$C_4$alkyl groups, or is a $C_2$-$C_6$alkenyl or -alkynyl group which is unsubstituted or substituted by 1 to 7 halogen atoms or 1 to 6 $C_1$-$C_6$alkoxy groups, or is a phenyl or benzyl group which is unsubstituted or substituted by 1 to 3 substituents selected from the group consisting of halogen atoms, $C_1$-$C_6$alkyl, $C_1$-$C_6$alkoxy, $C_1$-$C_4$alkylthio and nitro.

**2.** A compound of the formula I according to claim 1, in which $R_1$ is hydrogen, $R_2$ is methyl, ethyl, isopropyl or sec-butyl and R is hydrogen, tert-butyl, or $C_1$-$C_6$alkyl, $C_2$-$C_4$alkenyl, $C_2$-$C_4$alkynyl or $C_3$-$C_6$cycloalkyl, each of which is substituted by 1 to 4 halogen atoms or $C_1$-$C_4$alkoxy, or is phenyl which is unsubstituted or substituted by 1 to 3 substituents from the group consisting of halogen, $C_1$-$C_4$alkyl, $C_1$-$C_4$alkoxy, $C_1$-$C_4$alkylthio and nitro.

3. A compound of the formula I according to claim 1, in which $R_1$ is hydrogen, $R_2$ is methyl, ethyl, isopropyl or sec-butyl and R is hydrogen, tert-butyl, or $C_1$-$C_4$alkyl, $C_2$-$C_3$alkenyl, $C_2$-$C_3$alkynyl or $C_3$-$C_6$-cycloalkyl, each of which is substituted by 1 to 4 chlorine or fluorine atoms or methoxy, or is phenyl which is unsubstituted or substituted by chlorine, fluorine, $C_1$-$C_2$alkyl, $C_1$-$C_2$alkoxy, $C_1$-$C_2$alkylthio or nitro.

4. A compound of the formula I according to claim 1, in which $R_1$ is hydrogen, $R_2$ is ethyl or isopropyl and R is hydrogen, tert-butyl, $C_1$-$C_8$alkyl which is monosubstituted by $C_1$-$C_4$alkoxy or by mono- to trihalogenated phenoxy or substituted by 1 to 5 halogen atoms, or is a mono- to tetracyclic aliphatic group having a total of 3 to 10 carbon atoms in the ring or ring system and which is unsubstituted or substituted by one or more substituents from the group consisting of $C_1$-$C_4$alkyl and halogenated $C_2$-$C_4$alkenyl, or is mono- to trihalogenated $C_2$-$C_4$alkenyl, $C_3$-$C_4$alkynyl, or phenyl which is substituted by 1 to 3 substituents from the group consisting of halogen, $C_1$-$C_4$alkyl and nitro.

5. A compound of the formula I according to claim 1, in which $R_1$ is hydrogen, $R_2$ is ethyl or isopropyl and R is hydrogen, tert-butyl, $C_1$-$C_8$alkyl which is substituted by 1 to 3 substituents from the group consisting of chlorine and fluorine, or is fluorophenoxymethyl, $C_3$-$C_4$cycloalkyl which is unsubstituted or substituted by a methyl group, or is adamantyl, trichlorovinyl or monochlorophenyl.

6. A compound of the formula I according to claim 1, in which $R_1$ is the radical -Si($R_6$)$R_7$)($R_8$) in which $R_6$, $R_7$ and $R_8$, are independently of one another $C_1$-$C_4$alkyl, benzyl or phenyl, and R and $R_2$ are as defined in claim 1.

7. A compound of the formula I according to claim 1, in which $R_1$ is a radical from the group consisting of trimethylsilyl, diphenyl-tert-butylsilyl, bis(isopropyl)methylsilyl, triphenylsilyl and tert-butyldimethylsilyl.

8. A compound of the formula I according to claim 1, in which $R_1$ is the radical $R_5$-C(O) in which $R_5$ is $C_1$-$C_{10}$alkyl, $C_1$-$C_{10}$haloalkyl, or a radical from the group consisting of phenyl or benzyl which is unsubstituted or substituted by substituents from the group consisting of halogen, $C_1$-$C_3$alkyl, $C_1$-$C_3$haloalkyl, $C_1$-$C_3$alkoxy, $C_1$-$C_3$haloalkoxy, cyano and nitro, and R and $R_2$ are as defined in claim 1.

9. A compound according to claim 1, selected from the group consisting of:
13β-formyloxymilbemycin D
13β-pivaloyloxymilbemycin D
13β-formyloxymilbemycin $A_3$
13β-pivaloyloxymilbemycin $A_3$
13β-formyloxymilbemycin $A_4$
13β-pivaloyloxymilbemycin $A_4$
13β-(2'-methoxy-2'-methylpropionyloxy)milbemycin D
13β-(2'-methoxy-2'-methylpropionyloxy)milbemycin $A_4$
13β-trichloroacetoxymilbemycin D
13β-(4'-chlorobutanoyloxy)milbemycin D
13β-trichloroacryloyloxymilbemycin D
13β-cyclopropanecarbonyloxymilbemycin D
13β-cyclobutanecarbonyloxymilbemycin D
13β-(3'-chloro-2'2'-dimethylpropionyloxy)milbemycin $A_4$
13β-(3'-chloro-2'2'-dimethylpropionyloxy)milbemycin D
13β-(1'-methylcyclopropanecarbonyloxy)milbemycin $A_4$
13β-(1'-methylcyclopropanecarbonyloxy)milbemycin D
13β-(1-adamantanecarbonyloxy)milbemycin D
13β-(p-fluorophenoxyacetoxy)milbemycin $A_4$
13β-(2'-chloro-2'-methylpropionyloxy)milbemycin $A_4$
13β-(2',2'-dichloropropionyloxy)milbemycin $A_4$
13β-(p-chlorobenzoyloxy)milbemycin $A_4$
13β-(3',3',3'-trifluoropropionyloxy)milbemycin $A_4$
13β-chloroacetoxymilbemycin $A_4$
13β-(2'-chloro-3',3',3'-trifluoropropionyloxy)milbemycin D
13β-(3',3',3'-trifluoropropionyloxy)milbemycin D.

10. A compound according to claim 1, selected from the group consisting of:
5-O-tert-butyldimethylsilyl-13β-formyloxymilbemycin D
5-O-tert-butyldimethylsilyl-13β-pivaloyloxymilbemycin D
5-O-tert-butyldimethylsilyl-13β-formyloxymilbemycin $A_3$
5-O-tert-butyldimethylsilyl-13β-pivaloyloxymilbemycin $A_3$
5-O-tert-butyldimethylsilyl-13β-formyloxymilbemycin $A_4$
5-O-tert-butyldimethylsilyl-13β-pivaloyloxymilbemycin $A_4$
5-O-tert-butyldimethylsilyl-13β-(2'-methoxy-2'-methylpropionyloxy)-milbemycin D
5-O-tert-butyldimethylsilyl-13β-(2'-methoxy- 2'-methylpropionyloxy)-milbemycin $A_4$
5-O-tert-butyldimethylsilyl-13β-trichloroacetoxymilbemycin D
5-O-tert-butyldimethylsilyl-13β-(4'-chlorobutanoyloxy)milbemycin D
5-O-tert-butyldimethylsilyl-13β-trichloroacryloyloxymilbemycin D
5-O-tert-butyldimethylsilyl-13β-cyclopropanecarbonyloxymilbemycin D
5-O-tert-butyldimethylsilyl-13β-cyclobutanecarbonyloxymilbemycin D
5-O-tert-butyldimethylsilyl-13β-(3'-chloro-2'2'-dimethylpropionyloxy)milbemycin $A_4$
5-O-tert-butyldimethylsilyl-13β-(3'-chloro-2'2'-dimethylpropionyloxy)milbemycin D
5-O-tert-butyldimethylsilyl-13β-(1'-methylcyclopropanecarbonyloxy)milbemycin $A_4$
5-O-tert-butyldimethylsilyl-13β-(1'-methylcyclopropanecarbonyloxy)milbemycin D 5-O-tert-butyldimethylsilyl-13β-(1-adamantanecarbonyloxy)milbemycin D
5-O-tert-butyldimethylsilyl-13β-(p-fluorophenoxyacetoxy)milbemycin $A_4$
5-O-tert-butyldimethylsilyl-13β-(2'-chloro-2'-methyl-propionyloxy)-milbemycin $A_4$
5-O-tert-butyldimethylsilyl-13β-(2',2'-dichloropropionyloxy)milbemycin $A_4$
5-O-tert-butyldimethylsilyl-13β-(p-chlorobenzoyloxy)milbemycin $A_4$
5-O-tert-butyldimethylsilyl-13β-(3',3',3'-trifluoropropionyloxy)-milbemycin $A_4$
5-O-tert-butyldimethylsilyl-13β-chloroacetoxymilbemycin $A_4$
5-O-tert-butyldimethylsilyl-13β-(2'-chloro-3',3',3'-trifluoropropionyloxy)-milbemycin D
5-O-tert-butyldimethylsilyl-13β-(3',3',3'-trifluoropropionyloxy)-milbemycin D.

11. A process for the preparation of a compound of the formula I

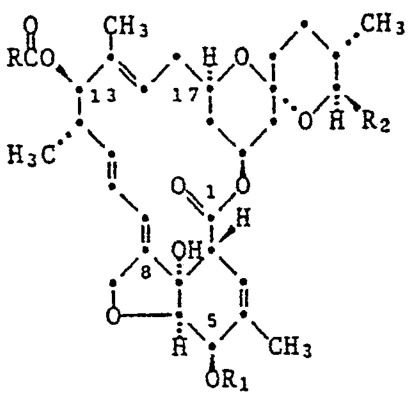

(I)

in which
$R_1$ is hydrogen, the silyl group, $-Si(R_6)(R_7)(R_8)$ in which $R_6$, $R_7$ and $R_8$ are independently of one another $C_1$-$C_4$alkyl, benzyl or phenyl, or the acyl group $R_5$-C(O)- in which $R_5$ is $C_1$-$C_{10}$alkyl, $C_1$-$C_{10}$haloalkyl or a radical from the group consisting of phenyl and benzyl which is unsubstituted or substituted by substituents from the group consisting of halogen, $C_1$-$C_3$alkyl, $C_1$-$C_3$haloalkyl, $C_1$-$C_3$alkoxy, $C_1$-$C_3$haloalkoxy, nitro and cyano, $R_2$ is methyl, ethyl, isopropyl or sec-butyl, and R is hydrogen, tert-butyl, a straight-chain or branched $C_1$-$C_{18}$alkyl group which is substituted by 1 to 7 halogen atoms, 1 to 6 $C_1$-$C_6$alkoxy groups or an unsubstituted or halogenated phenoxy group, or is a mono- to tetracyclic aliphatic group having 3 to 10 carbon atoms, which in compounds where $R_1$ is hydrogen and $R_2$ is ethyl or isopropyl can be substituted by one or more substituents from the group consisting of $C_1$-$C_4$alkyl and halogenated $C_2$-$C_4$alkenyl, or is a cycloalkyl group having 3 to 10 carbon atoms which is substituted by 1 to 7 halogen atoms, 1 to 6 $C_1$-$C_6$alkoxy groups or by $C_1$-$C_4$alkyl groups, or is a $C_2$-$C_6$alkenyl or -alkynyl group which is unsubstituted or substituted by 1 to 7 halogen atoms or 1 to 6 $C_1$-$C_6$alkoxy groups, or is a phenyl or benzyl group which is unsubstituted or

substituted by 1 to 3 substituents selected from the group consisting of halogen atoms, $C_1$-$C_6$alkyl, $C_1$-$C_6$alkoxy, $C_1$-$C_4$alkylthio and nitro, which comprises treating a compound of the formula II

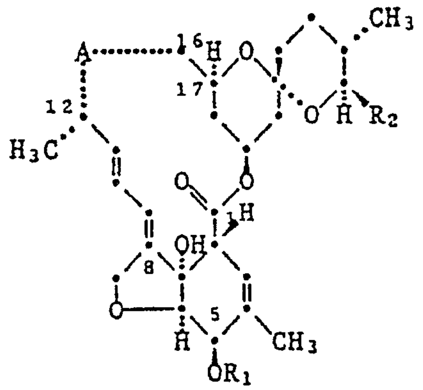

(II)

in which A is a group a or b

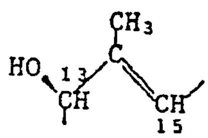

(a)

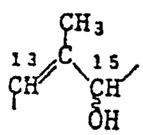

(b)

[= 13β-hydroxy-$\Delta^{14,15}$] [= $\Delta^{13,14}$-15-hydroxy]

$R_1$ is a protecting group and $R_2$ is as defined for formula I, with a reagent suitable for the introduction of a 13β-ester group and, if a free 5-hydroxy compound is desired, subsequently removing the protecting group $R_1$.

**12.** A process according to claim 11, wherein the reagent for the introduction of a 13β-ester group into a compound of the formula II is

i) in the case of a compound of the formula IIa or IIb:

a) an acid of the formula III

RCOOH (III)

or

b) an acid amide of the formula (IV)

RCON(alkyl)$_2$ (IV)

in which the alkyl moieties contain 1 to 4 carbon atoms and are preferably methyl

or

ii) in the case of a compound of the formula IIa:

c) an acid halide of the formula V

RCOhal (V)

in which hal is halogen, preferably chlorine or bromine,

or

d) an acid anhydride of the formula VI

$(RCO)_2O$ (VI)

in which R is as defined for formula I.

**13.** A process according to claim 12, which comprises carrying out the reaction of a compound of the formula IIa or IIb with an acid of the formula III or an acid amide of the formula IV in the presence of an orthoester of the formula VII

$R_3C(OR_4)_3$ (VII)

in which $R_3$ is hydrogen or $C_1$-$C_4$alkyl and $R_4$, is $C_1$-$C_4$alkyl, and also in the presence of a catalytically effective acid, in the temperature range from 0° to 150°C.

**14.** A process according to claim 12, which comprises carrying out the reaction of a compound of the formula IIa with an acid chloride or acid bromide of the formula V or an acid anhydride of the formula VI in an inert solvent at a temperature of from -20° to 100°C.

**15.** A process according to claim 14, wherein the neutralising agent used is a base.

**16.** A composition for controlling pests, which comprises, together with carriers, dispersing agents or carriers and dispensing agents, as active ingredient at least one compound of the formula I according to claim 1.

**17.** A method of controlling pests, which comprises applying to the host plants or to other loci of said pests, with the exception of human or animal bodies, a pesticidally effective amount of at least one compound of the formula I according to claim 1.

**18.** A method according to claim 17, wherein the pests to be controlled are endoparasites or ectoparasites that attack animals.

**19.** A method according to claim 17, wherein the pests to be controlled are plant-destructive parasites.

**20.** A compound of the formula I according to claim 1 for use as an anti-parasitic composition against endoparasites and ectoparasites that attack animals.

**21.** The use of a compound of the formula I according to claim 1 for the production of a medicament for the control of endoparasites and ectoparasites that attack animals.

Claims for the following Contracting State: AT

**1.** A composition for controlling pests, which comprises, together with carriers, dispersing agents or carriers and dispersing agents, as active ingredient at least one compound of the formula I

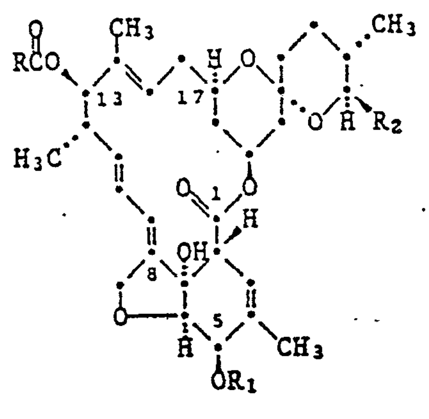

(I)

$R_1$ is hydrogen, the silyl group, $-Si(R_6)(R_7)(R_8)$ in which $R_6$, $R_7$ and $R_8$ are independently of one

another $C_1$-$C_4$alkyl, benzyl or phenyl, or the acyl group $R_5$-C(O)- in which $R_5$ is $C_1$-$C_{10}$ alkyl, $C_1$-$C_{10}$haloalkyl or a radical from the group consisting of phenyl and benzyl which is unsubstituted or substituted by substituents from the group consisting of halogen, $C_1$-$C_3$alkyl, $C_1$-$C_3$haloalkyl, $C_1$-$C_3$alkoxy, $C_1$-$C_3$haloalkoxy, nitro and cyano, $R_2$ is methyl, ethyl, isopropyl or sec-butyl, and R is hydrogen, tert-butyl, a straight-chain or branched $C_1$-$C_{18}$alkyl group which is substituted by 1 to 7 halogen atoms, 1 to 6 $C_1$-$C_6$alkoxy groups or an unsubstituted or halogenated phenoxy group, or is a mono- to tetracyclic aliphatic group having 3 to 10 carbon atoms, which in compounds where $R_1$ is hydrogen and $R_2$ is ethyl or isopropyl can be substituted by one or more substituents from the group consisting of $C_1$-$C_4$alkyl and halogenated $C_2$-$C_4$alkenyl, or is a cycloalkyl group having 3 to 10 carbon atoms which is substituted by 1 to 7 halogen atoms, 1 to 6 $C_1$-$C_6$alkoxy groups or by $C_1$-$C_4$alkyl groups, or is a $C_2$-$C_6$alkenyl or -alkynyl group which is unsubstituted or substituted by 1 to 7 halogen atoms or 1 to 6 $C_1$-$C_6$alkoxy groups, or is a phenyl or benzyl group which is unsubstituted or substituted by 1 to 3 substituents selected from the group consisting of halogen atoms, $C_1$-$C_6$alkyl, $C_1$-$C_6$alkoxy, $C_1$-$C_4$alkylthio and nitro.

2. A composition according to claim 1, comprising a compound of the formula I in which $R_1$ is hydrogen, $R_2$ is methyl, ethyl, isopropyl or sec-butyl and R is hydrogen, tert-butyl, or $C_1$-$C_6$alkyl, $C_2$-$C_4$alkenyl, $C_2$-$C_4$alkynyl or $C_3$-$C_6$cycloalkyl, each of which is substituted by 1 to 4 halogen atoms or $C_1$-$C_4$alkoxy, or is phenyl which is unsubstituted or substituted by 1 to 3 substituents from the group consisting of halogen, $C_1$-$C_4$alkyl, $C_1$-$C_4$alkoxy, $C_1$-$C_4$alkylthio and nitro.

3. A composition according to claim 1, comprising a compound of the formula I in which $R_1$ is hydrogen, $R_2$ is methyl, ethyl, isopropyl or sec-butyl and R is hydrogen, tert-butyl, or $C_1$-$C_4$alkyl, $C_2$-$C_3$alkenyl, $C_2$-$C_3$alkynyl or $C_3$-$C_6$-cycloalkyl, each of which is substituted by 1 to 4 chlorine or fluorine atoms or methoxy, or is phenyl which is unsubstituted or substituted by chlorine, fluorine, $C_1$-$C_2$alkyl, $C_1$-$C_2$alkoxy, $C_1$-$C_2$alkylthio or nitro.

4. A composition according to claim 1, comprising a compound of the formula I in which $R_1$ is hydrogen, $R_2$ is ethyl or isopropyl and R is hydrogen, tert-butyl, $C_1$-$C_8$alkyl which is monosubstituted by $C_1$-$C_4$alkoxy or by mono- to trihalogenated phenoxy or substituted by 1 to 5 halogen atoms, or is a mono- to tetracyclic aliphatic group having a total of 3 to 10 carbon atoms in the ring or ring system and which is unsubstituted or substituted by one or more substituents from the group consisting of $C_1$-$C_4$alkyl and halogenated $C_2$-$C_4$alkenyl, or is mono- to trihalogenated $C_2$-$C_4$alkenyl, $C_3$-$C_4$alkynyl, or phenyl which is substituted by 1 to 3 substituents from the group consisting of halogen, $C_1$-$C_4$alkyl and nitro.

5. A composition according to claim 1, comprising a compound of the formula I in which $R_1$ is hydrogen, $R_2$ is ethyl or isopropyl and R is hydrogen, tert-butyl, $C_1$-$C_8$alkyl which is substituted by 1 to 3 substituents from the group consisting of chlorine and fluorine, or is fluorophenoxymethyl, $C_3$-$C_4$cycloalkyl which is unsubstituted or substituted by a methyl group, or is adamantyl, trichlorovinyl or monochlorophenyl.

6. A composition according to claim 1, comprising a compound of the formula I in which $R_1$ is the radical -Si($R_6$)($R_7$)($R_8$) in which $R_6$, $R_7$ and $R_8$ are independently of one another $C_1$-$C_4$alkyl, benzyl or phenyl, and R and $R_2$ are as defined in claim 1.

7. A composition according to claim 1, comprising a compound of the formula I in which $R_1$ is a radical from the group consisting of trimethylsilyl, diphenyl-tert-butylsilyl, bis(isopropyl)methylsilyl, triphenylsilyl and tert-butyldimethylsilyl.

8. A composition according to claim 1, comprising a compound of the formula I in which $R_1$ is the radical $R_5$-C(O), in which $R_5$ is $C_1$-$C_{10}$alkyl, $C_1$-$C_{10}$haloalkyl, or a radical from the group consisting of phenyl or benzyl which is unsubstituted or substituted by substituents from the group consisting of halogen, $C_1$-$C_3$alkyl, $C_1$-$C_3$haloalkyl, $C_1$-$C_3$alkoxy, $C_1$-$C_3$haloalkoxy, cyano and nitro, and R and $R_2$ are as defined in claim 1.

9. A composition according to claim 1, comprising a compound of the formula I selected from the group consisting of:
13$\beta$-formyloxymilbemycin D

13β-pivaloyloxymilbemycin D
13β-formyloxymilbemycin $A_3$
13β-pivaloyloxymilbemycin $A_3$
13β-formyloxymilbemycin $A_4$
13β-pivaloyloxymilbemycin $A_4$
13β-(2'-methoxy-2'-methylpropionyloxy)milbemycin D
13β-(2'-methoxy-2'-methylpropionyloxy)milbemycin $A_4$
13β-trichloroacetoxymilbemycin D
13β-(4'-chlorobutanoyloxy)milbemycin D
13β-trichloroacryloyloxymilbemycin D
13β-cyclopropanecarbonyloxymilbemycin D
13β-cyclobutanecarbonyloxymilbemycin D
13β-(3'-chloro-2'2'-dimethylpropionyloxy)milbemycin $A_4$
13β-(3'-chloro-2'2'-dimethylpropionyloxy)milbemycin D
13β-(1'-methylcyclopropanecarbonyloxy)milbemycin $A_4$
13β-(1'-methylcyclopropanecarbonyloxy)milbemycin D
13β-(1-adammantanecarbonyloxy)milbemycin D
13β-(p-fluorophenoxyacetoxy)milbemycin $A_4$
13β-(2'-chloro-2'-methylpropionyloxy)milbemycin $A_4$
13β-(2'-2'-dichloropropionyloxy)milbemycin $A_4$
13β-(p-chlorobenzoyloxy)milbemycin $A_4$
13β-(3',3',3'-trifluoropropionyloxy)milbemycin $A_4$
13β-chloroacetoxymilbemycin $A_4$
13β-(2'-chloro-3',3',3'-trifluoropropionyloxy) milbemycin D
13β-(3',3',3'-trifluoropropionyloxy)milbemycin D.

**10.** A composition according to claim 1, comprising a compound of the formula I selected from the group consisting of:
5-O-tert-butyldimethylsilyl-13β-formyloxymilbemycin D
5-O-tert-butyldimethylsilyl-13β-pivaloyloxymilbemycin D
5-O-tert-butyldimethylsilyl-13β-formyloxymilbemycin $A_3$
5-O-tert-butyldimethylsilyl-13β-pivaloyloxymilbemycin $A_3$
5-O-tert-butyldimethylsilyl-13β-formyloxymilbemycin $A_4$
5-O-tert-butyldimethylsilyl-13β-pivaloyloxymilbemycin $A_4$
5-O-tert-butyldimethylsilyl-13β-(2'-methoxy-2'-methylpropionyloxy)-milbemycin D
5-O-tert-butyldimethylsilyl-13β-(2'-methoxy-2'-methylpropionyloxy)-milbemycin $A_4$
5-O-tert-butyldimethylsilyl-13β-trichloroacetoxymilbemycin D
5-O-tert-butyldimethylsilyl-13β-(4'chlorobutanoyloxy)milbemycin D
5-O-tert-butyldimethylsilyl-13β-trichloroacryloyloyxymilbemycin D
5-O-tert-butyldimethylsilyl-13β-cyclopropanecarbonyloxymilbemycin D
5-O-tert-butyldimethylsilyl-13β-cyclobutanecarbonyloxymilbemycin D
5-O-tert-butyldimethylsilyl-13β-(3'-chloro-2'2'-dimethylpropionyloxy)milbemycin $A_4$
5-O-tert-butyldimethylsilyl-13β-(3'-chloro-2'2'-dimethylpropionyloxy)milbemycin D
5-O-tert-butyldimethylsilyl-13β-(1'-methylcyclopropanecarbonyloxy)milbemycin $A_4$
5-O-tert-butyldimethylsilyl-13β-(1'-methylcyclopropanecarbonyloxy)milbemycin D
5-O-tert-butyldimethylsilyl-13β-(1-adamantanecarbonyloxy)milbemycin D
5-O-tert-butyldimethylsilyl-13β-(p-fluorophenoxyacetoxy)milbemycin $A_4$
5-O-tert-butyldimethylsilyl-13β-(2'-chloro-2'-methyl-propionyloxy)-milbemycin $A_4$
5-O-tert-butyldimethylsilyl-13β-(2',2'-dichloropropionyloxy)milbemycin $A_4$
5-O-tert-butyldimethylsilyl-13β-(p-chlorobenzoyloxy)milbemycin $A_4$
5-O-tert-butyldimethylsilyl-13β-(3',3',3'-trifluoropropionyloxy)-milbemycin $A_4$
5-O-tert-butyldimethylsilyl-13β-chloroacetoxymilbemycin $A_4$
5-O-tert-butyldimethylsilyl-13β-(2'-chloro3',3'-trifluoropropionyloxy)-milbemycin D
5-O-tert-butyldimethylsilyl-13β-(3',3',3'-trifluoropropionyloxy)-milbemycin D.

**11.** A process for the preparation of a compound of the formula I

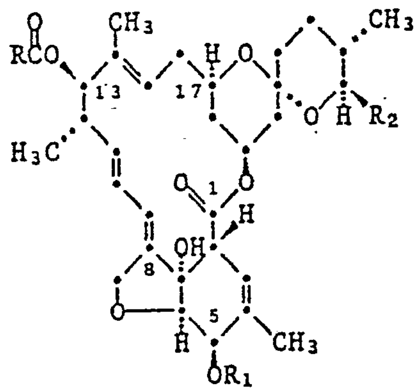

(I)

in which

$R_1$ is hydrogen, the silyl group, $-Si(R_6)(R_7)(R_8)$ in which $R_6$, $R_7$ and $R_8$ are independently of one another $C_1$-$C_4$alkyl, benzyl or phenyl, or the acyl group $R_5$-C(O)- in which $R_5$ is $C_1$-$C_{10}$ alkyl, $C_1$-$C_{10}$haloalkyl or a radical from the group consisting of phenyl and benzyl which is unsubstituted or substituted by substituents from the group consisting of halogen, $C_1$-$C_3$alkyl, $C_1$-$C_3$haloalkyl, $C_1$-$C_3$alkoxy, $C_1$-$C_3$haloalkoxy, nitro and cyano, $R_2$ is methyl, ethyl, isopropyl or sec-butyl, and R is hydrogen, tert-butyl, a straight-chain or branched $C_1$-$C_{18}$alkyl group which is substituted by 1 to 7 halogen atoms, 1 to 6 $C_1$-$C_6$alkoxy groups or an unsubstituted or halogenated phenoxy group, or is a mono- to tetracyclic aliphatic group having 3 to 10 carbon atoms, which in compounds where $R_1$ is hydrogen and $R_2$ is ethyl or isopropyl can be substituted by one or more substituents from the group consisting of $C_1$-$C_4$alkyl and halogenated $C_2$-$C_4$alkenyl, or is a cycloalkyl group having 3 to 10 carbon atoms which is substituted by 1 to 7 halogen atoms, 1 to 6 $C_1$-$C_6$alkoxy groups or by $C_1$-$C_4$alkyl groups, or is a $C_2$-$C_6$alkenyl or -alkynyl group which is unsubstituted or substituted by 1 to 7 halogen atoms or 1 to 6 $C_1$-$C_6$alkoxy groups, or is a phenyl or benzyl group which is unsubstituted or substituted by 1 to 3 substituents selected from the group consisting of halogen atoms, $C_1$-$C_6$alkyl, $C_1$-$C_6$alkoxy, $C_1$-$C_4$alkylthio and nitro, which comprises treating a compound of the formula II

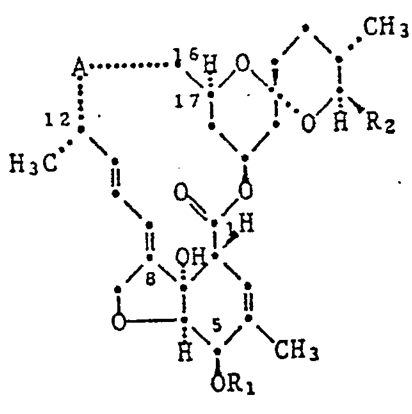

(II)

in which A is a group a or b

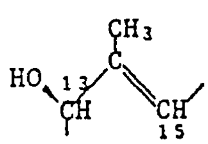

(a)

[= 13β-hydroxy-$\Delta^{14,15}$]

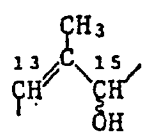

(b)

[= $\Delta^{13,14}$-15-hydroxy]

$R_1$ is a protecting group and $R_2$ is as defined for formula I, with a reagent suitable for the introduction of a 13$\beta$-ester group and, if a free 5-hydroxy compound is desired, subsequently removing the protecting group $R_1$.

**12.** A process according to claim 11, wherein the reagent for the introduction of a 13$\beta$-ester group into a compound of the formula II is

i) in the case of a compound of the formula IIa or IIb:

a) an acid of the formula III

RCOOH (III)

or

b) an acid amide of the formula (IV)

$RCON(alkyl)_2$ (IV)

in which the alkyl moieties contain 1 to 4 carbon atoms and are preferably methyl

or

ii) in the case of a compound of the formula IIa:

c) an acid halide of the formula V

RCOhal (V)

in which hal is halogen, preferably chlorine or bromine,

or

d) an acid anhydride of the formula VI

$(RCO)_2O$ (VI)

in which R is as defined for formula I.

**13.** A process according to claim 12, which comprises carrying out the reaction of a compound of the formula IIa or IIb with an acid of the formula III or an acid amide of the formula IV in the presence of an orthoester of the formula VII

$R_3C(OR_4)_3$ (VII)

in which $R_3$ is hydrogen or $C_1$-$C_4$alkyl and $R_4$ is $C_1$-$C_4$alkyl, and also in the presence of a catalytically effective acid, in the temperature range from 0° to 150°C.

**14.** A process according to claim 12, which comprises carrying out the reaction of a compound of the formula IIa with an acid chloride or acid bromide of the formula V or an acid anhydride of the formula VI in an inert solvent at a temperature of from -20° to 100°C.

**15.** A process according to claim 14, wherein the neutralising agent used is a base.

**16.** A method of controlling pests, which comprises applying to the host plants or to other loci of said pests, with the exception of human or animal bodies, a pesticidally effective amount of at least one compound of the formula I according to claim 1.

**17.** A method according to claim 16, wherein the pests to be controlled are endoparasites or ectoparasites that attack animals.

**18.** A method according to claim 17, wherein the pests to be controlled are plant-destructive parasites.

**19.** A compound of the formula I according to claim 1 for use as an anti-parasitic composition against endoparasites and ectoparasites that attack animals.

20. The use of a compound of the formula I according to claim 1 for the production of a medicament for the control of endoparasites and ectoparasites that attack animals.

## Revendications

1. Composés de formule I

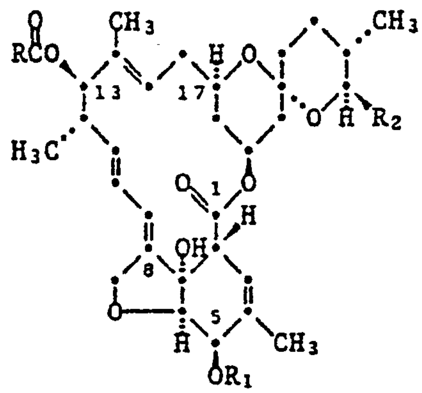

(I)

dans laquelle
$R_1$ est l'hydrogène, le groupe silyle-Si($R_6$)($R_7$)($R_8$), dans lequel $R_6$, $R_7$ et $R_8$ représentent indépendamment l'un de l'autre un alkyle $C_1$-$C_4$, benzyle ou phényle, ou le groupe acyle $R_5$-C(O)-, dans lequel $R_5$ représente un alkyle $C_1$-$C_{10}$, haloalkyle $C_1$-$C_{10}$ ou un reste du groupe phényle et benzyle non substitué ou substitué par des substituants du groupe constitué des halogène, alkyle $C_1$-$C_3$, haloalkyle $c_1$-$c_3$, alcoxy $C_1$-$C_3$, haloalcoxy $C_1$-$C_3$, nitro et cyano,
$R_2$ est le méthyle, l'éthyle ou sec.butyle et
R est l'hydrogène, tert.butyle, des groupes alkyles $C_1$-$C_{18}$ linéaires ou ramifiés substitués par 1 à 7 atomes d'halogène, 1 à 6 groupes alcoxy $C_1$-$C_6$ ou un groupe phénoxy non substitué ou halogéné, des groupes aliphatiques mono-à tétracycliques avec de 3 à 10 atomes de carbone, qui peuvent être mono- ou polysubstitués par des substituants du groupe constitué d'alkyle $C_1$-$C_4$ et d'alcényle $c_2$-$C_4$ halogéné, dans les composés pour lesquels $R_1$ est l'hydrogène et $R_2$ est l'éthyle ou l'isopropyle, des groupes cycloalkyles de 3 à 10 atomes de carbone substitués par 1 à 7 atomes de carbone, 1 à 6 groupes alcoxy $C_1$-$C_6$ ou par des groupes alkyles $C_1$-$C_4$, des groupes alcényle ou alcynyle $C_2$-$C_6$ non substitués ou substitués par 1 à 7 atomes d'halogène ou 1 à 6 groupes alcoxy $C_1$-$C_6$ ou un groupe benzyle ou phényle non substitué ou substitué par 1 à 3 substituants choisis dans le groupe constitué des atomes d'halogènes, alkyle $C_1$-$C_6$, alcoxy $C_1$-$C_6$, alkylthio $C_1$-$C_4$ ou nitro.

2. Composés de formule I selon la revendication 1, dans laquelle $R_1$ est 1'hydrogène, $R_2$ le méthyle, éthyle, isopropyle ou sec-butyle et R est l'hydrogène. tert.butyle ou un alkyle $C_1$-$C_6$, alcényle $C_2$-$C_4$, alcynyle $C_2$-$C_4$ ou cycloalkyle $C_3$-$C_6$ substitué par un alcoxy $C_1$-$C_4$ ou par 1 à 4 atomes d'halogène ; ou un phényle non substitué ou substitué une à trois fois par halogène, alkyle $C_1$-$C_4$, alcoxy $C_1$-$C_4$, alkylthio $C_1$-$C_4$ ou nitro.

3. Composés de formule I selon la revendication 1, dans laquelle $R_1$ est l'hydrogène, $R_2$ le méthyle, éthyle, isopropyle ou sec.butyle et R est l'hydrogène, tert.butyle ou un alkyle $C_1$-$C_4$, alcényle $C_2$-$C_3$, alcynyle $C_2$-$C_3$ ou cycloalkyle $C_3$-$C_6$ substitué par un méthoxy ou par 1 à 4 atomes de chlore ou de fluor ; ou un phényle non substitué ou substitué par du chlore, fluor, alkyle $C_1$-$C_2$, alcoxy $C_1$-$C_2$, alkylthio $C_1$-$C_2$ ou nitro.

4. Composés de formule I selon la revendication 1, dans laquelle $R_1$ est l'hydrogène, $R_2$ l'éthyle ou isopropyle et R est l'hydrogène, tert.butyle, alkyle $C_1$-$C_8$ qui est monosubstitué par un alcoxy $C_1$-$C_4$ ou un phénoxy mono-ou trihalogéné ou qui est substitué par 1 à 5 atomes d'halogène, un groupe aliphatique, mono- à tétracyclique non substitué ou mono- ou polysubstitué par des substituants du groupe constitué d'alkyle $C_1$-$C_4$ et alcényle halogéné $C_2$-$C_4$ avec globalement 3 à 10 atomes de carbone dans le cycle ou le système cyclique, un alcényle $c_2$-$C_4$, alcynyle $c_3$-$c_4$ mono- à trihalogéné ou un phényle non substitué une à trois fois par des substituants du groupe constitué d'halogène,

alkyle $C_1$-$C_4$ et nitro.

5. Composés de formule I selon la revendication 1, dans laquelle $R_1$ est l'hydrogène, $R_2$ est l'éthyle ou isopropyle et R est l'hydrogène, tert.butyle, alkyle $c_1$-$C_8$ qui est substitué une à trois fois par des substituants du groupe constitué du chlore et du fluor, le fluorométhyle, un cycloalkyle $C_3$-$C_4$ non substitué ou substitué par un groupe méthyle, l'adamantyle, le trichlorovinyle ou le monochlorophényle.

6. Composés de formule I selon la revendication 1, dans laquelle $R_1$ représente le reste -Si($R_6$)($R_7$)($R_8$), $R_6$, $R_7$ et $R_8$ représentant indépendamment l'un de l'autre un alkyle $C_1$-$C_4$, benzyle ou phényle et R et $R_2$ ont la signification donnée dans la revendication 1.

7. Composés de formule I selon la revendication 1, dans laquelle $R_1$ est un reste du groupe des triméthylsilyle, diphényl-tert.butylsilyle, bis(isopropyl)-méthylsilyle, triphénylsilyle et tert.butyl-diméthylsilyle.

8. Composés de formule I selon la revendication 1, dans laquelle $R_1$ représente le reste $R_5$-C(O)-, $R_5$ étant un alkyle $C_1$-$C_{10}$, haloalkyle $C_1$-$C_{10}$ ou un reste du groupe du phényle ou benzyle non substitué ou substitué par des substituants du groupe constitué d'halogène, alkyle $C_1$-$C_3$, haloalkyle $C_1$-$C_3$, alcoxy $C_1$-$C_3$, haloalcoxy $C_1$-$C_3$, cyano et nitro, et R et $R_2$ ont la signification donnée dans la revendication 1.

9. Composé selon la revendication 1 choisi dans le groupe :
13$\beta$-formyloxy-milbémycine D
13$\beta$-pivaloyloxy-milbémycine D
13$\beta$-formyloxy-milbémycine $A_3$
13$\beta$-pivaloyloxy-milbémycine $A_3$
13$\beta$-formyloxy-milbémycine $A_4$
13$\beta$-pivaloyloxy-milbémycine $A_4$.
13$\beta$-(2'-méthoxy-2'-méthylpropionyloxy)-milbémycine D
13$\beta$-(2'-méthoxy-2'-méthylpropionyloxy)-milbémycine $A_4$
13$\beta$-trichloroacétoxy-milbémycine D
13$\beta$-(4'-chloro-butanoyloxy)milbémycine D
13$\beta$-trichloroacryloyloxy-milbémycine D
13$\beta$-cyclopropanecarbonyloxy-milbémycine D
13$\beta$-cyclobutanecarbonyloxy-milbémycine D
13$\beta$-(3'-chloro-2'2'-diméthyl-propionyloxy)-milbémycine $A_4$
13$\beta$-(3'-chloro-2'2'-diméthyl-propionyloxy)-milbémycine D
13$\beta$-(1'-méthyl-cyclopropanecarbonyloxy)-milbémycine $A_4$
13$\beta$-(1'-méthyl-cyclopropanecarbonyloxy)-milbémycine D
13$\beta$-(1-adamantanecarbonyloxy)-milbémycine D
13$\beta$-(p-fluorophénoxyacétoxy)-milbémycine $A_4$
13$\beta$-(2'-chloro-2'-méthyl-propionyloxy)-milbémycine $A_4$
13$\beta$-(2',2'-dichloropropionyloxy)-milbémycine $A_4$
13$\beta$-(p-chlorobenzoyloxy)-milbémycine $A_4$
13$\beta$-(3',3',3'-trifluoropropionyloxy)-milbémycine $A_4$
13$\beta$-chloroacétoxy-milbémycine $A_4$
13$\beta$-(2'-chloro-3',3',3'-trifluoropropionyloxy)-milbémycine D
13$\beta$-(3',3',3'-trifluoropropionyloxy)-milbémycine D.

10. Composé selon la revendication 1, choisi dans le groupe :
5-O-tert.butyldiméthylsilyl-13$\beta$-formyloxy-milbémycine D
5-O-tert.butyldiméthylsilyl-13$\beta$-pivaloyloxy-milbémycine D
5-O-tert.butyldiméthylsilyl-13$\beta$-formyloxy-milbémycine $A_3$
5-O-tert.butyldiméthylsilyl-13$\beta$-pivaloyloxy-milbémycine $A_3$
5-O-tert.butyldiméthylsilyl-13$\beta$-formyloxy-milbémycine $A_4$
5-O-tert.butyldiméthylsilyl-13$\beta$-pivaloyloxy-milbémycine $A_4$
5-O-tert.butyldiméthylsilyl-13$\beta$-(2'-méthoxy-2'-méthylpropionyloxy)-milbémycine D
5-O-tert.butyldiméthylsilyl-13$\beta$-(2'-méthoxy-2'-méthylpropionyloxy)-milbémycine $A_4$

5-O-tert.butyldiméthylsilyl-13β-trichloroacétoxy-milbémycine D
5-O-tert.butyldiméthylsilyl-13β-(4'-chloro-butanoyloxy)-milbémycine D
5-O-tert.butyldiméthylsilyl-13β-trichloroacryloyloxy-milbémycine D
5-O-tert.butyldiméthylsilyl-13β-cyclopropanecarbonyloxymilbémycine D
5-O-tert.butyldiméthylsilyl-13β-cyclobutanecarbonyloxy-milbémycine D
5-O-tert.butyldiméthylsilyl-13β-(3'-chloro-2'2'-diméthylpropionyloxy)-milbémycine $A_4$
5-O-tert.butyldiméthylsilyl-13β-(3'-chloro-2'2'-diméthylpropionyloxy)-milbémycine D
5-O-tert.butyldiméthylsilyl-13β-(1'-méthyl-cyclopropanecarbonyloxy)-milbémycine $A_4$
5-O-tert.butyldiméthylsilyl-13β-(1'-méthyl-cyclopropanecarbonyloxy)-milbémycine D
5-O-tert.butyldiméthylsilyl-13β-(1-adamantanecarbonyloxy)-milbémycine D
5-O-tert.butyldiméthylsilyl-13β-(p-fluorophénoxyacétoxy)-milbémycine $A_4$
5-O-tert.butyldiméthylsilyl-13β-(2'-chloro-2'-méthylpropionyloxy)-milbémycine $A_4$
5-O-tert.butyldiméthylsilyl-13β-(2',2'-dichloropropionyloxy)-milbémycine $A_4$
5-O-tert.butyldiméthylsilyl-13β-(p-chlorobenzoyloxy)-milbémycine $A_4$
5-O-tert.butyldiméthylsilyl-13β-(3',3',3'-trifluoropropionyloxy)-milbémycine $A_4$
5-O-tert.butyldiméthylsilyl-13β-chloroacétoxy-milbémycine $A_4$
5-O-tert.butyldiméthylsilyl-13β-(2'-chloro-3',3',3'-trifluoropropionyloxy)-milbémycine D
5-O-tert.butyldiméthylsilyl-13β-(3',3',3'-trifluoropropionyloxy)-milbémycine D.

**11.** Procédé de préparation de composés de formule I

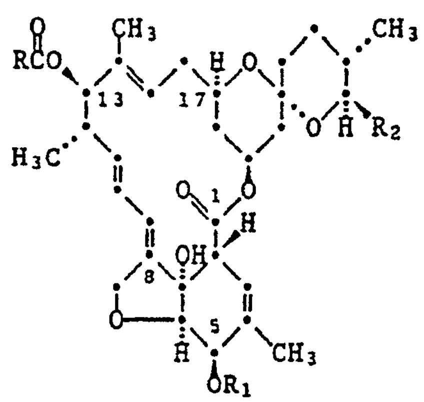

(I)

dans laquelle

$R_1$ est l'hydrogène, le groupe silyle $-Si(R_6)(R_7)(R_8)$, dans lequel $R_6$ $R_7$ et $R_8$ représentent indépendamment l'un de l'autre un alkyle $C_1$-$C_4$, benzyle ou phényle, ou le groupe acyle $R_5$-C(O)-, dans lequel $R_5$ représente un alkyle $C_1$-$C_{10}$, haloalkyle $C_1$-$C_{10}$ ou un reste du groupe phényle et benzyle non substitué ou substitué par des substituants du groupe constitué des halogène, alkyle $C_1$-$C_3$, haloalkyle $C_1$-$C_3$, alcoxy $C_1$-$C_3$, haloalcoxy $C_1$-$C_3$, nitro et cyano,

$R_2$ est le méthyle, l'éthyle ou sec.butyle et

R est l'hydrogène, tert.butyle, des groupes alkyles $C_1$-$C_{18}$ linéaires ou ramifiés substitués par 1 à 7 atomes d'halogène, 1 à 6 groupes alcoxy $C_1$-$C_6$ ou un groupe phénoxy non substitué ou halogéné, des groupes aliphatiques mono- à tétracycliques avec de 3 à 10 atomes de carbone, qui peuvent être mono- ou polysubstitués par des substituants du groupe constitué d'alkyle $C_1$-$C_4$ et d'alcényle $C_2$-$C_4$ halogéné, dans les composés pour lesquels $R_1$ est l'hydrogène et $R_2$ est l'éthyle ou l'isopropyle, des groupes cycloalkyles de 3 à 10 atomes de carbone substitués par 1 à 7 atomes de carbone, 1 à 6 groupes alcoxy $C_1$-$C_6$ ou par des groupes alkyles $C_1$-$C_4$, des groupes alcényle ou alcynyle $C_2$-$C_6$ non substitués ou substitués par 1 à 7 atomes d'halogène ou 1 à 6 groupes alcoxy $C_1$-$C_6$ ou un groupe benzyle ou phényle non substitué ou substitué par 1 à 3 substituants choisis dans le groupe constitué des atomes d'halogènes, alkyle $C_1$-$C_6$, alcoxy $C_1$-$C_6$, alkylthio $C_1$-$C_4$ ou nitro, caractérisé en ce qu'on traite des composés de formule II

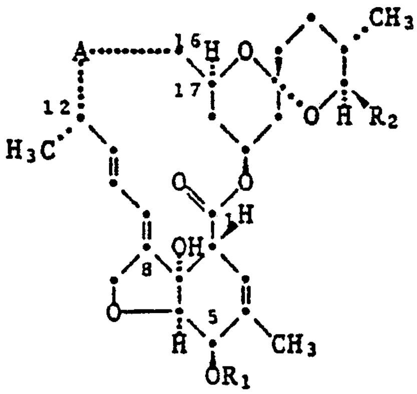

(II)

dans laquelle A est un groupe a ou b

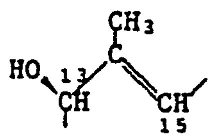

ou

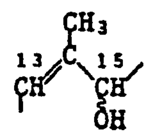

(a) (b)

[= 13β-hydroxy-$\Delta^{14,15}$] [= $\Delta^{13,14}$-15-hydroxy]

$R_1$ est un groupe protecteur et $R_2$ a les significations données pour la formule I, avec un réactif approprié pour introduire un groupe 13β-ester, puis on élimine le groupe protecteur $R_1$, dans la mesure où on souhaite avoir des composés 5-hydroxy libres.

**12.** Procédé selon la revendication 1, caractérisé en ce qu'on utilise comme réactif pour introduire un groupe 13 -ester dans des composés de formule II

i) dans des composés de formule IIa ou IIb :

a) un acide de formule III

RCOOH (III)

ou

b) un amide d'acide de formule IV

RCON(alkyl)$_2$ (IV),

dans laquelle les restes alkyles comprennent 1 à 4 atomes de carbone et représentent de préférence le méthyle,

ou

ii) dans des composés de formule IIa :

c) un halogénure d'acide de formule V

RCOhal (V)

dans laquelle hal représente un halogène, de préférence le chlore ou le brome,

ou

d) un anhydride d'acide de formule VI

(RCO)$_2$O (VI)

dans laquelle R a les significations indiquées pour la formule I.

**13.** Procédé selon la revendication 12, caractérisé en ce qu'on réalise la réaction de composés de formule IIa ou IIb avec un acide de formule III ou avec un amide d'acide de formule IV en présence d'un orthoester de formule VII

$R_3C(OR_4)_3$ (VII)

dans lequel $R_3$ est l'hydrogène ou un alkyle $C_1$-$C_4$ et $R_4$ un alkyle $C_1$-$C_4$, et en plus en présence d'acide à action catalytique à des températures comprises entre 0° et 150° C.

**14.** Procédé selon la revendication 12, caractérisé en ce qu'on réalise la réaction de composés de formule IIa avec un chlorure d'acide ou bromure d'acide de formule V ou un anhydride d'acide de formule VI dans un solvant inerte vis-à-vis de la réaction à des températures comprises entre -20° et 100° C.

**15.** Procédé selon la revendication 14, caractérisé en ce qu'on utilise une base comme agent de neutralisation.

**16.** Moyen de lutte contre des parasites, caractérisé en ce qu'outre les véhicules, agents dispersants ou agents de transfert et de dispersion, il contient comme principe actif au moins un composé de formule I selon la revendication 1.

**17.** Procédé de lutte contre des parasites, caractérisé en ce qu'on applique une quantité pesticide efficace d'au moins un composé de formule I selon la revendication 1 sur ou dans les plantes utiles ou d'autres lieux d'existence des parasites à l'exception du corps humain ou animal.

**18.** Procédé selon la revendication 17, caractérisé en ce que les parasites à combattre sont des endo- ou ectoparasites contaminant des animaux.

**19.** Procédé selon la revendication 17, caractérisé en ce que les nuisibles à combattre sont des parasites endommageant les plantes.

**20.** Composés de formule I selon la revendication 1 pour l'utilisation comme moyen antiparasitaire d'endo- et ectoparasites contaminant les animaux.

**21.** Utilisation d'un composé de formule I selon la revendication 1 pour préparer un médicament pour combattre les endo- et ectoparasites contaminant des animaux.

Revendications pour l'Etat contractant suivant: AT

**1.** Moyen de lutte contre les parasites, caractérisé en ce qu'outre les véhicules, agents dispersants ou agents de transfert et de dispersion, il contient comme principe actif au moins un composé de formule I

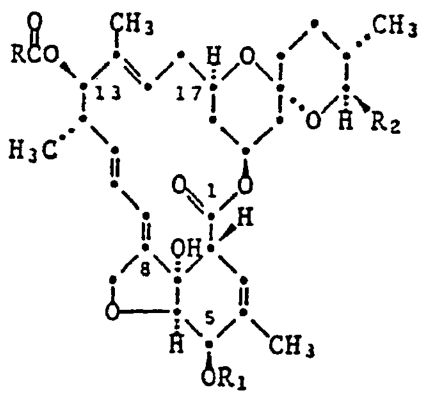

(I)

dans laquelle

$R_1$ est l'hydrogène, le groupe silyle -Si($R_6$)($R_7$)($R_8$), dans lequel $R_6$, $R_7$ et $R_8$ représentent indépendamment l'un de l'autre un alkyle $C_1$-$C_4$, benzyle ou phényle, ou le groupe acyle $R_5$-C(O)-, dans lequel $R_5$ représente un alkyle $C_1$-$C_{10}$, haloalkyle $C_1$-$C_{10}$ ou un reste du groupe phényle et benzyle non substitué ou substitué par des substituants du groupe constitué des halogène, alkyle $C_1$-$C_3$, haloalkyle $C_1$-$C_3$, alcoxy $C_1$-$C_3$, haloalcoxy $C_1$-$C_3$, nitro et cyano,
$R_2$ est le méthyle, l'éthyle, isopropyle ou sec.butyle, et
R est l'hydrogène, tert.butyle, des groupes alkyles $C_1$-$C_{18}$ linéaires ou ramifiés substitués par 1 à 7 atomes d'halogène, 1 à 6 groupes alcoxy $C_1$-$C_6$ ou un groupe phénoxy non substitué ou halogéné, des groupes aliphatiques mono-à tétracycliques avec de 3 à 10 atomes de carbone, qui peuvent être mono- ou polysubstitués par des substituants du groupe constitué d'alkyle $C_1$-$C_4$ et d'alcényle $C_2$-$C_4$halogéné, dans les composés pour lesquels $R_1$ est l'hydrogène et $R_2$ est l'éthyle ou l'isopropyle, des groupes cycloalkyles de 3 à 10 atomes de carbone substitués par 1 à 7 atomes de carbone, 1 à 6 groupes alcoxy $C_1$-$C_6$ ou par des groupes alkyles $C_1$-$C_4$, des groupes alcényle ou alcynyle $C_2$-$C_6$ non substitués ou substitués par 1 à 7 atomes d'halogène ou 1 à 6 groupes alcoxy $C_1$-$C_6$ ou un groupe benzyle ou phényle non substitué ou substitué par 1 à 3 substituants choisis dans le groupe constitué des atomes d'halogènes, alkyle $C_1$-$C_6$, alcoxy $C_1$-$C_6$, alkylthio $C_1$-$C_4$ ou nitro.

2. Moyen selon la revendication 1, qui contient un composé de formule I dans laquelle $R_1$ est l'hydrogène, $R_2$ le méthyle, éthyle, isopropyle ou sec.butyle et R est l'hydrogène, tert.butyle ou un alkyle $C_1$-$C_6$, alcényle $C_2$-$C_4$, alcynyle $C_2$-$C_4$ ou cycloalkyle $C_3$-$C_6$ substitué par un alcoxy $C_1$-$C_4$ ou par 1 à 4 atomes d'halogène ; ou un phényle non substitué ou substitué une à trois fois par halogène, alkyle $C_1$-$C_4$, alcoxy $C_1$-$C_4$, alkylthio $C_1$-$C_4$ ou nitro.

3. Moyen selon la revendication 1, qui contient un composé de formule I dans laquelle $R_1$ est l'hydrogène, $R_2$ le méthyle, éthyle, isopropyle ou sec.butyle et R est l'hydrogène, tert.butyle ou un alkyle $C_1$-$C_4$, alcényle $C_2$-$C_3$, alcynyle $C_2$-$C_3$ ou cycloalkyle $C_3$-$C_6$ substitué par un méthoxy ou par 1 à 4 atomes de chlore ou de fluor ; ou un phényle non substitué ou substitué par du chlore, fluor, alkyle $C_1$-$C_2$, alcoxy $C_1$-$C_2$, alkylthio $C_1$-$C_2$ ou nitro.

4. Moyen selon la revendication 1, qui contient un composé de formule I dans laquelle $R_1$ est l'hydrogène, $R_2$ l'éthyle ou isopropyle et R est l'hydrogène, tert. butyle, alkyle $C_1$-$C_8$ qui est monosubstitué par un alcoxy $C_1$-$C_4$ ou un phénoxy mono- ou trihalogéné ou qui est substitué par 1 à 5 atomes d'halogène, un groupe aliphatique, mono- à tétracyclique non substitué ou mono- ou polysubstitué par des substituants du groupe constitué d'alkyle $C_1$-$C_4$ et alcényle halogéné $C_2$-$C_4$ avec globalement 3 à 10 atomes de carbone dans le cycle ou le système cyclique, un alcényle $C_2$-$C_4$, alcynyle $C_3$-$C_4$ mono- à trihalogéné ou un phényle substitué une à trois fois par des substituants du groupe constitué d'halogène, alkyle $C_1$-$C_4$ et nitro.

5. Moyen selon la revendication 1, qui contient un composé de formule I dans laquelle $R_1$ est l'hydrogène, $R_2$ est l'éthyle ou isopropyle et R est l'hydrogène, tert. butyle, alkyle $C_1$-$C_8$ qui est substitué une à trois fois par des substituants du groupe constitué du chlore et du fluor, le fluorométhyle, un cycloalkyle $C_3$-$C_4$ non substitué ou substitué par un groupe méthyle, l'adamantyle, le trichlorovinyle ou le monochlorophényle.

6. Moyen selon la revendication 1, qui contient un composé de formule I dans laquelle $R_1$ représente le reste -Si($R_6$)($R_7$)($R_8$), $R_6$, $R_7$ et $R_8$ représentant indépendamment l'un de l'autre un alkyle $C_1$-$C_4$, benzyle ou phényle et R et $R_2$ ont la signification donnée dans la revendication 1.

7. Moyen selon la revendication 1, qui contient un composé de formule I dans laquelle $R_1$ est un reste du groupe des triméthylsilyle, diphényl-tert.butylsilyle, bis(isopropyl)-méthylsilyle, triphénylsilyle et tert. butyl-diméthylsilyle.

8. Moyen selon la revendication 1, qui contient un composé de formule I dans laquelle $R_1$ représente le reste $R_5$-C(O)-, $R_5$ étant un alkyle $C_1$-$C_{10}$, haloalkyle $C_1$-$C_{10}$ ou un reste du groupe du phényle ou benzyle non substitué ou substitué par des substituants du groupe constitué d'halogène, alkyle $C_1$-$C_3$, haloalkyle $C_1$-$C_3$, alcoxy $C_1$-$C_3$, haloalcoxy $C_1$-$C_3$, cyano et nitro, et R et $R_2$ ont la signification donnée dans la revendication 1.

**9.** Moyen selon la revendication 1 qui contient un composé de formule I, choisi dans le groupe :
13β-formyloxy-milbémycine D
13β-pivaloyloxy-milbémycine D
13β-formyloxy-milbémycine $A_3$
13β-pivaloyloxy-milbémycine $A_3$
13β-formyloxy-milbémycine $A_4$
13β-pivaloyloxy-milbémycine $A_4$.
13β-(2'-méthoxy-2'-méthylpropionyloxy)-milbémycine D
13β-(2'-méthoxy-2'-méthylpropionyloxy)-milbémycine $A_4$
13β-trichloroacétoxy-milbémycine D
13β-(4'-chloro-butanoyloxy)milbémycine D
13β-trichloroacryloyloxy-milbémycine D
13β-cyclopropanecarbonyloxy-milbémycine D
13β-cyclobutanecarbonyloxy-milbémycine D
13β-(3'-chloro-2'2'-diméthyl-propionyloxy)-milbémycine $A_4$
13β-(3'-chloro-2'2'-diméthyl-propionyloxy)-milbémycine D
13β-(l'-méthyl-cyclopropanecarbonyloxy)-milbémycine $A_4$
13β-(1'-méthyl-cyclopropanecarbonyloxy)-milbémycine D
13β-(1-adamantanecarbonyloxy)-milbémycine D
13β-(p-fluorophénoxyacétoxy)-milbémycine $A_4$
13β-(2'-chloro-2'-méthyl-propionyloxy)-milbémycine $A_4$
13β-(2',2'-dichloropropionyloxy)-milbémycine $A_4$
13β-(p-chlorobenzoyloxy)-milbémycine $A_4$
13β-(3,3',3'-trifluoropropionyloxy)-milbémycine $A_4$
13β-chloroacétoxy-milbémycine $A_4$
13β-(2'-chloro-3',3',3'-trifluoropropionyloxy)-milbémycine D
13β-(3',3',3'-trifluoropropionyloxy)-milbémycine D.

**10.** Moyen selon la revendication 1, qui contient un composé de formule I choisi dans le groupe :
5-O-tert.butyldiméthylsilyl-13β-formyloxy-milbémycine D
5-O-tert.butyldiméthylsilyl-13β-pivaloyloxy-milbémycine D
5-O-tert.butyldiméthylsilyl-13β-formyloxy-milbémycine $A_3$
5-O-tert.butyldiméthylsilyl-13β-pivaloyloxy-milbémycine $A_3$
5-O-tert.butyldiméthylsilyl-13β-formyloxy-milbémycine $A_4$
5-O-tert.butyldiméthylsilyl-13β-pivaloyloxy-milbémycine $A_4$
5-O-tert.butyldiméthylsilyl-13β-(2'-méthoxy-2'-méthylpropionyloxy)-milbémycine D
5-O-tert.butyldiméthylsilyl-13β-(2'méthoxy-2'-méthylpropionyloxy)-milbémycine $A_4$
5-O-tert.butyldiméthylsilyl-13β-trichloroacétoxy-milbémycine D
5-O-tert.butyldiméthylsilyl-13β-(4'-chloro-butanoyloxy)-milbémycine D
5-O-tert.butyldiméthylsilyl-13β-trichloroacryloyloxymilbémycine D
5-O-tert.butyldiméthylsilyl-13β-cyclopropanecarbonyloxy-milbémycine D
5-O-tert.butyldiméthylsilyl-13β-cyclobutanecarbonyloxy-milbémycine D
5-O-tert.butyldiméthylsilyl-13β-(3'-chloro-2'2'-diméthylpropionyloxy)-milbémycine $A_4$
5-O-tert.butyldiméthylsilyl-13β-(3'-chloro-2'2'-diméthylpropionyloxy)-milbémycine D
5-O-tert.butyldiméthylsilyl-13β-(1'-méthyl-cyclopropanecarbonyloxy)-milbémycine $A_4$
5-O-tert.butyldiméthylsilyl-13β-(1'-méthyl-cyclopropanecarbonyloxy)-milbémycine D
5-O-tert.butyldiméthylsilyl-13β-(1-adamantanecarbonyloxy)-milbémycine D
5-O-tert.butyldiméthylsilyl-13β-(p-fluorophénoxyacétoxy)-milbémycine $A_4$
5-O-tert.butyldiméthylsilyl-13β-(2'-chloro-2'-méthylpropionyloxy)-milbémycine $A_4$
5-O-tert.butyldiméthylsilyl-13β-(2',2',-dichloropropionyloxy)-milbémycine $A_4$
5-O-tert.butyldiméthylsilyl-13β-(p-chlorobenzoyloxy)-milbémycine $A_4$
5-O-tert.butyldiméthylsilyl-13β-(3',3',3'-trifluoropropionyloxy)-milbémycine $A_4$
5-O-tert.butyldiméthylsilyl-13β-chloroacétoxy-milbémycine $A_4$
5-O-tert.butyldiméthylsilyl-13β-(2'chloro-3',3',3'-trifluoropropionyloxy)-milbémycine D
5-O-tert.butyldiméthylsilyl-13β-(3',3',3'-trifluoropropionyloxy)-milbémycine D.

**11.** Procédé de préparation de composés de formule I

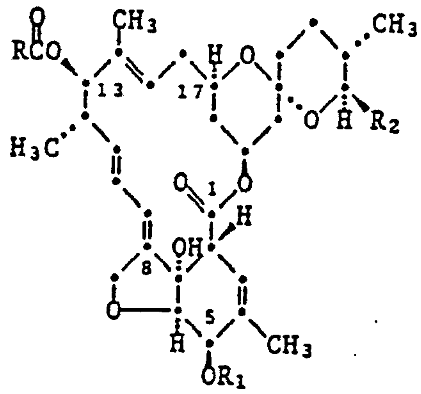

(I)

dans laquelle

$R_1$ est l'hydrogène, le groupe silyle $-Si(R_6)(R_7)(R_8)$, dans lequel $R_6$, $R_7$ et $R_8$ représentent indépendamment l'un de l'autre un alkyle $C_1$-$C_4$, benzyle ou phényle, ou le groupe acyle $R_5$-C(O)-, dans lequel $R_5$ représente un alkyle $C_1$-$C_{10}$, haloalkyle $C_1$-$C_{10}$ ou un reste du groupe phényle et benzyle non substitué ou substitué par des substituants du groupe constitué des halogène, alkyle $C_1$-$C_3$, haloalkyle $C_1$-$C_3$, alcoxy $C_1$-$C_3$, haloalcoxy $C_1$-$C_3$, nitro et cyano,

$R_2$ est le méthyle, l'éthyle, isopropyle ou sec.butyle et R est l'hydrogène, tert.butyle, des groupes alkyles $C_1$-$C_{18}$ linéaires ou ramifiés substitués par 1 à 7 atomes d'halogène, 1 à 6 groupes alcoxy $C_1$-$C_6$ ou un groupe phénoxy non substitué ou halogéné, des groupes aliphatiques mono-à tétracycliques avec de 3 à 10 atomes de carbone, qui peuvent être mono- ou polysubstitués par des substituants du groupe constitué d'alkyle $C_1$-$C_4$ et d'alcényle $C_2$-$C_4$ halogéné, dans les composés pour lesquels $R_1$ est l'hydrogène et $R_2$ est l'éthyle ou l'isopropyle, des groupes cycloalkyles de 3 à 10 atomes de carbone substitués par 1 à 7 atomes de carbone, 1 à 6 groupes alcoxy $C_1$-$C_6$ ou par des groupes alkyles $C_1$-$C_4$, des groupes alcényle ou alcynyle $C_2$-$C_6$ non substitués ou substitués par 1 à 7 atomes d'halogène ou 1 à 6 groupes alcoxy $C_1$-$C_6$ ou un groupe benzyle ou phényle non substitué ou substitué par 1 à 3 substituants choisis dans le groupe constitué des atomes d'halogènes, alkyle $C_1$-$C_6$, alcoxy $C_1$-$C_6$, alkylthio $C_1$-$C_4$ ou nitro,

caractérisé en ce qu'on traite des composés de formule II

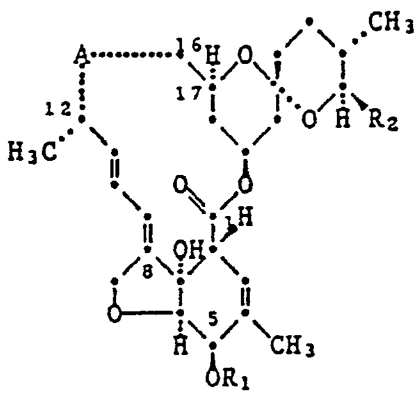

(II)

dans laquelle A est un groupe a ou b

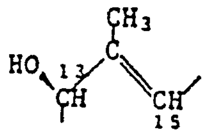 ou 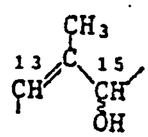

(a) [= 13β-hydroxy-$\Delta^{14,15}$]

(b) [= $\Delta^{13,14}$-15-hydroxy]

$R_1$ est un groupe protecteur et $R_2$ a les significations données pour la formule I, avec un réactif approprié pour introduire un groupe 13β-ester, puis on élimine le groupe protecteur $R_1$, dans la mesure où l'on souhaite avoir des composés 5-hydroxy libres.

**12.** Procédé selon la revendication 1, caractérisé en ce qu'on utilise comme réactif pour introduire un groupe 13 -ester dans des composés de formule II

i) dans des composés de formule IIa ou IIb :

a) un acide de formule III

RCOOH (III)

ou

b) un amide d'acide de formule IV

$RCON(alkyl)_2$ (IV)

dans laquelle les restes alkyles comprennent 1 à 4 atomes de carbone et représentent de préférence le méthyle,

ou

ii) dans des composés de formule IIa :

c) un halogénure d'acide de formule V

RCOhal (V)

dans laquelle hal représente un halogène, de préférence le chlore ou le brome,

ou

d) un anhydride d'acide de formule VI

$(RCO)_2O$ (VI)

dans laquelle R a les significations indiquées pour la formule I.

**13.** Procédé selon la revendication 12, caractérisé en ce qu'on réalise la réaction de composés de formule IIa ou IIb avec un acide de formule III ou avec un amide d'acide de formule IV en présence d'un orthoester de formule VII

$R_3C(OR_4)_3$ (VII)

dans lequel $R_3$ est l'hydrogène ou un alkyle $C_1$-$C_4$ et $R_4$ un alkyle $C_1$-$C_4$, et en plus en présence d'acide à action catalytique à des températures comprises entre 0° et 150° C.

**14.** Procédé selon la revendication 12, caractérisé en ce qu'on réalise la réaction de composés de formule IIa avec un chlorure d'acide ou bromure d'acide de formule V ou un anhydride d'acide de formule VI dans un solvant inerte vis-à-vis de la réaction à des températures comprises entre -20° et 100° C.

**15.** Procédé selon la revendication 14, caractérisé en ce qu'on utilise une base comme agent de neutralisation.

**16.** Procédé de lutte contre les parasites, caractérisé en ce qu'on applique une quantité pesticide efficace d'au moins un composé de formule I selon la revendication 1 sur ou dans les plantes utiles ou d'autres lieux d'existence des parasites à l'exception du corps humain ou animal.

**17.** Procédé selon la revendication 16, caractérisé en ce que les parasites à combattre sont des endo- ou ectoparasites contaminant des animaux.

**18.** Procédé selon la revendication 16, caractérisé en ce que les nuisibles à combattre sont des parasites endommageant les plantes.

**19.** Composés de formule I selon la revendication 1 pour l'utilisation comme moyen antiparasitaire d'endo- et ectoparasites contaminant les animaux.

**20.** Utilisation d'un composé de formule I selon la revendication 1 pour préparer un médicament pour combattre les endo- et ectoparasites contaminant des animaux.